# FASCICULE DE BREVET EUROPEEN

(11) **EP 3 250 688 B1**
(45) Date de publication et mention de la délivrance du brevet: **28.07.2021**
(21) Numéro de dépôt: 16703283.8
(22) Date de dépôt: 29.01.2016
(51) Int. Cl.: C12N 9/22, C12N 15/10, C12N 15/63, C12N 15/90

(54) **PROCEDE POUR INDUIRE DES RECOMBINAISONS MEIOTIQUES CIBLEES**
VERFAHREN ZUR INDUZIERUNG VON ZIELGERICHTETEN MEIOTISCHEN REKOMBINATIONEN
METHOD FOR INDUCING TARGETED MEIOTIC RECOMBINATIONS

(30) Priorité: 29.01.2015 FR 1550707; 22.06.2015 FR 1555725
(43) Date de publication de la demande: 06.12.2017
(60) Demande divisionnaire: 20205199.1
(73) Titulaire: Meiogenix, 75011 Paris (FR); Institut Curie, 75248 Paris Cedex 05 (FR); Centre National de la Recherche Scientifique, 75016 Paris (FR); SORBONNE UNIVERSITE, 75006 Paris (FR)
(72) Inventeur: BASTIANELLI, Giacomo, 75012 Paris (FR); SERERO, Alexandre, 92700 Colombes (FR); NICOLAS, Alain, 75015 Paris (FR)
(74) Mandataire: Cabinet Becker et Associés
(86) Numéro de dépôt international: PCT/EP2016/052000
(87) Numéro de publication internationale: WO 2016/120480

(56) Documents cités:
- WO-A1-2014/104878
- US-A1- 2004 033 494
- J. E. DICARLO ET AL: "Genome engineering in Saccharomyces cerevisiae using CRISPR-Cas systems", NUCLEIC ACIDS RESEARCH, vol. 41, no. 7, 4 mars 2013 (2013-03-04), pages 4336-4343, XP055086617, ISSN: 0305-1048, DOI: 10.1093/nar/gkt135
- M. JINEK ET AL: "A Programmable Dual-RNA-Guided DNA Endonuclease in Adaptive Bacterial Immunity (Supplementary Material)", SCIENCE, vol. 337, no. 6096, 28 juin 2012 (2012-06-28), XP055067747, US ISSN: 0036-8075, DOI: 10.1126/science.1225829
- PECINA A ET AL: "TARGETED STIMULATION OF MEIOTIC RECOMBINATION", CELL, CELL PRESS, US, vol. 111, no. 2, 18 octobre 2002 (2002-10-18), pages 173-184, XP001174111, ISSN: 0092-8674, DOI: 10.1016/S0092-8674(02)01002-4
- LAM ISABEL ET AL: "Mechanism and regulation of meiotic recombination initiation.", COLD SPRING HARBOR PERSPECTIVES IN BIOLOGY JAN 2015, vol. 7, no. 1, A016634, janvier 2015 (2015-01), pages 1-35, XP002751162, ISSN: 1943-0264, DOI: 10.1101/cshperspect.a016634

## Description

La présente invention relève du domaine des eucaryotes, plus particulièrement du domaine de la microbiologie. Elle concerne notamment un procédé pour améliorer ou modifier une souche de levure en induisant des recombinaisons méiotiques ciblées.

### ARRIERE-PLAN TECHNOLOGIQUE DE L'INVENTION

Les levures trouvent leur application dans des domaines industriels extrêmement variés. Du fait de l'innocuité d'un grand nombre d'espèces, les levures sont notamment utilisées dans l'industrie alimentaire comme agent de fermentation en boulangerie, en brasserie, vinification ou distillerie, ou sous forme d'extraits comme éléments nutritionnels ou agents de sapidité. Elles peuvent également trouver leur usage dans la production industrielle de bioéthanol ou de molécules d'intérêt telles que des vitamines, des antibiotiques, des vaccins, des enzymes ou des hormones stéroïdiennes, ou encore dans les procédés de dégradation des matières cellulosiques.

La diversité des applications industrielles des levures implique qu'il existe une demande constante pour des souches de levure présentant des caractéristiques améliorées ou, tout au moins, adaptées à une nouvelle utilisation ou de nouvelles conditions de culture.

Afin d'obtenir une souche présentant une caractéristique particulière, l'homme du métier peut utiliser la reproduction sexuée en croisant deux souches parentales présentant des caractéristiques intéressantes et en sélectionnant une souche hybride apportant la combinaison recherchée des caractéristiques parentales. Cette méthode est cependant aléatoire et l'étape de sélection peut engendrer des délais conséquents.

De manière alternative, il peut également modifier génétiquement la souche par une technique d'ADN recombinant. Cette modification peut néanmoins constituer un frein à son exploitation, que ce soit pour des raisons réglementaires, sanitaires ou environnementales.

Une troisième alternative consiste à provoquer un réassortiment des allèles d'origine paternelle et maternelle dans le génome, au cours de la recombinaison méiotique. La recombinaison méiotique est un échange d'ADN entre chromosomes homologues au cours de la méiose. Elle est initiée par la formation de cassures double-brin dans l'une ou l'autre des chromatides homologues, suivie de la réparation de ces cassures, utilisant comme matrice une chromatide du chromosome homologue. Les recombinaisons méiotiques ont cependant l'inconvénient d'être aléatoires et non-uniformes. En effet, les sites de coupures double-brin à l'origine de ces recombinaisons ne sont pas distribués de façon homogène dans le génome. On distingue ainsi des régions chromosomiques dites 'chaudes' où la fréquence de recombinaison est élevée, et des régions chromosomiques dites 'froides' où la fréquence de recombinaison peut être jusqu'à 100 fois plus faible.

Spo11 est la protéine catalysant les cassures double-brin au cours de la méiose. Elle agit sous forme de dimère en coopération avec de nombreux partenaires. A l'heure actuelle, les facteurs déterminant le choix des sites de coupure double-brin par Spo11 et ses partenaires restent encore mal compris.

Le contrôle de la formation des cassures double-brin et, de fait, des recombinaisons méiotiques, est crucial pour le développement de techniques d'ingénierie génétique. Il a récemment été montré qu'il est possible de modifier les sites de formation de cassures double-brin en fusionnant Spo11 avec le domaine de liaison à l'ADN de l'activateur de transcription Gal4 (Pecina et al, 2002 Cell, 111, 173-184,). La protéine de fusion Gal4-Spo11 permet d'introduire des cassures double-brin dans des régions chromosomiques dites 'froides', au niveau des sites de liaison de Gal4 à l'ADN.

Cependant, dans cette dernière approche, l'introduction de cassures double-brin est conditionnée par la présence de sites de liaison Gal4, il reste donc impossible d'induire des phénomènes de recombinaisons méiotiques ciblées indépendamment de sites de liaison spécifiques.

### RESUME DE L'INVENTION

L'objectif de la présente invention est de proposer un procédé permettant d'induire des recombinaisons méiotiques ciblées dans des cellules eucaryotes, de préférence des cellules de levure ou de plante, dans n'importe quelle région du génome, indépendamment de tout site de liaison connu, et notamment dans des régions chromosomiques dites 'froides'.

Ainsi, selon un premier aspect, la présente invention concerne un procédé pour induire des recombinaisons méiotiques ciblées dans une cellule eucaryote comprenant
- l'introduction dans ladite cellule :
   a) d'une protéine de fusion comprenant un domaine Cas9 et un domaine Spo11 ou d'un acide nucléique codant ladite protéine de fusion ; et
   b) d'un ou plusieurs ARN guides ou d'un ou plusieurs acides nucléiques codant lesdits ARN guides, lesdits ARN guides comprenant une structure ARN de fixation au domaine Cas9 de la protéine de fusion et une séquence complémentaire de la région chromosomique ciblée ; et
- l'induction de l'entrée en prophase I de méiose de ladite cellule.

La protéine de fusion peut comprendre en outre une séquence signal de localisation nucléaire.

De préférence, le domaine Cas9 de la protéine de fusion est une protéine Cas9 déficiente pour l'activité nucléase.

L'acide nucléique codant ladite protéine de fusion peut être placé sous le contrôle d'un promoteur constitutif, inductible ou spécifique de la méiose.

Un ou plusieurs ARN guides supplémentaires ciblant une ou plusieurs autres régions chromosomiques, ou acides nucléiques codant lesdits ARN guides supplémentaires, peuvent être introduits dans la cellule eucaryote.

De préférence, la cellule eucaryote est une levure. L'entrée en prophase I de la levure peut alors être induite par son transfert dans un milieu de sporulation.

Alternativement, la cellule eucaryote est une cellule de plante.

De préférence, l'introduction de la protéine de fusion, ou de l'acide nucléique codant pour celle-ci, et du ou des ARNg, ou du ou des acides nucléiques codant pour ceux-ci, dans ladite cellule est simultanée.

Alternativement, l'introduction de la protéine de fusion, ou de l'acide nucléique codant pour celle-ci, et du ou des ARNg, ou du ou des acides nucléiques codant pour ceux-ci, dans ladite cellule est séquentielle.

L'introduction de l'acide nucléique codant pour la protéine de fusion et du ou des acides nucléiques codant pour le ou les ARNg dans ladite cellule peut également être obtenue par croisement de deux cellules dans lesquelles ont respectivement été introduits l'acide nucléique codant pour la protéine de fusion et le ou les acides nucléiques codant pour le ou les ARNg.

La présente invention concerne aussi, selon un deuxième aspect, une protéine de fusion telle que définie dans le procédé ci-dessus.

Selon un troisième aspect, la présente invention concerne encore un acide nucléique codant pour la protéine de fusion définie ci-dessus.

La présente invention concerne également, selon un quatrième aspect, une cassette d'expression ou un vecteur comprenant un acide nucléique tel que défini ci-dessus.

De préférence, le vecteur est un plasmide comprenant une origine de réplication bactérienne, une cassette d'expression comprenant un acide nucléique tel que défini ci-dessus, un ou plusieurs marqueurs de sélection, et/ou une ou plusieurs séquences permettant l'insertion ciblée du vecteur, de la cassette d'expression ou de l'acide nucléique dans le génome de la cellule hôte. En particulier, le plasmide comprend une origine de réplication bactérienne, de préférence l'origine ColE1, une cassette d'expression comprenant un acide nucléique tel que défini ci-dessus sous le contrôle d'un promoteur, de préférence le promoteur ADH1, un terminateur, de préférence le terminateur ADH1, un ou plusieurs marqueurs de sélection, de préférence des marqueurs de résistance tels que le gène de résistance à la kanamycine ou à l'ampicilline, une ou plusieurs séquences permettant l'insertion ciblée du vecteur, de la cassette d'expression ou de l'acide nucléique dans le génome de la cellule hôte, de préférence au niveau du locus *TRP1* du génome d'une levure. De manière préférée, le plasmide comprend, ou consiste en, une séquence nucléotidique sélectionnée parmi la SEQ ID NO : 1 et la SEQ ID NO : 2.

Selon un cinquième aspect, la présente invention concerne également une cellule hôte comprenant une protéine de fusion, un acide nucléique, une cassette ou un vecteur tel que défini ci-dessus.

De préférence, la cellule hôte est une cellule eucaryote, de manière encore préférée une cellule de levure, de plante, de champignon ou une cellule animale, et de manière tout particulièrement préférée, la cellule hôte est une cellule de plante ou une cellule de levure.

De préférence, la cellule hôte est une cellule de levure, de manière encore préférée une levure sélectionnée dans le groupe constitué de *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus, Saccharomyces pastorianus* (aussi nommée *Saccharomyces carlsbergensis*)*,* et des hybrides obtenus à partir d'au moins une souche appartenant à l'une de ces espèces, et de manière tout particulièrement préférée la cellule hôte est *Saccharomyces cerevisiae.*

Alternativement, la cellule hôte est une cellule de plante, de manière encore préférée une cellule de plante sélectionnée dans le groupe constitué du riz, du blé, du soja, du maïs, de la tomate, *d'Arabidopsis thaliana,* de l'orge, du colza, du coton, de la canne à sucre, et de la betterave, et de manière tout particulièrement préférée ladite cellule hôte est une cellule de riz.

La présente invention concerne encore, dans un sixième aspect, un procédé pour générer des variants d'un organisme eucaryote, à l'exception de l'homme, comprenant :
- l'introduction dans une cellule dudit organisme:
   a) d'une protéine de fusion comprenant un domaine Cas9 et un domaine Spo11, ou d'un acide nucléique codant ladite protéine de fusion ; et
   b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- la genèse d'un variant de l'organisme à partir de ladite cellule recombinée.

De préférence, l'organisme eucaryote est une levure ou une plante, de manière encore préférée une levure, notamment une souche de levure d'intérêt industriel.

Dans un septième aspect, la présente invention concerne également un procédé pour identifier ou localiser l'information génétique codant pour une caractéristique d'intérêt dans un génome de cellule eucaryote comprenant :
- l'introduction dans la cellule eucaryote:
   a) d'une protéine de fusion comprenant un domaine Cas9 et un domaine Spo11, ou d'un acide nucléique codant ladite protéine de fusion ; et
   b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- l'analyse des génotypes et phénotypes des cellules recombinées afin d'identifier ou de localiser l'information génétique codant pour la caractéristique d'intérêt.

De préférence, la cellule eucaryote est une levure ou une plante, de manière encore préférée une levure, notamment une souche de levure d'intérêt industriel.

De préférence, la caractéristique d'intérêt est un caractère quantitatif d'intérêt (QTL).

La présente invention concerne encore, dans un huitième aspect, une trousse comprenant une protéine de fusion, un acide nucléique, une cassette, un vecteur ou une cellule hôte tel que défini ci-dessus.

Dans un neuvième aspect, la présente invention concerne enfin l'utilisation d'une trousse telle que définie ci-dessus pour mettre en œuvre un procédé tel que défini ci-dessus, en particulier pour (i) induire des recombinaisons méiotiques ciblées dans une cellule eucaryote, (ii) générer des variants d'un organisme eucaryote, et/ou (iii) identifier ou localiser l'information génétique codant pour une caractéristique d'intérêt dans un génome de cellule eucaryote.

### BREVE DESCRIPTION DES DESSINS

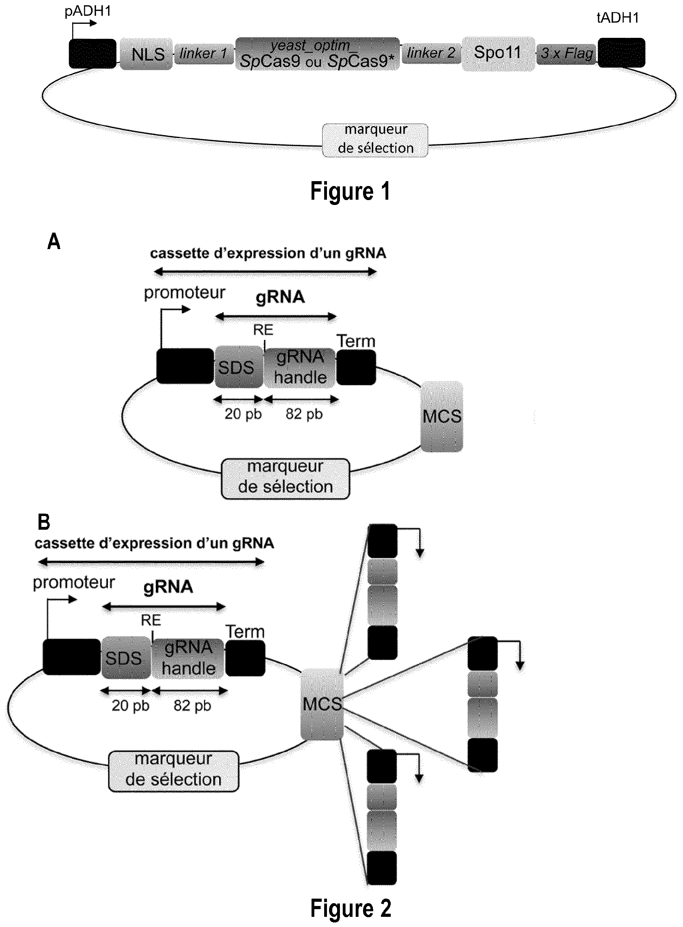

**Figure 1** **:** Schéma représentant les plasmides P1 (SEQ ID NO : 1) et P2 (SEQ ID NO : 2) de la fusion *Sp*Cas9-Spo11 ou *Sp*Cas9*-Spo11. P1 et P2 codent respectivement pour l'expression de NLS-*Sp*Cas9-Spo11 et NLS-*Sp*Cas9*-Spo11 chez la levure. Les blocs noirs représentent le promoteur constitutif ADH1 (pADH1) et le terminateur ADH1 (tADH1). La flèche indique l'orientation de la transcription sous le contrôle du promoteur ADH1.
**Figure 2** **:** Schéma représentant les plasmides d'expression d'ARNg dans des cellules de levure. (A) Schéma du plasmide contenant une unique cassette d'expression d'ARNg afin de cibler une seule région du génome de la levure. RE indique le site de restriction où la séquence déterminant la spécificité de l'ARNg (SDS) est insérée par le procédé de Gibson. Le promoteur et le terminateur (Term) sont dépendants de l'ARN polymérase III. La flèche sur le promoteur indique le sens de la transcription de la séquence. La cassette d'expression d'un ARNg contient un ARNg encadré par un promoteur et un terminateur. (B) Schéma du plasmide contenant plusieurs cassettes d'expression d'ARNg afin de cibler de multiples régions du génome de la levure (ciblage multiplexé). Les différentes cassettes d'ARNg se distinguent par leur séquence déterminant leur spécificité (SDS). Elles ont été introduites successivement dans le site de clonages multiples (MCS) par les techniques classiques de clonage/ligature.

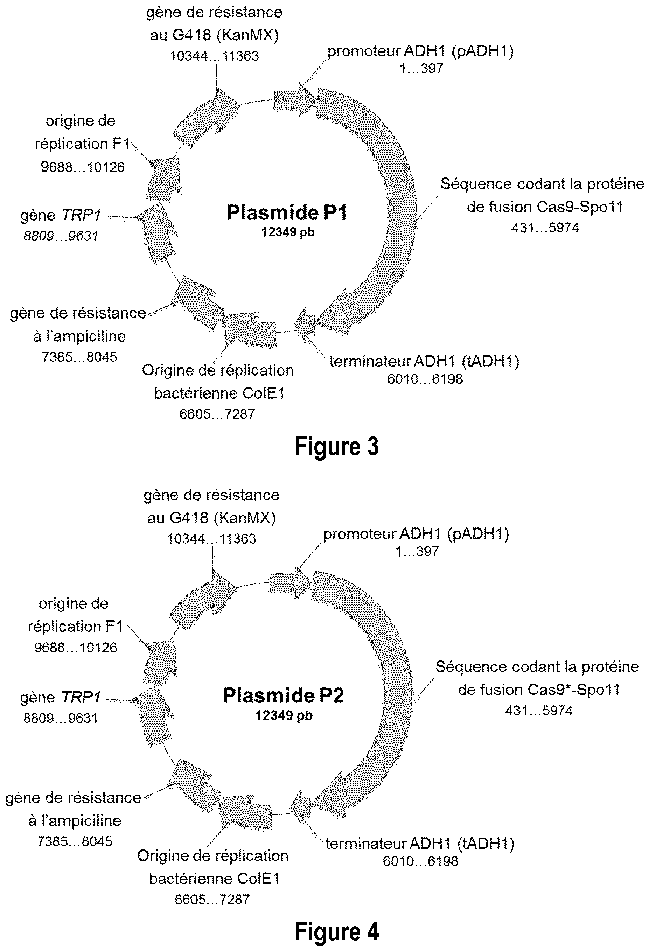

**Figure 3** **:** Schéma représentant le plasmide P1 (SEQ ID NO : 1).
**Figure 4** **:** Schéma représentant le plasmide P2 (SEQ ID NO : 2).

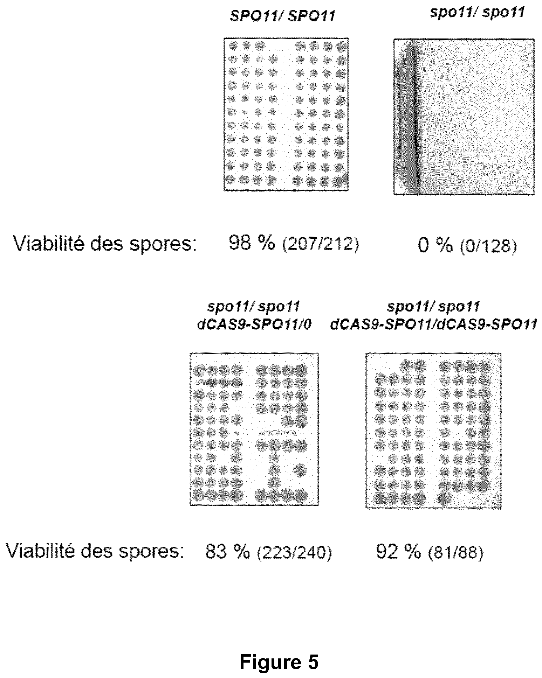

**Figure 5** **:** Viabilité des spores issues de la sporulation des souches exprimant ou non la protéine de fusion SpCas9*-Spo11. Croissance des spores issues de la méiose des souches diploïdes *SPO11*/*SPO11* (ORD7339), *spo11*/*spo11* (AND2822), *spo11*/*spo11 dCAS9-SPO11*/*0* (AND2820) et *spo11*/*spo11 dCAS9-SPO11*/*dCAS9-SPO11* (AND2823).

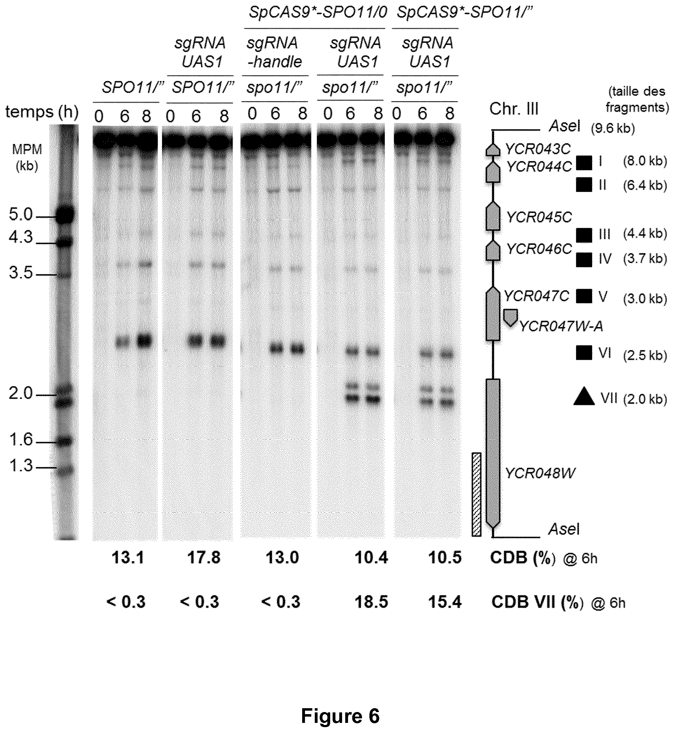

**Figure 6** **:** Ciblage des CDBs méiotiques par la protéine de fusion *Sp*Cas9*-Spo11 et un ARN guide spécifique de la région YCR048W. Sur la droite du gel, une carte indique la position des gènes (régions codantes, flèches grises) et la position de la sonde. Les carrés noirs indiquent les sites de CDBs naturels. Le triangle noir indique les sites de CDBs ciblés par l'ARN guide UAS1-YCR048W. Le pourcentage de CDBs correspond au ratio entre l'intensité du signal du fragment concerné par rapport au signal total de la piste.

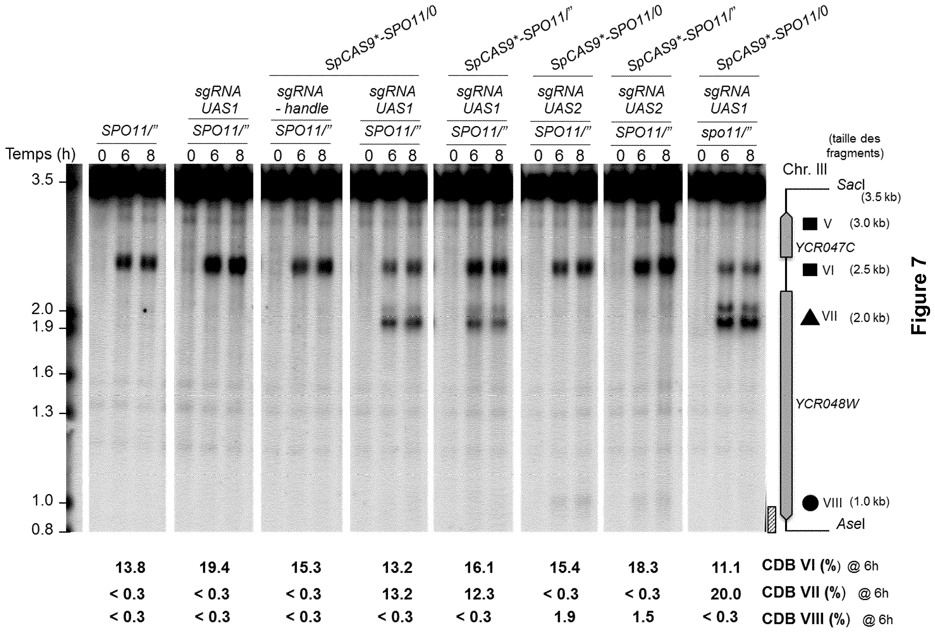

**Figure 7** **:** Ciblage des CDBs méiotiques par la protéine de fusion *Sp*Cas9*-Spo11 et de deux ARN guides spécifiques de la région YCR048W. Sur la droite du gel, une carte indique la position des gènes (régions codantes, flèches grises) et la position de la sonde. Les carrés noirs indiquent les sites de CDBs naturels. Le triangle noir indique les sites de CDBs ciblés par l'ARN guide UAS1-YCR048W. Le rond noir indique les sites de CDBs ciblés par l'ARN guide UAS2-YCR048W. Le pourcentage de CDBs correspond au ratio entre l'intensité du signal du fragment concerné par rapport au signal total de la piste.

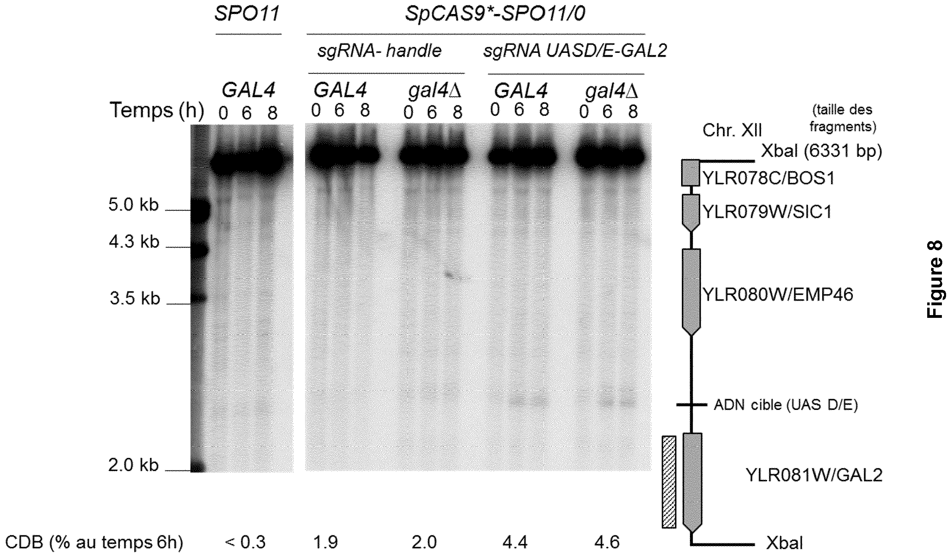

**Figure 8** **:** Ciblage des CDBs méiotiques par la protéine de fusion *Sp*Cas9*-Spo11 et un ARN guide spécifique de la région GAL2. Sur la droite du gel, une carte indique la position des gènes (régions codantes, flèches grises) et la position de la sonde. Le pourcentage de CDBs correspond au ratio entre l'intensité du signal du fragment concerné par rapport au signal total de la piste.

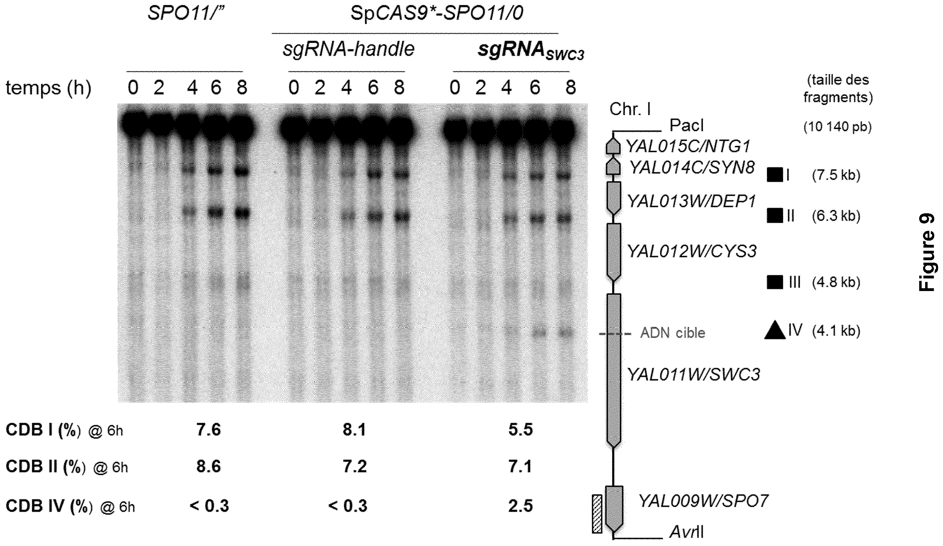

**Figure 9** **:** Ciblage des CDBs méiotiques par la protéine de fusion *Sp*Cas9*-Spo11 et un ARN guide spécifique de la région SWC3. Sur la droite du gel, une carte indique la position des gènes (régions codantes, flèches grises) et la position de la sonde (rectangle hachuré). Le pourcentage de CDBs correspond au ratio entre l'intensité du signal du fragment concerné par rapport au signal total de la piste.

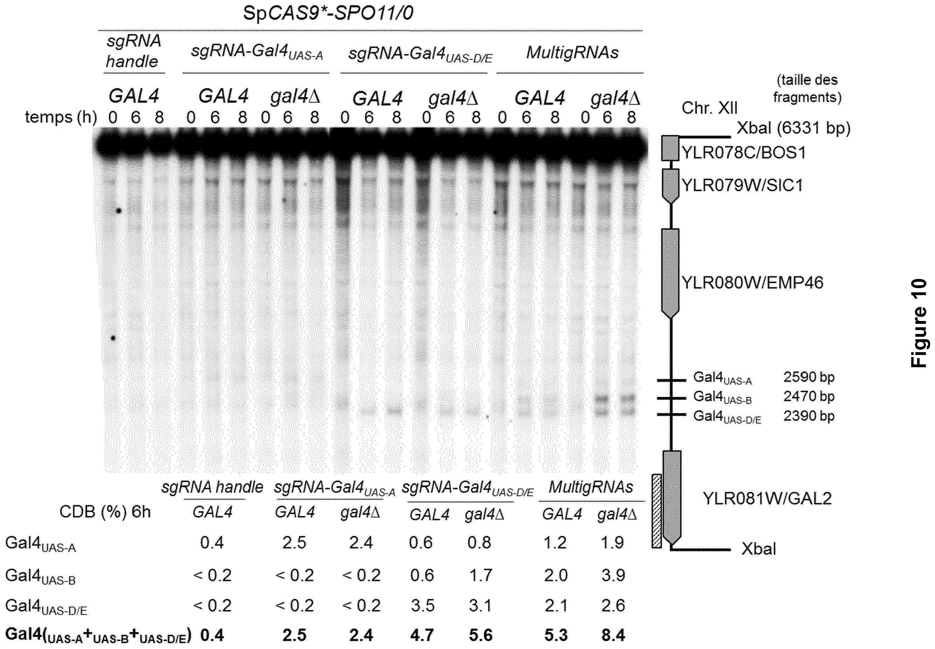

**Figure 10** **:** Ciblage multiplexé des CDBs méiotiques par la protéine de fusion *Sp*Cas9*-Spo11 et plusieurs ARNs guide spécifiques de la région GAL2. Sur la droite du gel, une carte indique la position des gènes (régions codantes, flèches grises) et la position de la sonde (rectangle hachuré). Le pourcentage de CDBs correspond au ratio entre l'intensité du signal du fragment concerné par rapport au signal total de la piste.

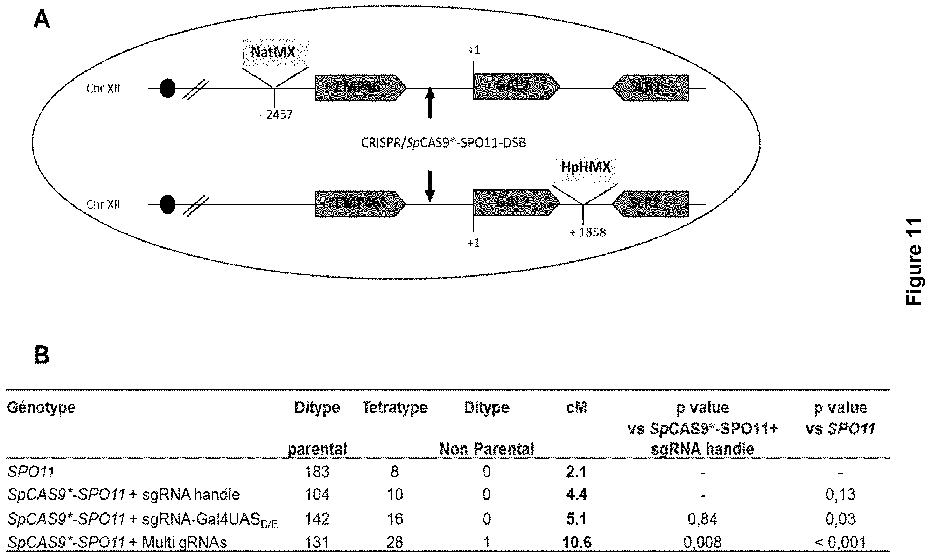

**Figure 11** **:** Stimulation de la recombinaison méiotique par la protéine *Sp*Cas9*-SPO11 et un ARN guide dans la région cible GAL2. (A), Schéma du test génétique permettant la détection des recombinants au site GAL2. (B), Test de recombinaison génétique au locus *GAL2.*

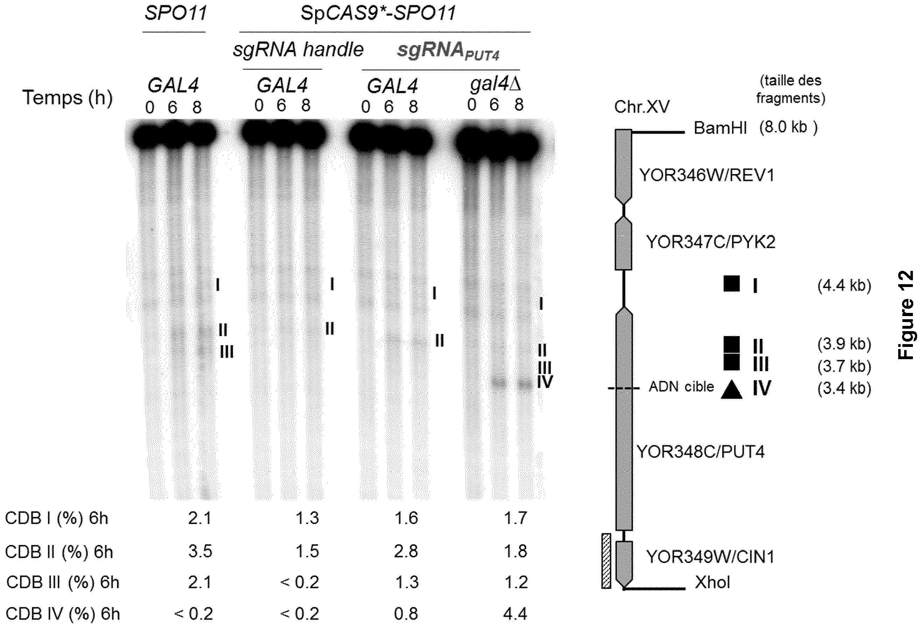

**Figure 12** **:** Ciblage des CDBs méiotiques par la protéine *Sp*Cas9*-Spo11 et un ARN guide spécifique de la séquence codante du gène PUT4. Sur la droite du gel, une carte indique la position des gènes (régions codantes, flèches grises) et la position de la sonde (rectangle hachuré). Le pourcentage de CDBs correspond au ratio entre l'intensité du signal du fragment concerné par rapport au signal total de la piste.

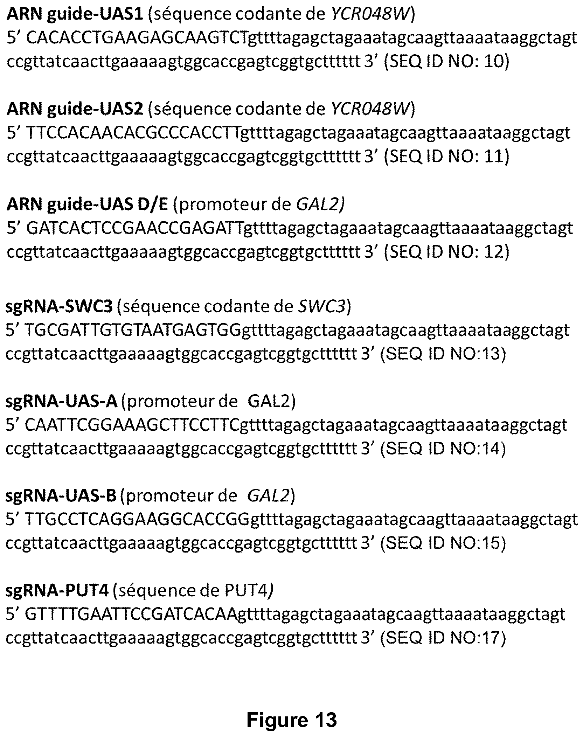

**Figure 13** **:** Séquence des ARNg utilisés. En caractères majuscules et minuscules sont indiquées respectivement la séquence qui détermine la spécificité de l'ARNg (« specificity-determining sequence », longueur de 20 nucléotides) et la séquence constituant la structure de l'ARNg (« handle », longueur de 82 nucléotides).

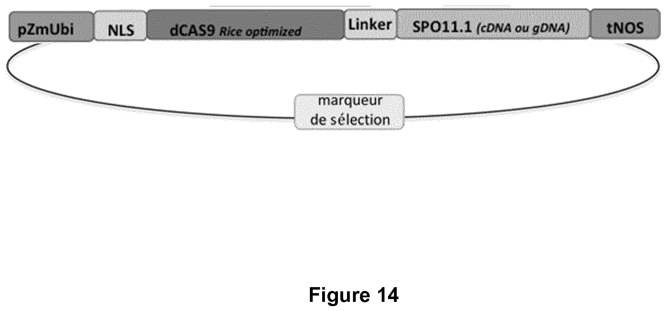

**Figure 14** **:** Schéma de la construction permettant l'expression de la protéine de fusion dCas9-Spo11 chez le riz. pZmUbi et tNOS correspondent, respectivement, au promoteur et au terminateur utilisés dans cette construction.

### DESCRIPTION DETAILLEE DE L'INVENTION

Le système CRISPR-Cas9 (Clustered Regularly Interspaced Short Palindromic Repeats) est un système de défense bactérien contre les ADN étrangers. Ce système repose essentiellement sur l'association d'une protéine Cas9 et d'un ARN « guide » (ARNg ou sgRNA) responsable de la spécificité du site de clivage. Il permet la réalisation de cassures d'ADN double-brin (CDB) aux sites ciblés par le système CRISPR/Cas9. Ce système a déjà été utilisé pour faire de l'ingénierie ciblée du génome dans des cellules eucaryotes (cf. par exemple la demande de brevet EP2764103), notamment des cellules humaines (Cong L et al.,2013, Science 339(6121):819-823 ; Mali P et al.,2013, Science, 339(6121):823-826 ; Cho SW et al., 2013, Nature Biotechnology 31(3):230-232), de rat (Li D, et al., 2013, Nature Biotechnology, 31(8):681-683 ; WO 2014/089290), de souris (Wang H et al., 2013, Cell, 153(4):910-918), de lapin (Yang D et al., 2014, Journal of Molecular Cell Biology, 6(1):97-99), de grenouille (Nakayama T et al., 2013, Genesis, 51(12):835-843), de poisson (Hwang WY et al., 2013, Nature Biotechnology, 31(3):227-229), de plantes (Shan Q et al., 2013, Nature Biotechnology, 31(8):686-688 ; Jiang W et al., 2013, Nucleic Acids Research, 41(20):el88.), de drosophile (Yu Z et al, 2013, Genetics, 195(1):289-291), de nématode (Friedland AE et al., 2013, Nature Methods, 10(8):741-743), de levure (DiCarlo J, et al., 2013, Genome engineering in Saccharomyces cerevisiae using CRISPR-Cas systems. Nucleic Acids Research 41(7):4336-4343.), mais aussi des cellules bactériennes (Jiang W et al., 2013, Nature Biotechnology, 31(3):233-239). En revanche, ce système n'a jamais été utilisé pour cibler des sites de recombinaison méiotique dans quelque organisme que ce soit.

Les inventeurs ont démontré qu'il était possible de modifier le système CRISPR-Cas9 afin d'induire des recombinaisons méiotiques ciblées dans une cellule eucaryote, et en particulier dans une levure. Ils ont en effet montré que l'expression combinée d'une protéine de fusion Spoll-Cas9 et d'un ou plusieurs ARN guides permettait de cibler l'action de la transestérase Spo11 qui est responsable des cassures double-brin durant la méiose. La réparation de ces cassures en utilisant comme matrice une chromatide du chromosome homologue induit les phénomènes de recombinaison recherchés.

Ainsi, la présente invention concerne un procédé pour induire des recombinaisons méiotiques ciblées dans une cellule eucaryote comprenant
- l'introduction dans ladite cellule :
   a) d'une protéine de fusion comprenant un domaine Cas 9 et un domaine Spo11, ou d'un acide nucléique codant ladite protéine de fusion ; et
   b) d'un ou plusieurs ARN guides ou d'un ou plusieurs acides nucléiques codant lesdits ARN guides, lesdits ARN guides comprenant une structure ARN de fixation au domaine Cas9 de la protéine de fusion et une séquence complémentaire de la région chromosomique ciblée ; et
- l'induction de l'entrée en prophase I de méiose de ladite cellule.

Tel qu'utilisé ici, le terme « cellule eucaryote », se réfère à une cellule de levure, de plante, de champignon ou une cellule animale, en particulier une cellule de mammifère telle qu'une cellule de souris ou de rat, ou une cellule d'insecte. La cellule eucaryote est non-humaine.

Selon un mode de réalisation particulier, la cellule eucaryote est une cellule de levure, en particulier une levure d'intérêt industriel. Des exemples de levures d'intérêt comprennent, sans y être limités, les levures du genre *Saccharomyces sensu stricto, Schizosaccharomyces, Yarrowia, Hansenula, Kluyveromyces, Pichia* ou *Candida,* ainsi que les hybrides obtenus à partir d'une souche appartenant à l'un de ces genres.

De préférence, la levure d'intérêt appartient au genre *Saccharomyces.* Elle peut notamment appartenir à une espèce sélectionnée dans le groupe constitué de *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus* and *Saccharomyces pastorianus* (aussi nommée *Saccharomyces carlsbergensis),* ou est un hybride obtenu à partir d'une souche appartenant à l'une de ces espèces tel que par exemple un hybride *S. cerevisiae*/*S. paradoxus* ou un hybride *S. cerevisiae*/*S. uvarum.*

Selon un autre mode de réalisation particulier, la cellule eucaryote est une cellule de champignon, en particulier une cellule de champignon d'intérêt industriel. Des exemples de champignons comprennent, sans y être limités, les cellules de champignons filamenteux. Les champignons filamenteux incluent les champignons appartenant aux subdivisions *Eumycota* et *Oomycota.* Les cellules de champignons filamenteux peuvent être choisies dans le groupe constitué des cellules de *Trichoderma, Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium* ou *Trametes.*

Selon encore un autre mode de réalisation particulier, la cellule eucaryote est une cellule de plante, en particulier une cellule de plante d'intérêt agronomique. Des exemples de plantes comprennent, sans y être limités, le riz, le blé, le soja, le maïs, la tomate, *Arabidopsis thaliana,* l'orge, le colza, le coton, la canne à sucre, et la betterave. Selon un mode préféré, la cellule eucaryote est une cellule de riz.

De préférence, la cellule eucaryote est hétérozygote pour le ou les gène(s) ciblé(s) par le ou les ARN guide(s).

Tel qu'utilisé ici, le terme « protéine de fusion » se réfère à une protéine chimérique comprenant au moins deux domaines issus de la combinaison de différentes protéines ou fragments de protéines. L'acide nucléique codant cette protéine est obtenu par recombinaison des régions codant les protéines ou fragments de protéine de façon à ce que ceux-ci soient en phase et transcrits sur le même ARNm. Les différents domaines de la protéine de fusion peuvent être directement adjacents ou être séparés par des séquences de liaison (ou linker) qui introduisent une certaine flexibilité structurale dans la construction.

La protéine de fusion utilisée dans la présente invention comprend un domaine Cas9 et un domaine Spo11.

Le domaine Cas9 est le domaine de la protéine de fusion qui est capable d'interagir avec les ARN guides et de cibler l'activité nucléase du domaine Spo11 vers une région chromosomique donnée. Le domaine Cas9 peut être constitué d'une protéine Cas9 (aussi appelée Csnl ou Csx12), sauvage ou modifiée, ou d'un fragment de cette protéine capable d'interagir avec les ARN guides. La protéine Cas9 peut notamment être modifiée pour moduler son activité enzymatique. Ainsi, l'activité nucléase de la protéine Cas9 peut être modifiée ou inactivée. La protéine Cas9 peut également être tronquée afin de supprimer les domaines de la protéine non-essentiels aux fonctions de la protéine de fusion, en particulier les domaines de la protéine Cas9 qui ne sont pas nécessaires à l'interaction avec les ARN guides.

La protéine Cas9 ou le fragment de celle-ci tel qu'utilisé dans la présente invention, peut être obtenu à partir de toute protéine Cas9 connue (Makarova et al, 2008, Nat. Rev. Microbiol., 9, pp. 466-477). Des exemples de protéines Cas9 utilisables dans la présente invention incluent, sans y être limités, les protéines Cas9 de *Streptococcus pyogenes, Streptococcus thermophilus, Streptococcus sp., Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius,Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus,* ou *Acaryochloris marina.* D'autres protéines Cas9 utilisables dans la présente invention sont également décrites dans l'article de Makarova et al. (Makarova et al, 2008, Nat. Rev. Microbiol., 9, pp. 466-477). De préférence, le domaine Cas9 comprend, ou consiste en, la protéine Cas9 de *Streptococcus pyogenes* (Numéro d'entrée NCBI : WP_010922251.1, SEQ ID NO : 8) ou un fragment de celle-ci capable d'interagir avec les ARN guides.

Selon un mode de réalisation particulier, le domaine Cas9 est constitué d'une protéine Cas9 entière, de préférence la protéine Cas9 de *Streptococcus pyogenes.*

De manière générale, les protéines Cas9 comprennent deux domaines nucléases : un domaine apparenté à un domaine RuvC et un domaine apparenté à un domaine HNH. Ces deux domaines coopèrent pour créer des cassures double-brin de l'ADN (Jinek et al, Science, 337 : 816-821). Chacun de ces domaines nucléases peut être inactivé par délétion, insertion ou substitution en recourant à des techniques bien connues de l'homme du métier comme la mutagénèse dirigée, la mutagénèse par PCR ou la synthèse totale de gènes. Ainsi, le domaine RuvC peut être inactivé par exemple par la substitution D10A et le domaine HNH peut être inactivé par exemple par la substitution H840A (Jinek et al, Science, 337 : 816-821), les positions indiquées étant celles de la SEQ ID NO : 8.

Dans les séquences peptidiques décrites dans ce document, les acides aminés sont représentés par leur code à une lettre selon la nomenclature suivante : C : cystéine; D : acide aspartique; E : acide glutamique; F : phénylalanine; G : glycine; H : histidine; I : isoleucine; K : lysine; L : leucine ; M : méthionine ; N : asparagine ; P : proline ; Q : glutamine ; R : arginine ; S : sérine ; T : thréonine ; V : valine ; W : tryptophane et Y : tyrosine.

Selon un mode de réalisation, le domaine Cas9 est déficient pour au moins une activité nucléase. Ce domaine peut être obtenu en inactivant au moins l'un des domaines nucléases de la protéine Cas9 tels que décrits ci-dessus.

Selon un mode de réalisation particulier, le domaine Cas9 comprend, ou consiste en, une protéine Cas9 ou un fragment de protéine Cas9, dépourvu d'activité nucléase (aussi appelé Cas9* ou dCas9). Cette forme catalytiquement inactive peut être obtenue en inactivant les deux domaines nucléases de la protéine Cas9 comme mentionné ci-dessus, par exemple en introduisant les deux mutations ponctuelles substituant l'aspartate 10 et l'histidine 840 par des alanines.

Selon un mode de réalisation préféré, le domaine Cas9 comprend, ou consiste en, une protéine Cas9, de préférence la protéine Cas9 de *Streptococcus pyogenes* (spCas9), dépourvue d'activité nucléase (spCas9*).

Selon un mode de réalisation particulier, le domaine Cas9 comprend, ou consiste en, la séquence présentée en SEQ ID NO : 8 dans laquelle l'aspartate en position 10 et l'histidine en position 840 ont été substitués par des alanines.

Spo11 est une protéine apparentée à la sous-unité catalytique A d'une topo-isomérase de type II présente chez les archaebactéries (Bergerat et al, Nature, vol. 386, pp 414-7). Elle catalyse les cassures double-brin de l'ADN initiant les recombinaisons méiotiques. C'est une protéine très conservée pour laquelle il existe des homologues chez tous les eucaryotes. Spo11 est active sous forme d'un dimère formé de deux sous-unités, dont chacune clive un brin d'ADN. Bien qu'essentielle, Spo11 n'agit pas seule pour générer des cassures double-brin au cours de la méiose. Chez la levure *S. cerevisiae,* par exemple, elle coopère avec les protéines, Rec102, Recl03/Skl8, Rec104, Rec114, Merl, Mer2/Rec107, Mei4, Mre2/Nam8, Mrell, Rad50, Xrs2/Nbsl, Hop1, Red1, Mek1, Set1 et Spp1 ainsi qu'avec d'autres partenaires décrits dans les articles de Keeney et al. (2001 Curr. Top. Dev. Biol, 52, pp. 1-53), Smith et al. (Curr. Opin. Genet. Dev, 1998, 8, pp. 200-211) et Acquaviva et al. (2013 Science, 339, pp. 215-8). Il a cependant été récemment démontré que le ciblage de Spo11 à un site donné est suffisant pour déclencher le processus de recombinaison méiotique (Pecina et al, 2002 Cell, 111, 173-184). Il est à noter que plusieurs homologues de la protéine Spo11 peuvent co-exister dans une même cellule, notamment chez les plantes. De préférence, la protéine Spo11 est une des protéines Spo11 de la cellule eucaryote d'intérêt.

Le domaine Spo11 de la protéine de fusion Cas9-Spo11 est en général le domaine responsable des cassures double-brin. Ce domaine peut être constitué d'une protéine Spo11 ou d'un fragment de celle-ci capable d'induire des cassures double brin de l'ADN.

La protéine Spo11 ou le fragment de celle-ci tel qu'utilisé dans la présente invention, peut être obtenu à partir de toute protéine Spo11 connue telle que la protéine Spo11 de *Saccharomyces cerevisiae* (Gene ID: 856364, Numéro d'entrée NCBI : NP_011841 (SEQ ID NO : 9) Esposito and Esposito, Genetics, 1969, 61, pp. 79-89), la protéine AtSpoll-1 et AtSpoll-2 d'*Arabidopsis thaliana* (Grêlon M. et al, 2001, Embo J., 20, pp. 589-600), la protéine murine mSpoll (Baudat F et al, Molecular Cell, 2000, 6, pp. 989-998), la protéine Spo11 de *C*. *elegans* ou encore la protéine Spo11 de drosophile meiW68 (McKim et al, 1998, Genes Dev, 12(18), pp. 2932-42). Bien entendu, ces exemples ne sont pas limitatifs et toute protéine Spo11 connue peut être utilisée dans le procédé selon l'invention.

Selon un mode de réalisation préféré, le domaine Spo11 comprend, ou consiste en, une protéine Spo11, de préférence une protéine Spo11 de *Saccharomyces cerevisiae,* telle que par exemple la protéine de séquence SEQ ID NO : 9.

Selon un mode de réalisation particulier, le domaine Spo11 est déficient pour l'activité nucléase. En particulier, le domaine Spo11 peut comprendre, ou consister en, la protéine mutante Spo11-Y135F, une protéine mutante incapable d'induire des cassures double-brin de l'ADN (Neale MJ, 2002, Molecular Cell, 9, 835-846). La position indiquée est celle de la SEQ ID NO : 9.

La capacité de la protéine de fusion selon l'invention à induire des cassures double brin de l'ADN, peut provenir du domaine Cas9 ou du domaine Spo11. Ainsi, la protéine de fusion comprend au moins un domaine, Cas9 ou Spo11, présentant une activité nucléase, de préférence le domaine Spo11.

Selon un mode de réalisation particulier, plusieurs protéines de fusion selon l'invention comprenant différents domaines Spo11 peuvent être introduites dans la même cellule. En particulier, lorsque plusieurs homologues de Spo11 existent dans la cellule eucaryote d'intérêt, les différentes protéines de fusion peuvent comprendre chacune un homologue différent de Spo11. A titre d'exemple, deux protéines de fusion selon l'invention comprenant respectivement des domaines Spo11-1 et Spo11-2 d'*Arabidopsis thaliana* peuvent être introduits dans la même cellule, de préférence dans la même cellule *d'Arabidopsis thaliana.* Toujours à titre d'exemple, une ou plusieurs protéines de fusion selon l'invention comprenant des domaines Spo11-1, Spo11-2, Spo11-3 et/ou Spo11-4 de riz peuvent être introduits dans la même cellule, de préférence dans la même cellule de riz. De nombreux homologues de Spo11 ont été identifiés dans différentes espèces, en particulier dans les espèces végétales (Sprink T et Hartung F, Frontiers in Plant Science, 2014, Vol. 5, article 214, doi: 10.3389/fpls.2014.00214 ; Shingu Y et al, BMC Mol Biol, 2012, doi: 10.1186/1471-2199-13-1). L'homme du métier peut aisément identifier les homologues de Spo11 dans une espèce donnée, notamment au moyen de techniques bien connues de bio-informatique.

La protéine de fusion selon l'invention comprend un domaine Spo11 et un domaine Cas9 tels que définis ci-dessus.

Selon un mode de réalisation, le domaine Spo11 est du côté N-terminal et le domaine Cas9 du côté C-terminal de la protéine de fusion. Selon un autre mode de réalisation, le domaine Spo11 est du côté C-terminal et le domaine Cas9 du côté N-terminal de la protéine de fusion.

La protéine de fusion peut également comprendre une séquence signal de localisation nucléaire (SLN). Les séquences SLN sont bien connues de l'homme du métier et comprennent en général une courte séquence d'acides aminés basiques. A titre d'exemple, la séquence SLN peut comprendre la séquence PKKKRKV (SEQ ID NO : 3). La séquence SLN peut être présente à l'extrémité N-terminale, C-terminale ou dans une région interne de la protéine de fusion, de préférence à l'extrémité N-terminale de la protéine de fusion.

La protéine de fusion peut également comprendre un domaine supplémentaire de pénétration dans la cellule, c'est-à-dire un domaine facilitant l'entrée de la protéine de fusion dans la cellule. Ce type de domaine est bien connu de l'homme du métier et peut comprendre par exemple une séquence peptidique de pénétration dérivée de la protéine TAT du VIH-1 telle que GRKKRRQRRRPPQPKKKRKV (SEQ ID NO : 4), dérivée de la séquence TLM du virus de l'hépatite B humaine telle que PLSSIFSRIGDPPKKKRKV (SEQ ID NO : 5), ou une séquence peptidique polyarginine. Ce domaine de pénétration dans la cellule peut être présent à l'extrémité N-terminale, C-terminale ou être à l'intérieur de la protéine de fusion, de préférence à l'extrémité N-terminale.

La protéine de fusion peut comprendre en outre une ou des séquences de liaison (linkers) entre les domaines Cas9 et Spo11, et optionnellement entre ces domaines et les autres domaines de la protéine tels que la séquence signal de localisation nucléaire ou le domaine de pénétration dans la cellule. La longueur de ces séquences de liaison est aisément ajustable par l'homme du métier. En général, ces séquences comprennent entre 10 et 20 acides aminés, de préférence environ 15 acides aminés et de manière encore préférée 12 acides aminés. Les séquences de liaison entre les différents domaines peuvent être de longueurs identiques ou différentes.

Selon un mode de réalisation particulier, la protéine de fusion comprend, ou consiste en, successivement, depuis l'extrémité N-terminale jusqu'à l'extrémité C-terminale : un signal de localisation nucléaire, une première séquence de liaison (linkerl), un domaine Cas9, une seconde séquence de liaison (linker2) et un domaine Spo11.

Selon un autre mode de réalisation particulier, la protéine de fusion comprend, ou consiste en, successivement, depuis l'extrémité N-terminale jusqu'à l'extrémité C-terminale : un signal de localisation nucléaire, une première séquence de liaison (linkerl), un domaine Spo11, une seconde séquence de liaison (linker2) et un domaine Cas9.

La protéine de fusion peut en outre comprendre une étiquette (ou tag) qui est une séquence définie d'acides aminés. Cette étiquette peut notamment être utilisée afin de détecter l'expression de la protéine de fusion, d'identifier les protéines interagissant avec la protéine de fusion ou de caractériser les sites de fixation de la protéine de fusion dans le génome. La détection de l'étiquette attachée à la protéine de fusion peut être réalisée avec un anticorps spécifique de ladite étiquette ou toute autre technique bien connu de l'homme du métier. L'identification des protéines interagissant avec la protéine de fusion peut être réalisée, par exemple, par des techniques de co-immunoprécipitation. La caractérisation des sites de fixation de la protéine de fusion dans le génome peut être réalisée, par exemple, par des techniques d'immunoprécipitation, d'immunoprécipitation de la chromatine couplée à une PCR quantitative en temps réel (ChIP-qPCR), d'immunoprécipitation de la chromatine couplée à des techniques de séquençage (ChIP-Seq), de cartographie à l'aide d'oligonucléotides (oligo mapping) ou toute autre technique bien connue de l'homme du métier.

Cette étiquette peut être présente à l'extrémité N-terminale de la protéine de fusion, à l'extrémité C-terminale de la protéine de fusion ou en position non-terminale dans la protéine de fusion. De préférence, l'étiquette est présente à l'extrémité C-terminale de la protéine de fusion. La protéine de fusion peut comprendre une ou plusieurs étiquettes, identiques ou différentes.

Les étiquettes, telles qu'utilisées dans la présente invention, peuvent être choisies parmi les nombreuses étiquettes bien connues de l'homme du métier. En particulier, les étiquettes utilisées dans la présente invention peuvent être des étiquettes peptidiques et/ou des étiquettes protéiques. De préférence, les étiquettes utilisées dans la présente invention sont des étiquettes peptidiques. Des exemples d'étiquettes peptidiques utilisables dans la présente invention incluent, sans y être limités, les étiquettes formées de répétitions d'au moins six Histidines (His), en particulier les étiquettes formées de six ou huit histidines, ainsi que les étiquettes Flag, polyglutamates, hémagglutinine (HA), calmoduline, Strep, E-tag, myc, V5, Xpress, VSV, S-tag, Avi, SBP, Softag 1, Softag 2, Softag 3, isopetag, SpyTag, tétracystéines et des combinaisons de celles-ci. Des exemples d'étiquettes protéiques utilisables dans la présente invention incluent, sans y être limités, les étiquettes Glutathion-S-Transférase (GST), protéine A de *Staphlococcus aureus,* Nus A, protéine de liaison à la chitine (CBP), thiorédoxine, protéine de liaison au maltose (MBP), protéines transporteuses de biotine carboxylées (BCCP), fragment constant des immunoglobulines (Fc), les étiquettes comprenant une protéine fluorescente telles que les protéines GFP (Green Fluorescent Protein), RFP (Red Fluorescent Protein), CFP (Cyan Fluorescent Protein) ou YFP (Yellow Fluorescent Protein), et des combinaisons de celles-ci.

Selon un mode de réalisation préféré, la protéine de fusion comprend une étiquette formée de six histidines et/ou d'un ou plusieurs motifs Flag, de préférence trois motifs Flag. Selon un mode de réalisation particulier, la protéine de fusion comprend une étiquette formée de six histidines et trois motifs Flag.

De manière alternative, le domaine Spo11 de la protéine de fusion Cas9-Spo11 peut être remplacé par l'un des partenaires de Spo11 capable de recruter Spo11, c'est-à-dire une protéine qui forme un complexe avec Spo11 et induit ainsi la formation des cassures double-brin. Ce partenaire peut être choisi parmi les protéines citées dans les articles de Keeney et al. (2001 Curr. Top. Dev. Biol, 52, pp. 1-53), Smith et al. (Curr. Opin. Genet. Dev, 1998, 8, pp. 200-211) et Acquaviva et al. (2013 Science, 339, pp. 215-8), et plus particulièrement dans le groupe constitué de Rec102, Rec103/Sk18, Rec104, Rec114, Merl, Mer2/Rec107, Mei4, Mre2/Nam8, Mre11, Rad50, Xrs2/Nbsl, Hop1, Red1, Mekl, Set1 et Spp1. De manière préférée, le partenaire remplaçant le domaine Spo11 est Mei4 ou Spp1.

Tous les modes de réalisation décrits pour la protéine de fusion Cas9-Spo11 s'appliquent également aux protéines de fusion dans lesquelles le domaine Spo11 est remplacé par l'un de ses partenaires.

La protéine de fusion telle que décrite ci-dessus peut être introduite dans la cellule sous une forme protéique, notamment sous sa forme mature ou sous la forme d'un précurseur, de préférence sous sa forme mature, ou sous la forme d'un acide nucléique codant ladite protéine.

Lorsque la protéine de fusion est introduite dans la cellule sous une forme protéique, des groupements protecteurs peuvent être ajoutés aux extrémités C- et/ou N-terminales afin d'améliorer la résistance de la protéine de fusion aux peptidases. Par exemple, le groupement protecteur à l'extrémité N-terminale peut être une acylation ou une acétylation et le groupement protecteur à l'extrémité C-terminale peut être une amidation ou une estérification. L'action des protéases peut également être contrecarrée par l'utilisation d'acides aminés de configuration D, la cyclisation de la protéine par formation de ponts disulfures, d'anneaux lactames ou de liaisons entre les extrémités N- et C-terminales. La protéine de fusion de l'invention peut également comprendre des liaisons pseudo-peptidiques remplaçant les liaisons peptidiques « classiques » CONH et conférant une résistance accrue aux peptidases, telles que CHOH-CH2, NHCO, CH2-O, CH2CH2, CO-CH2, N-N, CH=CH, CH2NH, et CH2-S. La protéine de fusion peut également comprendre un ou plusieurs acides aminés qui sont des acides aminés rares notamment l'hydroxyproline, l'hydroxylysine, l'allohydroxylysine, la 6-N-méthylysine, la N-éthylglycine, la N-méthylglycine, la N-éthylasparagine, l'allo-isoleucine, la N-méthylisoleucine, la N-méthylvaline, la pyroglutamine, l'acide aminobutyrique ; ou des acides aminés synthétiques notamment l'ornithine, la norleucine, la norvaline et la cyclohexyl-alanine.

La protéine de fusion selon l'invention peut être obtenue par synthèse chimique classique (en phase solide ou en phase homogène liquide) ou par synthèse enzymatique (Kullmann W, Enzymatic peptide synthesis, 1987, CRC Press, Florida). Elle peut également être obtenue par une méthode consistant à cultiver une cellule hôte exprimant un acide nucléique codant la protéine de fusion et récupérer ladite protéine à partir de ces cellules ou du milieu de culture.

Tel qu'utilisé dans la présente demande, le terme « ARN guide » ou « ARNg » désigne une molécule d'ARN capable d'interagir avec le domaine Cas9 de la protéine de fusion afin de la guider vers une région chromosomique cible.

Chaque ARNg comprend deux régions :
- une première région (communément appelée région « SDS »), à l'extrémité 5' de l'ARNg, qui est complémentaire de la région chromosomique cible et qui imite l'ARNcr du système CRISPR endogène, et
- une seconde région (communément appelé région « handle »), à l'extrémité 3' de l'ARNg, qui mime les interactions d'appariement de bases entre l'ARNtracr (trans-activating crRNA) et l'ARNcr du système CRISPR endogène et présente une structure double brin en tige et boucle s'achevant en 3' par une séquence essentiellement simple brin. Cette seconde région est essentielle à la fixation de l'ARNg au domaine Cas9 de la protéine de fusion.

La première région de l'ARNg varie selon la séquence chromosomique ciblée. En revanche, les régions « handle » des différents ARNg utilisés peuvent être identiques ou différentes. Selon un mode de réalisation particulier, la région « handle » comprend, ou consiste en, la séquence de 82 nucléotides en 3' des séquences SEQ ID NO : 10 à 16 (séquence en minuscule sur la Figure 13).

La région « SDS » de l'ARNg qui est complémentaire de la région chromosomique cible, comprend en général entre 10 et 25 nucléotides. De préférence, cette région a une longueur de 19, 20 ou 21 nucléotides, et de manière particulièrement préférée de 20 nucléotides.

La seconde région de l'ARNg a une structure en tige et boucle (ou structure en épingle à cheveux). Les longueurs de la tige et de la boucle peuvent varier. De préférence, la boucle a une longueur de 3 à 10 nucléotides et la tige une longueur de 6 à 20 nucléotides. La tige peut optionnellement présenter des régions désappariées (formant des « bosses ») de 1 à 10 nucléotides. De préférence, la longueur globale de cette région « handle » est de 50 à 100 nucléotides, et de manière plus particulièrement préférée de 82 nucléotides.

La longueur totale d'un ARNg est en général de 50 à 140 nucléotides, de préférence de 80 à 125 nucléotides, et de manière plus particulièrement préférée de 90 à 110 nucléotides. Selon un mode de réalisation particulier, un ARNg tel qu'utilisé dans la présente invention a une longueur de 102 nucléotides.

L'ARNg est de préférence formé d'une seule molécule d'ARN comprenant les deux domaines. De manière alternative, l'ARNg peut être formé de deux molécules d'ARN distinctes, la première molécule comprenant la région « SDS » et la moitié de la tige de la seconde région, et la deuxième molécule comprenant la deuxième moitié de la tige de l'ARNg. Ainsi, l'appariement des deux molécules d'ARN par leurs séquences complémentaires au niveau de la tige, forme un ARNg fonctionnel.

L'homme du métier peut aisément définir la séquence et la structure des ARNg selon la région chromosomique à cibler en utilisant des techniques bien connues (voir par exemple l'article de Di Carlo et al, Nucleic Acids Research 2013, 1-8).

Dans le procédé selon l'invention, un ou plusieurs ARNg peuvent être utilisés simultanément. Ces différents ARNg peuvent cibler des régions chromosomiques identiques ou différentes, de préférence différentes.

Les ARNg peuvent être introduits dans la cellule eucaryote sous la forme de molécules d'ARNg matures, sous la forme de précurseurs ou sous la forme d'un ou plusieurs acides nucléiques codant lesdits ARNg.

Lorsque le ou les ARNg sont introduits dans la cellule directement sous la forme de molécules d'ARN (matures ou précurseurs), ces ARNg peuvent contenir des nucléotides modifiés ou des modifications chimiques leur permettant, par exemple, d'augmenter leur résistance aux nucléases et ainsi d'augmenter leur durée de vie dans la cellule. Ils peuvent notamment comprendre au moins un nucléotide modifié ou non naturel tel que, par exemple, un nucléotide comportant une base modifiée, telle que l'inosine, la méthyl-5-désoxycytidine, la diméthylarnino-5-désoxyuridine, la désoxyuridine, la diamino-2,6-purine, la bromo-5-désoxyuridine ou toute autre base modifiée permettant l'hybridation. Les ARNg utilisés selon l'invention peuvent également être modifiés au niveau de la liaison internucléotidique comme par exemple les phosphorothioates, les H-phosphonates ou les alkyl-phosphonates, ou au niveau du squelette comme par exemple les alpha-oligonucléotides, les 2'-O-alkyl ribose ou les PNA (Peptid Nucleic Acid) (Egholm et al, 1992 J. Am. Chem. Soc., 114, 1895- 1897).

Les ARNg peuvent être des ARN naturels, synthétiques ou produits par des techniques de recombinaison. Ces ARNg peuvent être préparés par toutes méthodes connues de l'homme du métier telles que, par exemple, la synthèse chimique, la transcription *in vivo* ou des techniques d'amplification.

Selon un mode de réalisation, le procédé comprend l'introduction dans la cellule eucaryote de la protéine de fusion et d'un ou plusieurs ARNg capables de cibler l'action de la protéine de fusion vers une région chromosomique donnée. La protéine et les ARNg peuvent être introduits dans le cytoplasme ou le noyau de la cellule eucaryote par toute méthode connue de l'homme du métier, par exemple par microinjection. La protéine de fusion peut notamment être introduite dans la cellule en tant qu'élément d'un complexe protéine-ARN comprenant au moins un ARNg.

Selon un autre mode de réalisation, le procédé comprend l'introduction dans la cellule eucaryote de la protéine de fusion et d'un ou plusieurs acides nucléiques codant un ou plusieurs ARNg.

Selon encore un autre mode de réalisation, le procédé comprend l'introduction dans la cellule eucaryote d'un acide nucléique codant la protéine de fusion et d'un ou plusieurs ARNg.

Selon encore un autre mode de réalisation, le procédé comprend l'introduction dans la cellule eucaryote d'un acide nucléique codant la protéine de fusion et d'un ou plusieurs acides nucléiques codant un ou plusieurs ARNg.

La protéine de fusion, ou l'acide nucléique codant celle-ci, et le ou les ARNg, ou le ou les acides nucléiques codant ceux-ci, peuvent être introduits simultanément ou séquentiellement dans la cellule.

Alternativement, et plus particulièrement concernant les cellules de plante, l'acide nucléique codant pour la protéine de fusion et le ou les acides nucléiques codant pour le ou les ARNg peuvent être introduits dans une cellule par croisement de deux cellules dans lesquelles ont respectivement été introduits l'acide nucléique codant pour la protéine de fusion et le ou les acides nucléiques codant pour le ou les ARNg.

Alternativement, et plus particulièrement concernant les cellules de plante, l'acide nucléique codant pour la protéine de fusion et le ou les acides nucléiques codant pour le ou les ARNg peuvent être introduits dans une cellule par mitose d'une cellule dans laquelle l'acide nucléique codant pour la protéine de fusion et le ou les acides nucléiques codant pour le ou les ARNg ont précédemment été introduits.

Dans les modes de réalisation où la protéine de fusion et/ou le ou les ARNg sont introduits dans la cellule eucaryote sous la forme d'un acide nucléique codant pour ladite protéine et/ou le/lesdits ARNg, l'expression dédits acides nucléiques permet de produire la protéine de fusion et/ou le ou les ARNg dans la cellule.

Au sens de l'invention, on entend par « acide nucléique » toute molécule à base d'ADN ou d'ARN. Il peut s'agir de molécules synthétiques ou semi-synthétiques, recombinantes, éventuellement amplifiées ou clonées dans des vecteurs, modifiées chimiquement, comprenant des bases non-naturelles ou des nucléotides modifiés comprenant par exemple une liaison modifiée, une base purique ou pyrimidique modifiée, ou un sucre modifié. De préférence, l'utilisation des codons est optimisée selon la nature de la cellule eucaryote.

Les acides nucléiques codant pour la protéine de fusion et ceux codant pour les ARNg peuvent être placés sous le contrôle de promoteurs identiques ou différents, constitutifs ou inductibles, en particulier de promoteurs spécifiques de la méiose. Selon un mode de réalisation préféré, les acides nucléiques sont placés sous le contrôle de promoteurs constitutifs tels que le promoteur ADH1 ou les promoteurs pRPR1 et SNR52 dépendant de l'ARN polymérase III, de manière encore préféré le promoteur pRPR1.

La nature du promoteur peut également dépendre de la nature de la cellule eucaryote. Selon un mode de réalisation particulier, la cellule eucaryote est une cellule de plante, de préférence une cellule de riz, et les acides nucléiques sont placés sous le contrôle d'un promoteur choisi parmi les promoteurs pZmUbi (promoteur de l'ubiquitine de maïs) et les promoteurs de la polymérase III U3 et U6. Selon un mode de réalisation préféré, l'acide nucléique codant pour la protéine de fusion est placé sous le contrôle du promoteur pZmUbi et les acides nucléiques codant pour les ARNg sont placés sous le contrôle du promoteur U3 ou U6, de préférence du promoteur U3.

Les acides nucléiques codant la protéine de fusion et le ou les ARNg peuvent être disposés sur la même construction, en particulier sur le même vecteur d'expression, ou sur des constructions distinctes. De manière alternative, les acides nucléiques peuvent être insérés dans le génome de la cellule eucaryote en des régions identiques ou distinctes. Selon un mode de réalisation préféré, les acides nucléiques codant la protéine de fusion et le ou les ARNg sont disposés sur le même vecteur d'expression.

Les acides nucléiques tels que décrits ci-dessus peuvent être introduits dans la cellule eucaryote par toute méthode connue par l'homme du métier, en particulier par microinjection, transfection, électroporation et biolistique.

Optionnellement, l'expression ou l'activité de la protéine Spo11 endogène de la cellule eucaryote peut être supprimée afin de mieux contrôler les phénomènes de recombinaison méiotiques. Cette inactivation peut être réalisée par des techniques bien connues de l'homme du métier, notamment en inactivant le gène codant la protéine Spo11 endogène ou en inhibant son expression au moyen d'ARN interférents.

Après l'introduction dans la cellule eucaryote de la protéine de fusion et d'un ou de plusieurs ARNg, ou des acides nucléiques codant ceux-ci, le procédé selon l'invention comprend l'induction de l'entrée en prophase I de méiose de ladite cellule.

Cette induction peut se faire selon différentes méthodes, bien connues de l'homme du métier.

A titre d'exemple, lorsque la cellule eucaryote est une cellule de souris, l'entrée des cellules en prophase I de méiose peut être induite par l'ajout d'acide rétinoïque (Bowles J et al, 2006, Sciences, 312(5773), pp. 596-600).

Lorsque la cellule eucaryote est une cellule de plante, l'induction de la méiose se fait selon un processus naturel. Selon un mode de réalisation particulier, après transformation d'un cal comprenant une ou des cellules de plante, une plante est régénérée et placée en conditions favorisant l'induction d'une phase reproductive et ainsi du processus de méiose. Ces conditions sont bien connues de l'homme du métier.

Lorsque la cellule eucaryote est une levure, cette induction peut être réalisée par le transfert de la levure dans un milieu de sporulation, en particulier d'un milieu riche à un milieu de sporulation, ledit milieu de sporulation étant de préférence dépourvu de source de carbone fermentescible ou d'azote, et l'incubation des levures dans le milieu de sporulation durant un temps suffisant pour induire des cassures double-brin Spo11-dépendantes. L'initiation du cycle méiotique dépend de plusieurs signaux : la présence des deux allèles de type sexuel MATa et MATa, l'absence de source d'azote et de carbone fermentescible.

Tel qu'utilisé dans ce document, le terme « milieu riche » se réfère à un milieu de culture comprenant une source de carbone fermentescible et une source d'azote ainsi que tous les éléments nutritifs nécessaires aux levures pour se multiplier par division mitotique. Ce milieu peut être aisément choisi par l'homme du métier et peut, par exemple, être sélectionné parmi le groupe constitué du milieu YPD (1% extrait de levures, 2% bactopeptone et 2% glucose), du milieu YPG (1% extrait de levures, 2% bactopeptone et 3% glycérol) et d'un milieu synthétique complet (ou milieu SC) (Treco and Lundblad, 2001, Curr. Protocol. Mol. Biol., Chapter 13, Unit 13.1).

Tel qu'utilisé dans ce document, le terme « milieu de sporulation » se réfère à tout milieu induisant l'entrée en prophase de méiose des cellules de levure sans croissance végétative, en particulier à un milieu de culture ne comprenant pas de source de carbone fermentescible ni de source d'azote mais comprenant une source de carbone métabolisable par respiration telle que l'acétate. Ce milieu peut être aisément choisi par l'homme du métier et peut, par exemple, être sélectionné parmi le groupe constitué du milieu KAc 1% (Wu and Lichten, 1994, Science, 263, pp. 515-518), le milieu SPM (Kassir and Simchen, 1991, Meth. Enzymol., 194, 94-110) et des milieux de sporulation décrits dans l'article de Sherman (Sherman, Meth. Enzymol., 1991, 194, 3-21).

Selon un mode de réalisation préféré, avant d'être incubées dans le milieu de sporulation, les cellules sont cultivées durant quelques cycles de division dans un milieu de pré-sporulation de façon à obtenir une sporulation efficace et synchrone. Le milieu de pré-sporulation peut être aisément choisi par l'homme du métier. Ce milieu peut être, par exemple, le milieu SPS (Wu and Lichten, 1994, Science, 263, pp. 515-518).

Le choix des milieux (milieu riche, milieu de pré-sporulation, milieu de sporulation) dépend des caractéristiques physiologiques et génétiques de la souche de levure, notamment si cette souche est auxotrophe pour un ou plusieurs composés.

Une fois la cellule engagée en prophase I de méiose, le processus méiotique peut se poursuivre jusqu'à produire quatre cellules filles présentant les recombinaisons recherchées.

De manière alternative, lorsque la cellule eucaryote est une levure, et en particulier une levure du genre *Saccharomyces,* les cellules peuvent être replacées en conditions de croissance afin de reprendre un processus mitotique. Ce phénomène appelé « retum-to-growth » ou « RTG » a été décrit précédemment dans la demande de brevet WO 2014/083142 et se produit lorsque les cellules entrées en méiose en réponse à une carence nutritionnelle sont remises en présence d'une source de carbone et d'azote après la formation des cassures double-brin Spo11 dépendantes mais avant la première division de méiose (Honigberg and Esposito, Proc. Nat. Acad. Sci USA, 1994, 91, 6559-6563). Dans ces conditions, elles interrompent leur progression dans les stades de différenciation méiotique pour reprendre un mode de croissance mitotique tout en induisant les recombinaisons recherchées lors de la réparation des cassures double-brin provoquées par Spo11 (Sherman et Roman, Genetics, 1963, 48,255-261 ; Esposito et Esposito, Proc. Nat. Acad. Sci, 1974, 71, pp. 3172-3176 ; Zenvirth et al, Genes to Cells, 1997, 2, pp. 487-498).

Le procédé peut comprendre en outre l'obtention de la ou des cellules présentant la ou les recombinaisons recherchées.

Le procédé selon l'invention peut être utilisé dans toutes les applications où il est souhaitable d'améliorer et de contrôler les phénomènes de recombinaison méiotique. En particulier, l'invention permet d'associer, de manière préférentielle, des traits génétiques d'intérêt. Cette association préférentielle permet, d'une part, de réduire le temps nécessaire à leur sélection, d'autre part, de générer des combinaisons naturelles possibles mais improbables. Enfin, selon le mode de réalisation choisi, les organismes obtenus par ce procédé peuvent être considérés comme des organismes non génétiquement modifiés (non-OGM).

Selon un autre aspect, la présente invention concerne un procédé pour générer des variants d'un organisme eucaryote, à l'exception de l'homme, de préférence une levure ou une plante, de manière encore préférée une levure, notamment une souche de levure d'intérêt industriel, comprenant
- l'introduction dans une cellule dudit organisme:
   a) d'une protéine de fusion comprenant un domaine Cas9 et un domaine Spo11, ou d'un acide nucléique codant ladite protéine de fusion ; et
   b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- la genèse d'un variant de l'organisme à partir de ladite cellule recombinée.

Dans ce procédé, le terme de « variant » doit être compris de façon large, il désigne un organisme ayant au moins une différence génotypique ou phénotypique par rapport aux organismes parents.

Les cellules recombinées peuvent être obtenues en laissant la méiose se poursuivre jusqu'à l'obtention des spores, ou, dans le cas des levures, en replaçant les cellules en conditions de croissance après l'induction des cassures double-brin afin de reprendre un processus mitotique.

Lorsque la cellule eucaryote est une cellule de plante, un variant de la plante peut être généré par fusion des gamètes de plante, au moins l'une des gamètes étant une cellule recombinée par la méthode selon l'invention.

La présente invention concerne également un procédé pour identifier ou localiser l'information génétique codant pour une caractéristique d'intérêt dans un génome de cellule eucaryote, de préférence une levure, comprenant :
- l'introduction dans la cellule eucaryote:
   a) d'une protéine de fusion comprenant un domaine Cas9 et un domaine Spo11, ou d'un acide nucléique codant ladite protéine de fusion ; et
   b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- l'analyse des génotypes et phénotypes des cellules recombinées afin identifier ou localiser l'information génétique codant pour la caractéristique d'intérêt.

De préférence, la caractéristique d'intérêt est un caractère quantitatif d'intérêt (QTL).

Selon un autre aspect, la présente invention concerne une protéine de fusion comprenant un domaine Cas9 et un domaine Spo11 telle que décrite ci-dessus.

La présente invention concerne également un acide nucléique codant ladite protéine de fusion selon l'invention.

L'acide nucléique selon l'invention peut être sous la forme d'ADN et/ou d'ARN, simple brin ou double brin. Selon un mode de réalisation préférée, l'acide nucléique est une molécule d'ADN isolée, synthétisée par des techniques recombinantes bien connues de l'homme du métier. L'acide nucléique selon l'invention peut être déduit de la séquence de la protéine de fusion selon l'invention et l'usage des codons peut être adapté selon la cellule hôte dans laquelle l'acide nucléique doit être transcrit.

La présente invention concerne en outre une cassette d'expression comprenant un acide nucléique selon l'invention lié de manière opérationnelle aux séquences nécessaires à son expression. Notamment, l'acide nucléique peut être sous le contrôle d'un promoteur permettant son expression dans une cellule hôte. De manière générale, une cassette d'expression comprend, ou consiste en, un promoteur permettant d'initier la transcription, un acide nucléique selon l'invention, et un terminateur de transcription.

Le terme « cassette d'expression » désigne une construction d'acide nucléique comprenant une région codante et une région régulatrice, liées de manière opérationnelle. L'expression "lié de manière opérationnelle" indique que les éléments sont combinés de manière à ce que l'expression de la séquence codante soit sous le contrôle du promoteur transcriptionnel. Typiquement, la séquence du promoteur est placée en amont du gène d'intérêt, à une distance de celui-ci compatible avec le contrôle de son expression. Des séquences d'espacement peuvent être présentes, entre les éléments régulateurs et le gène, dès lors qu'elles n'empêchent pas l'expression. La cassette d'expression peut également comprendre au moins une séquence activatrice « enhancer » liée de manière opérationnelle au promoteur.

Une large variété de promoteurs utilisables pour l'expression de gènes d'intérêt dans des cellules ou des organismes hôtes sont à la disposition de l'homme du métier. Ils incluent des promoteurs constitutifs ainsi que des promoteurs inductibles qui sont activés ou réprimés par des stimuli physiques ou chimiques exogènes.

De préférence, l'acide nucléique selon l'invention est placé sous le contrôle d'un promoteur constitutif ou d'un promoteur spécifique de la méiose.

Des exemples de promoteurs spécifiques de la méiose utilisables dans le cadre de la présente invention comprennent, sans y être limités, les promoteurs endogènes de Spo11, les promoteurs des partenaires de Spo11 pour la formation des cassures double-brin, le promoteur Rec8 (Murakami & Nicolas, 2009, Mol. Cell. Biol, 29, 3500-16,), ou le promoteur Spo13 (Malkova et al, 1996, Genetics, 143, 741-754,).

D'autres promoteurs inductibles peuvent également être utilisés tels que le promoteur estradiol (Carlie & Amon, 2008 Cell, 133, 280-91,), le promoteur méthionine (Care et al, 1999, Molecular Microb 34, 792-798,), les promoteurs induits par les chocs thermiques, les métaux, les stéroïdes, les antibiotiques et l'alcool.

Des promoteurs constitutifs utilisables dans le cadre de la présente invention sont, à titre d'exemples non limitatifs : le promoteur des gènes immédiats précoces du cytomégalovirus (CMV), le promoteur du virus simien (SV40), le promoteur tardif majeur des adénovirus, le promoteur du virus du sarcome de Rous (RSV), le promoteur du virus de la tumeur mammaire de souris (MMTV), le promoteur de la phosphoglycerate kinase (PGK), le promoteur du facteur d'élongation ED1-alpha, les promoteurs de l'ubiquitine, les promoteurs de l'actine, les promoteurs de la tubuline, les promoteurs des immunoglobulines, le promoteur de l'alcool déshydrogénase 1 (ADH1), les promoteurs dépendants de l'ARN polymérase III tels que les promoteurs U6, U3, H1, 7SL, pRPR1 (« Ribonuclease P RNA 1 »), SNR52 (« small nuclear RNA 52 »), ou le promoteur pZmUbi.

Le terminateur de transcription peut être aisément choisi par l'homme du métier. De préférence, ce terminateur est RPR1t, la séquence flanquante du 3' du gène SUP4 de *Saccharomyces cerevisiae* ou le terminateur de la nopaline synthase tNOS.

La présente invention concerne en outre un vecteur d'expression comprenant un acide nucléique ou une cassette d'expression selon l'invention. Ce vecteur d'expression peut être utilisé pour transformer une cellule hôte et permettre l'expression de l'acide nucléique selon l'invention dans ladite cellule. Les vecteurs peuvent être construits par des techniques classiques de biologie moléculaire, bien connues de l'homme du métier.

Avantageusement, le vecteur d'expression comprend des éléments régulateurs permettant l'expression de l'acide nucléique selon l'invention. Ces éléments peuvent comporter par exemple des promoteurs de transcription, des activateurs de transcription, des séquences terminatrices, des codons d'initiation et de terminaison. Les méthodes pour sélectionner ces éléments en fonction de la cellule hôte dans laquelle l'expression est souhaitée, sont bien connues de l'homme du métier.

Dans un mode de réalisation particulier, le vecteur d'expression comprend un acide nucléique codant la protéine de fusion selon l'invention, placé sous le contrôle d'un promoteur constitutif, de préférence le promoteur ADH1 (pADH1). Il peut également comprendre une séquence terminatrice telle que le terminateur ADH1 (tADH1).

Le vecteur d'expression peut comprendre une ou plusieurs origines de réplication, bactérienne ou eucaryote. Le vecteur d'expression peut notamment comprendre une origine de réplication bactérienne fonctionnelle chez *E.coli* telle que l'origine de réplication ColE1. Alternativement, le vecteur peut comprendre une origine de réplication eucaryote, de préférence fonctionnelle chez S. *cerevisiae.*

Le vecteur peut comporter en outre des éléments permettant sa sélection dans une cellule hôte bactérienne ou eucaryote comme, par exemple, un gène de résistance à un antibiotique ou un gène de sélection assurant la complémentation du gène respectif délété dans le génome de la cellule hôte. De tels éléments sont bien connus de l'homme du métier et largement décrits dans la littérature.

Dans un mode de réalisation particulier, le vecteur d'expression comprend un ou plusieurs gènes de résistance à des antibiotiques, de préférence un gène de résistance à l'ampicilline, à la kanamycine, à l'hygromycine, à la généticine et/ou la nourséothricine.

Le vecteur d'expression peut également comprendre une ou plusieurs séquences permettant l'insertion ciblée du vecteur, de la cassette d'expression ou de l'acide nucléique dans le génome d'une cellule hôte. De préférence, l'insertion est réalisée au niveau d'un gène dont l'inactivation permet la sélection des cellules hôtes ayant intégré le vecteur, la cassette ou l'acide nucléique, tel que le locus *TRP1.*

Le vecteur peut être circulaire ou linéaire, simple- ou double-brin. Il est avantageusement choisi parmi les plasmides, phages, phagemides, virus, cosmides et chromosomes artificiels. De manière préférée, le vecteur est un plasmide.

La présente invention concerne en particulier un vecteur, de préférence un plasmide, comprenant une origine de réplication bactérienne, de préférence l'origine ColE1, un acide nucléique tel que défini ci-dessus sous le contrôle d'un promoteur, de préférence un promoteur constitutif tel que le promoteur ADH1, un terminateur, de préférence le terminateur ADH1, un ou plusieurs marqueurs de sélection, de préférence des marqueurs de résistance tels que le gène de résistance à la kanamycine ou à l'ampicilline, et une ou plusieurs séquences permettant l'insertion ciblée du vecteur, de la cassette d'expression ou de l'acide nucléique dans le génome de la cellule hôte, de préférence au niveau du locus *TRP1* du génome d'une levure.

Dans un mode de réalisation particulier, l'acide nucléique selon l'invention porté par le vecteur code pour une protéine de fusion comprenant une ou plusieurs étiquette, de préférence comprenant une étiquette constituée de six histidines et/ou un ou plusieurs motifs Flag, de préférence trois motifs Flag. De préférence la ou les étiquettes sont en C-terminal.

Selon un mode de réalisation particulier le vecteur d'expression est le plasmide P1 de séquence nucléotidique SEQ ID NO : 1 ou le plasmide P2, de séquence nucléotidique SEQ ID NO : 2.

La présente invention concerne également l'utilisation d'un acide nucléique, d'une cassette d'expression ou d'un vecteur d'expression selon l'invention pour transformer ou transfecter une cellule. La cellule hôte peut être transformée/transfectée de manière transitoire ou stable et l'acide nucléique, la cassette ou le vecteur peut être contenu dans la cellule sous forme d'épisome ou intégré dans le génome de la cellule hôte.

La présente invention concerne une cellule hôte comprenant une protéine de fusion, un acide nucléique, une cassette ou un vecteur d'expression selon l'invention.

De préférence, la cellule est une cellule eucaryote, en particulier une cellule de levure, de plante, de champignon ou une cellule animale. De manière particulièrement préférée, la cellule hôte est une cellule de levure. La cellule hôte est non-humaine.

Selon un mode de réalisation particulier, la cellule eucaryote est une cellule de levure, en particulier une levure d'intérêt industriel. Des exemples de levures d'intérêt comprennent, sans y être limités, les levures du genre *Saccharomyces sensu stricto, Schizosaccharomyces, Yarrowia, Hansenula, Kluyveromyces, Pichia* ou *Candida,* ainsi que les hybrides obtenus à partir d'une souche appartenant à l'un de ces genres.

De préférence, la levure d'intérêt appartient au genre *Saccharomyces.* de préférence une levure sélectionnée dans le groupe constitué de *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus, Saccharomyces pastorianus* (aussi nommée *Saccharomyces carlsbergensis*)*,* et des hybrides obtenus à partir d'au moins une souche appartenant à l'une de ces espèces, de manière encore préférée ladite cellule hôte eucaryote est *Saccharomyces cerevisiae.*

Selon un autre mode de réalisation particulier, la cellule eucaryote est une cellule de champignon, en particulier une cellule de champignon d'intérêt industriel. Des exemples de champignons comprennent, sans y être limités, les cellules de champignons filamenteux. Les champignons filamenteux incluent les champignons appartenant aux subdivisions *Eumycota* et *Oomycota.* Les cellules de champignons filamenteux peuvent être choisies dans le groupe constitué des cellules de *Trichoderma, Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium* ou *Trametes.*

Dans un autre mode de réalisation préféré, la cellule est une cellule de plante, de préférence une cellule de plante sélectionnée dans le groupe constitué du riz, du blé, du soja, du maïs, de la tomate, d'*Arabidopsis thaliana,* de l'orge, du colza, du coton, de la canne à sucre, de la betterave, de manière encore préférée ladite cellule hôte eucaryote est une cellule de riz.

La présente invention concerne également l'utilisation de la protéine de fusion, de l'acide nucléique, de la cassette d'expression ou du vecteur d'expression selon l'invention pour (i) induire des recombinaisons méiotiques ciblées dans une cellule eucaryote, (ii) générer des variants d'un organisme eucaryote, et/ou (iii) identifier ou localiser l'information génétique codant pour une caractéristique d'intérêt dans un génome de cellule eucaryote.

La présente invention concerne encore une trousse comprenant une protéine de fusion, un acide nucléique, une cassette d'expression ou un vecteur d'expression selon l'invention, ou une cellule hôte transformée ou transfectée avec un acide nucléique, une cassette d'expression ou un vecteur d'expression selon l'invention. Elle concerne également l'utilisation de ladite trousse pour mettre en œuvre un procédé selon l'invention, en particulier pour (i) induire des recombinaisons méiotiques ciblées dans une cellule eucaryote, (ii) générer des variants d'un organisme eucaryote, et/ou (iii) identifier ou localiser l'information génétique codant pour une caractéristique d'intérêt dans un génome de cellule eucaryote.

Les procédés selon l'invention peuvent être des procédés *in vitro, in vivo* ou *ex vivo.*

Les exemples qui suivent sont présentés dans un but illustratif et non limitatif.

### EXEMPLES

### 1. Conception, synthèse et clonage d'une séquence nucléotidique codant pour la protéine SpCas9 et pour sa forme SpCas9* qui est déficiente pour l'activité nucléase.

Le gène *SpCas9* codant la protéine Cas9 provient de la souche bactérienne *Streptococcus pyogenes.* La forme catalytiquement inactive de SpCas9 (*Sp*Cas9*) se distingue de celle de ***Sp*Cas9** par deux mutations ponctuelles : l'aspartate 10 et l'histidine 840 ont tous les deux été substitués en alanines (Asp¹⁰->Ala¹⁰ et His⁸⁴⁰->Ala⁸⁴⁰).

En raison des variations de fréquence d'utilisation des codons génétiques entre *Streptococcus pyogenes* et *Saccharomyces cerevisiae,* les séquences des gènes *SpCas9* et **SpCas9*** ont été adaptées afin d'optimiser leur expression chez la levure (yeast_optim_*Sp*Cas9 et yeast_optim_*Sp*Cas9*). Les séquences en acides aminés des deux protéines n'ont pas été modifiées.

### 2. Ingénierie des séquences yeast_optim_SpCas9 et yeast_optim_SpCas9* afin de fusionner les protéines SpCas9 et SpCas9* avec la transestérase méiotique Spo11.

Des étapes d'ingénierie des séquences yeast_optim_*Sp*Cas9 et yeast_optim_*Sp*Cas9* ont permis de fusionner les protéines ***Sp*Cas9** et **SpCas9*** avec un signal de localisation nucléaire (NLS) associé à une séquence de liaison (linker 1) en N-terminus et avec une seconde séquence de liaison (linker 2) en C-terminus (qui séparera les protéines SpCas9 et **SpCas9*** de la protéine Spo11 dans la construction finale). Les séquences nucléotidiques ainsi obtenues et codant pour les séquences protéiques NLS-*linker1-Sp*Cas9*-linker2* et NLS-*linker1-SpCas9*-linker2* ont été ensuite clonées dans un plasmide intégratif, contenant la forme complète de la protéine Spo11 de *Saccharomyces cerevisiae* étiquetée avec une séquence codant pour le double motif 6xHis-3xFlag en C-terminus et dont l'expression est contrôlée par le promoteur constitutif pADH1. Les constructions plasmidiques qui en résultaient, P1 et P2, contenaient donc la fusion en phase du N-terminus de NLS*-linker1-Sp*Cas9-*linker2* et NLS*-linker1-Sp*Cas9**-linker2* à la protéine Spo11. En conséquence P1 et P2 permettaient respectivement l'expression constitutive chez la levure des protéines de fusion NLS-*Sp*Cas9-Spo11-6xHis-3xFlag (SEQ ID NO : 6) et NLS-*Sp*Cas9*-Spo11-6xHis-3xFlag (SEQ ID NO : 7) (Figure 1).

### 3. Ingénierie des vecteurs d'expression d'ARN « guides » uniques et multiples.

A partir d'un plasmide 2 micron (2µ) (Farzadfard F et al, 2013, ACS Synth. Biol., 2, pp. 604-613 ; DiCarlo JE et al., 2013, Nucleic Acids Res., 41(7), pp. 4336-4343) contenant la région « handle » (82 nucléotides) d'un ARN « guide » (ARNg), placée sous le contrôle d'un promoteur constitutif dépendant de l'ARN polymerase III tel que pRPR1 ou SNR52, le vecteur d'expression d'un unique ARNg de 102 nucléotides a été construit en clonant la séquence de 20 nucléotides déterminant la spécificité de l'ARNg (région « SDS ») au niveau d'un site de restriction situé immédiatement en 5' de la séquence codant la région « handle » du vecteur linéarisé, par le procédé d'assemblage de Gibson (Figure 2A).

Ce vecteur d'expression présentait une séquence comportant de nombreux sites de restrictions uniques (multiple cloning sites (MCS)) en aval du terminateur (RPR1t ou séquence flanquante du 3' de SUP4. Aussi, afin d'obtenir un système permettant le ciblage multiplexé des sites de recombinaison méiotiques, plusieurs cassettes d'expression d'ARNg ont été introduites dans le vecteur d'expression au niveau de son MCS. Les cassettes d'expression d'ARNg sont composées d'un promoteur constitutif dépendant de l'ARN polymérase III (pRPR1 ou SNR52), de l'ARNg spécifique et d'un terminateur (RPR1t ou la séquence flanquante du 3' de SUP4). Ces cassettes d'expression d'ARNg ont tout d'abord été clonées dans des vecteurs d'expression d'ARNg unique (voir ci-dessus), puis ont été amplifiée par PCR avant d'être clonées successivement au niveau du site de clonages multiples (MCS) du vecteur d'expression d'un unique ARNg par des techniques d'insertion/ligature classiques (Figure 2B). Cette stratégie aboutit à concaténer plusieurs cassettes d'ARNg dans un vecteur d'expression unique.

### 4. Co-expression des protéines de fusion SpCas9-Spo11 et SpCas9*-Spo11 avec les ARNg chez la levure.

Afin d'introduire les fusions NLS-*Sp*Cas9-Spo11 ou NLS-*Sp*Cas9*-Spo11 dans le locus chromosomique *TRP1,* des souches de levure *Saccharomyces cerevisiae* ont été transformées par choc thermique avec les vecteurs P1 ou P2 linéarisés. Ces protéines de fusion portant l'étiquette C-terminale 3xFlag ont été placées sous le contrôle du promoteur constitutif ADH1. Après transformation, les cellules ont été étalées sur des boites de Pétri contenant un milieu sélectif (adaptés aux marqueurs de sélection portés par les plasmides P1 et P2) afin de sélectionner les transformants ayant intégré la fusion dans leur génome.

Le vecteur d'expression du ou des ARNg a ensuite été introduit dans des souches diploïdes de levure exprimant les protéines de fusion NLS-*Sp*Cas9-Spo11 ou NLS-*Sp*Cas9*-Spo11 par transformation par choc thermique. Les cellules ont alors été étalées sur un milieu sélectif pour les marqueurs de sélection des plasmides d'expression du ou des ARNg. Les plasmides d'expression d'ARNg comportaient une origine de réplication 2 micron (2µ) ce qui leur permettaient de se maintenir avec un nombre de copies élevé dans chaque cellule de levure (50-100 copies/cellule).

La formation des cassures double-brin méiotiques générées de façon unique ou multiplexée par les protéines de fusion *Sp*Cas9-Spo11 ou *Sp*Cas9*-Spo11 aux sites génomiques ciblés par des ARNg uniques ou multiples est ensuite détectée par l'analyse Southern Blot de l'ADN génomique extrait des cellules diploïdes cultivées en milieu de sporulation.

### 5. Complémentation des spores issues de la sporulation des souches inactivées pour le gène SPO11 par l'expression de la protéine de fusion SpCas9*-Spo11

Les inventeurs ont analysé la viabilité des spores issues de la méiose des souches diploïdes de *Saccharomyces cerevisiae* suivantes :
- une souche *SPO11*/*SPO11* (ORD7339) comprenant deux copies de l'allèle sauvage du gène SPO11,
- une souche *spo11*/*spo11* (AND2822) comprenant deux copies de l'allèle muté du gène SPO11. Cet allèle muté correspond à l'allèle sauvage dans lequel a été inséré un marqueur génétique inactivant totalement le gène,
- une souche *spo11*/*spo11 dCAS9-SPO11*/*0* (AND2820) comprenant deux copies de l'allèle muté du gène SPOllet une copie du gène codant pour la protéine de fusion *Sp*Cas9*-Spo11 intégrée dans une des deux copies du chromosome IV dans le locus TRP1, et
- une souche *spo11*/*spo11 dCAS9-SPO11*/*dCAS9-SPO11* (AND2823) comprenant deux copies de l'allèle muté du gène SPO11 et deux copies du gène codant pour la protéine de fusion *Sp*Cas9*-Spo11 intégrée dans les deux copies du chromosome IV dans le locus TRP1.

Les résultats présentés sur la Figure 5 montrent que l'expression de la protéine de fusion *Sp*Cas9*-Spo11 complémente l'inviabilité des spores issues de la sporulation des souches inactivées pour le gène SPO11.

### 6. Ciblage des cassures double-brin méiotiques par la protéine de fusion SpCas9*-Spo11 et un ARN guide spécifique de la région YCR048W

La cassette d'expression de *Sp*Cas9*-Spo11 (*dCAS9-SPO11*) a été intégrée au locus chromosomique *TRP1* (chromosome IV). L'ARN guide (sgRNA) UAS1-YCR048W (SEQ ID NO : 10) a été exprimé par le plasmide réplicatif (non-intégratif) multicopies tel que décrit dans la Figure 2A. Le gène de fusion portant la construction *dCAS9-SPO11* a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *dCAS9-SPO11* a été vérifiée par Southern blot.

Les souches utilisées sont les suivantes:
- *SPO11*/*SPO11* (ORD7304),
- *SPO11*/*SPO11* exprimant l'ARN guide (sgRNA) UAS1-YCR48W (ANT2524),
- *spo11*/*spo11 dCAS9-SPO11*/*0* exprimant l'ARN guide "handle", c'est-à-dire un ARN guide sans la région SDS qui est spécifique de la cible chromosomique (ANT2527),
- *spo11*/*spo11 dCAS9-SPO11*/*0* exprimant l'ARN guide (sgRNA) UAS1-YCR48W (ANT2528), et
- *spo11*/*spo11 dCAS9-SPO11*/*dCAS9-SPO11* exprimant l'ARN guide (sgRNA) UAS1-YCR48W (ANT2529).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par l'enzyme de restriction *AseI.* L'ADN a été sondé avec un fragment interne au locus *YCR048W.* La quantification des bandes a été effectuée avec le logiciel ImageJ.

Les résultats présentés sur la Figure 6 montre que l'expression de la construction *Sp*Cas9*-Spo11 (*dCAS9-SPO11*) induit des cassures d'ADN double brin (CDBs) méiotiques aux sites naturels de coupure de la protéine Spo11 (région *YCR043C-YCR048W* du chromosome III, (CDBs I à VI symbolisés par des carrés noirs dans la Figure 6) ainsi qu'au site UAS1 (CDB VII triangle noir) ciblé par l'ARN guide UAS1-YCR048W et localisé dans la région codante du gène *YCR048W.*

### 7. Ciblage des cassures double-brin méiotiques par la protéine de fusion SpCas9*-Spo11 et un ARN guide spécifique de la région YCR048W

La cassette d'expression de *Sp*Cas9*-Spo11 (*dCAS9-SPO11*) a été intégrée au locus chromosomique *TRP1* (chromosome IV). L'ARN guide (sgRNA) UAS1-YCR048W ou UAS2-YCR048W (SEQ ID NO : 11) a été exprimé par le plasmide réplicatif (non-intégratif) multicopies tel que décrit dans la Figure 2A. Le gène de fusion portant la construction *dCAS9-SPO11* a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *dCAS9-SPO11* a été vérifiée par Southern blot.

Les souches utilisées sont les suivantes:
- *SPO11*/*SPO11* (ORD7304),
- *SPO11*/*SPO11* exprimant l'ARN guide (sgRNA) UAS1-YCR048W (ANT2524),
- *SPO11*/*SPO11 dCAS9-SPO11*/*0* exprimant l'ARN guide "handle", c'est-à-dire un ARN guide sans la région SDS qui est spécifique de la cible chromosomique (ANT2518),
- *SPO11*/*SPO11 dCAS9-SPO11*/*0* exprimant l'ARN guide (sgRNA) UAS1-YCR048W (ANT25 19),
- *SPO11*/*SPO11 dCAS9-SPO11*/*dCAS9-SPO11* exprimant l'ARN guide (sgRNA) UAS1-YCR48W (ANT2522),
- *SPO11*/*SPO11 dCAS9-SPO11*/*0* exprimant l'ARN guide (sgRNA) UAS2-YCR048W (ANT2520),
- *SPO11*/*SPO11 dCAS9-SPO11*/*dCAS9-SPO11* exprimant l'ARN guide (sgRNA) UAS2-YCR048W (ANT2523), et
- *spo11*/*spo11 dCAS9-SPO11*/*0* exprimant l'ARN guide (sgRNA) UAS1-YCR048W (ANT2528).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par les enzymes de restriction *AseI* et *SacI.* L'ADN a été sondé avec un fragment interne au locus *YCR048W.* La quantification des bandes a été effectuée avec le logiciel ImageJ.

Les résultats présentés sur la Figure 7 indiquent que l'expression de la construction *dCAS9-SPO11* induit des cassures d'ADN double brin (CDBs) méiotiques aux sites naturels de coupure de la protéine Spo11 (région *YCR047C-YCR048W* du chromosome III (CDBs V et VI symbolisés par des carrés) ainsi qu'au site UAS1-YCR048W (CDB VII symbolisé par un triangle) ciblé par l'ARN guide UAS1-YCR048W dans la région codante en 5' du gène *YCR048W et* au site UAS2-YCR048W (CDB VIII symbolisé par un rond) ciblé par l'ARN guide UAS2-YCR048W dans la région codante en 3' du gène *YCR048W.* Ces résultats montrent que le ciblage est effectif dans les souches porteuses du gène SPO11 sauvage ou spo1 1 muté.

### 8. Ciblage des cassures double-brin méiotiques par la protéine de fusion SpCas9*-Spo11 et un ARN guide spécifique de la région GAL2

La cassette d'expression de *Sp*Cas9*-Spo11 (*dCAS9-SPO11*) a été intégrée au locus chromosomique *TRP1* (chromosome IV). L'ARN guide (sgRNA) UAS D/E-GAL2 (SEQ ID NO : 12) a été exprimé par le plasmide réplicatif (non-intégratif) multicopies tel que décrit dans la Figure 2A. Le gène de fusion portant la construction *dCAS9-SPO11* a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *dCAS9-SPO11* a été vérifiée par Southern blot.

Les souches utilisées sont les suivantes:
- *SPO11*/*SPO11* (ORD7304),
- *spo11*/*spo11 GAL4*/*GAL4 dCAS9-SPO11*/*0* exprimant l'ARN guide "handle", c'est-à-dire un ARN guide sans la région SDS qui est spécifique de la cible chromosomique (ANT2527),
- *spo11*/*spo11 gal4*/*gal4 dCAS9-SPO11*/*0* exprimant l'ARN guide "handle" (ANT2536) (les deux allèles du gène GAL4 sont mutés et donc inactifs),
- *spo11*/*spo11 GAL4*/*GAL4 dCAS9-SPO11*/*0* exprimant l'ARN guide UAS D/E-GAL2 (ANT2530), et
- *spo11*/*spo11 gal4*/*gal4 dCAS9-SPO11*/*0* exprimant l'ARN guide UAS D/E-GAL2 (ANT2533).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par l'enzyme de restriction *XbaI.* L'ADN a été sondé avec la partie terminale du gène GAL2. La quantification des bandes a été effectuée avec le logiciel ImageJ.

Les résultats présentés sur la Figure 8 montrent que l'expression de la construction *dCAS9-SPO11* induit des cassures d'ADN double brin (CDBs) méiotiques au site UAS D/E du promoteur du gène GAL2 ciblé par l'ARN guide UAS D/E-GAL2.

### 9. Ciblage des cassures double brin méiotiques par la protéine de fusion SpCas9*-Spo11 et un ARN guide spécifique de la région SWC3

La cassette d'expression de *Sp*CAS9*-SPO11 (*dCas9-SPO11*) a été intégrée au locus chromosomique *TRP1* (chromosome IV). L'ARN guide (sgRNA*_{SWC3}*) SWC3 (SEQ ID NO : 13) a été exprimé par le plasmide réplicatif (non-intégratif) multicopies tel que décrit précédemment (Figure 2A). Le gène de fusion portant la construction *Sp*CAS9*-SPO11 a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *Sp*CAS9*-SPO11 a été vérifiée par Southern blot.

Les souches utilisées sont les suivantes:
- *SPO11*/*SPO11* (ORD7304),
- *spo11*/*spo11* Sp*CAS9*-SPO11*/*0* exprimant l'ARN guide "handle", c'est-à-dire un ARN guide sans la région SDS qui est spécifique de la cible chromosomique (ANT2527),
- *spo11*/*spo11* Sp*CAS9*-SPO11*/*0* exprimant l'ARN guide (sgRNA*_{SWC3}*) SWC3 (ANT2564).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par les enzymes de restriction *Pac*I et *Avr*II. L'ADN a été sondé avec un fragment interne au locus *SPO7.* La quantification des bandes a été effectuée avec le logiciel ImageJ.

Les résultats présentés sur la Figure 9 montrent que l'expression de la construction *Sp*CAS9*-Spo11 induit des cassures d'ADN double brin (CDBs) méiotiques aux sites naturels de coupure de la protéine Spo11 (région *SIN8-SWC3)* du chromosome I, (CDBs I, II et III symbolisés par des carrés noirs dans la Figure 9) ainsi qu'au site ciblé (CDBs IV symbolisé par un rond) par l'ARN guide (sgRNA_{SWC3}) SWC3 et localisé dans la région codante du gène *SWC3.*

### 10. Ciblage des cassures d'ADN double brin méiotiques par la protéine SpCas9*-Spo11 et plusieurs ARN guides multiplexés spécifiques de la région GAL2

La cassette d'expression de *Sp*CAS9*-Spo11 (*dCas9-SPO11*) a été intégrée au locus chromosomique *TRP1* (chromosome IV). Les ARNs guide (sgRNA*_{UAS-A}*) UAS-A (SEQ ID NO : 14), (sgRNA*_{UAS-B}*) UAS-B (SEQ ID NO : 15) et (sgRNA_{*UAS*-*D*/*E*}) UAS-D/E (SEQ ID NO : 12) ont été exprimés individuellement et en multiplexe (Multi gRNAs) par le plasmide réplicatif (non-intégratif) multicopies tel que décrit précédemment (Figure 2A). Le gène de fusion portant la construction *Sp*CAS9*-SPO11 a été exprimé sous le contrôle du promoteur constitutif *ADH1.* Les ARN guides ont été exprimés sous le contrôle du promoteur constitutif *RPR1.* Les cellules de levure ont été transformées par la méthode classique d'électroporation. L'intégration de la cassette porteuse de la construction *Sp*CAS9*-SPO11 a été vérifiée par Southern blot.

Les souches utilisées sont les suivantes:
- *spol11*/*spo11 GAL4*/*GAL4* Sp*CAS9*-SPO11*/*0* exprimant l'ARN guide "handle", c'est-à-dire un ARN guide sans la région SDS qui est spécifique de la cible chromosomique (ANT2527),
- *spo11*/*spo11 GAL4*/*GAL4* Sp*CAS9*-SPO11*/*0* exprimant l'ARN guide (sgRNA*_{UAS}-_{A}*) UAS-A (ANT2532),
- *spo11*/*spo11 gal4*/*gal4* Sp*CAS9*-SPO11*/*0* exprimant l'ARN guide (sgRNA*_{UAS-A}*) UAS-A (ANT2534),
- *spo11*/*spo11 GAL4*/*GAL4* Sp*CAS9*-SPO11*/*0* exprimant l'ARN guide (sgRNA_{*UAS*-*D*/*E*}) UAS-D/E (ANT2530),
- *spo11*/*spo11 gal4*/*gal4* Sp*CAS9*-SPO11*/*0* exprimant l'ARN guide (sgRNA_{*UAS*-*D*/*E*}) UAS-D/E (ANT2533),
- *spo11*/*spo11 GAL4*/*GAL4* Sp*CAS9*-SPO11*/*0* exprimant en multiplex les ARNs guide (sgRNA*_{UAS-A}*) UAS-A, (sgRNA*_{UAS-B}*) UAS-B et (sgRNA_{*UAS*-*D*/*E*}) UAS-D/E (MultigRNAs) (ANT2551),
- *spo11*/*spo11 gal4*/*gal4* Sp*CAS9*-SPO11*/*0* exprimant en multiplex les ARNs guide (sgRNA*_{UAS-A}*) UAS-A, (sgRNA*_{UAS-B}*) UAS-B et (sgRNA_{*UAS*-*D*/*E*}) UAS-D/E (MultigRNAs) (ANT2552).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par l'enzyme de restriction *Xba*I*.* L'ADN a été sondé avec la partie terminale du gène *GAL2.* La quantification des bandes a été effectuée avec le logiciel ImageJ.

Les résultats présentés sur la Figure 10 indiquent que l'expression de la construction *Sp*CAS9*-Spo1 (*dCas9-SPO11*) induit des cassures d'ADN double brin (CDBs) méiotiques aux sites cibles dans le promoteur du gène GAL2 sur le chromosome XII par le ou les ARN(s) guide. En particulier, la co-expression de *Sp*Cas9*-Spo11 avec les sgRNAs individuels sgRNA*_{UAS-A}* et *s*gRNA_{*UAS-D*/*E*} conduit à la génération de CDBs, respectivement, aux sites UAS-A et UAS-D/E. De manière intéressante le ciblage de *Sp*Cas9*-Spo11 par la co-expression des sgRNA*_{UAS-A}*, sgRNA*_{UAS-B}* et sg*RNA*_{*UAS-D*/*E*} conduit à la formation de CDBs multiplex aux différents sites cibles.

### 11. Stimulation de la recombinaison méiotique par la protéine SpCas9*-SPO11 et de plusieurs ARN guides multiplexés dans la région cible GAL2

Afin de détecter les crossing-overs induits par l'expression de CRISPR/*Sp*Cas9*-Spo11 les cassettes NatMX et HphMX (qui confèrent respectivement la résistance à la nourseothricine et à l'hygromycine) ont été insérées en *trans* en amont et en aval du gène GAL2 dans des cellules diploides (cf. figure 11A).

La cassette d'expression de *Sp*CAS9*-Spo11 (*dCAS9-SPO11*) a été intégrée au locus chromosomique *TRP1* (chromosome IV). L'ARN guide (sgRNA_{*UAS*-*D*/*E*}) UAS-D/E (SEQ ID NO : 12) a été exprimé par le plasmide réplicatif (non-intégratif) multicopies tel que décrit précédemment (Figure 2A). L'expression en multiplexe des ARNs guide (sgRNA*_{UAS}-_{A}*) UAS-A (SEQ ID NO : 14), (sgRNA*_{UAS}-_{B}*) UAS-B (SEQ ID NO : 15) et (sgRNA_{*UAS*-*D*/*E*}) UAS-D/E (SEQ ID NO : 12) a été effectuée à partir du même plasmide d'expression d'ARN guides décrit ci-dessus (Multi gRNAs). Le gène de fusion portant la construction *SpCAS9*-SPO11* a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *Sp*CAS9*-SPO11 a été vérifiée par Southern blot.

Les souches utilisées sont les suivantes:
- *SPO11*/*SPO11 pEMP46::NatMX*/*0 tGAL2::KanMX*/*O* (ANT2527),
- *spo11*/*spo11 pEMP46: : NatMX*/*0 tGAL2::KanMX*/*O* Sp*CAS9*-SPO11*/*0* exprimant l'ARN guide "handle", c'est-à-dire un ARN guide sans la région SDS qui est spécifique de la cible chromosomique (ANT2539),
- *spo11*/*spo11 pEMP46::NatMX*/*0 tGAL2::KanMX*/*O* Sp*CAS9*-SPO11*/*0* exprimant l'ARN guide (sgRNA_{*UAS*-*D*/*E*}) UAS-D/E (ANT2540),
- *spo11*/*spo11 pEMP46::NatMX*/*0 tGAL2::KanMX*/*0* Sp*CAS9*-SPO11*/*0* exprimant en multiplex les ARNs guide (sgRNA*_{UAS-A}*) UAS-A, (sgRNA*_{UAS}-_{B}*) UAS-B et (sgRNA_{*UAS*-*D*/*E*}) UAS-D/E (MultigRNAs) (ANT2557).

Après sporulation,les tétrades composés de 4 spores ont été disséquées et les spores génotypées après germination pour la ségrégation à la nourseothricine et à l'hygromycine. Le nombre de tétrades montrant un ditype parental (P) a été comparé à ceux montrant un tétratype (T) et un ditype non-parental (NPD). La distance génétique en centimorgans a été déterminée d'après la formule cM = 100(T+6NPD)/2(PD+T+NPD). L'augmentation du nombre de tétratypes chez les cellules exprimant *Sp*CAS9*-SPO11 (souches ANT2540 et ANT 2557) a été statistiquement testé avec le test de Fisher en calculant la "p value" par rapport aux cellules co-exprimant *Sp*CAS9*-SPO11 et l'ARN guide "handle " (souche ANT2539).

Les résultats présentés sur la Figure 11B montent que l'expression de la construction *SpCAS9**-SPO11 (*dCAS9-SPO11)* stimule la recombinaison méiotique dans la région GAL2 cible.

### 12. Ciblage des cassures d'ADN double brin méiotiques par la protéine de fusion SpCas9*-Spo11 et un ARN guide spécifique de la séquence codante du gène PUT4

La cassette d'expression de *Sp*CAS9*-Spo11 (*dCAS9-SPO11*) a été intégrée au locus chromosomique *TRP1* (chromosome IV). L'ARN guide (*sgRNA_{PUT4})* PUT4 (SEQ ID NO : 16) a été exprimé par le plasmide réplicatif (non-intégratif) multicopies tel que décrit précédemment (Figure 2A). Le gène de fusion portant la construction *Sp*CAS9*-SPO11 a été exprimé sous le contrôle du promoteur constitutif *ADH1.* L'ARN guide a été exprimé sous le contrôle du promoteur constitutif *RPR1.* Les cellules de levure ont été transformées par la méthode classique d'électroporation. L'intégration de la cassette porteuse de la construction *Sp*CAS9*-SPO11 a été vérifiée par Southern blot.

Les souches utilisées sont les suivantes:
- *SPO11*/*SPO11* (ORD7304),
- *spo11*/*spo11* Sp*CAS9*-SPO11*/*0* exprimant l'ARN guide "handle", c'est-à-dire un ARN guide sans la région SDS qui est spécifique de la cible chromosomique (ANT2527),
- *spo11*/*spo11* Sp*CAS9*-SPO11*/*0* exprimant l'ARN guide (sgRNA*_{PUT4}*) PUT4 (ANT2547).

Les cellules ont été collectées après transfert dans le milieu de sporulation (1% Kac) et prélevées aux temps indiqués (heures). Les souches sont homozygotes pour la délétion du gène *SAE2* ce qui inhibe la réparation des cassures d'ADN double brin (CDBs). L'accumulation des CDBs a été détectée par Southern blot après digestion de l'ADN génomique par les enzymes de restriction BamHI et *Xho*I*.* L'ADN a été sondé avec un fragment interne au locus *CIN1.* La quantification des bandes a été effectuée avec le logiciel ImageJ.

Les résultats présentés sur la Figure 12 montrent que l'expression de la construction *Sp*CAS9*-SPO11 (*dCas9-SPO11*) induit des cassures d'ADN double brin (CDBs) méiotiques aux sites naturels de coupure de la protéine Spo11 (région *PYK2-PUT4)* du chromosome XV, (CDBs I, II, III et V symbolisés par des carrés noirs dans la Figure 11) ainsi qu'au site ciblé (CDBs IV symbolisés par un triangle noir) par l'ARN guide (sgRNA*_{PUT4}*) PUT4 et localisé dans la région codante du gène *PUT4.*

### 13. Conclusion

Les résultats présentés dans les Figures 5 à 12 montrent que :
- l'expression de la protéine de fusion *Sp*Cas9*-Spo11 (*dCAS9-SPO11*) complémente l'inviabilité des spores issues de la sporulation des souches inactivées pour le gène SPO11,
- l'expression de la protéine de fusion *Sp*Cas9*-Spo11 (*dCAS9-SPO11*) induit la formation des cassures double-brin méiotiques aux sites naturels de CDBs,
- la co-expression de la protéine de fusion *Sp*Cas9*-Spo11 (*dCAS9-SPO11*) et d'un ARNg induit la formation de cassures double-brin méiotiques aux sites naturels de CDBs et au site ciblé,
- la co-expression de la protéine de fusion *Sp*Cas9*-Spo11 (*dCAS9-SPO11*) et d'ARNg multiplexés induit la formation de cassures double-brin méiotiques CDBs aux différents sites ciblés, et
- le ciblage est effectif dans les souches de fond génétique SPO11 sauvage et spo11 muté.

### 14. Induction de cassures double brin méiotiques par la protéine de fusion SpCas9*-Spo11 chez le riz

### a) Réalisation du vecteur de transformation dCas9-SPO11 (cf. figure 14)

Cas9 étant une protéine d'origine procaryote, les codons utilisés sont optimisés selon l'espèce végétale dans laquelle la protéine soit être exprimée. Les codons de la protéine Cas9 de *Streptococcus pyogenes* sont donc optimisés pour son expression chez le riz (cf. Miao et al, Cell Research, 2013, pp. 1-4). Par ailleurs, la protéine Cas9 est inactivée par mutation des deux sites catalytiques RuvC et HNH (Asp¹⁰->Ala¹⁰ et His⁸⁴⁰->Ala⁸⁴⁰) . La forme catalytiquement inactive de ***Sp*Cas9** est nommée ***Sp*Cas9*** ou dCas9.

Dans un premier temps, le codon stop de dCas9 est éliminé et une séquence de liaison (ou linker) est ajoutée en phase en C-terminal de dCas9. La séquence de liaison peut être une séquence déjà connue dans la littérature pour une utilisation chez l'espèce végétale concernée ou une séquence optimisée. La séquence de liaison CCGGAATTTATGGCCATGGAGGCCCCGGGGATCCGT (SEQ ID NO : 17) utilisée chez la levure est également compatible avec une utilisation chez le riz.

Un signal de localisation nucléaire (NLS) est également ajouté en N-terminal de dCas9. Optionnellement, une séquence de liaison peut être ajoutée entre le NLS et dCas9, tel que par exemple la séquence GGTATTCATGGAGTTCCTGCTGCG (SEQ ID NO : 18).

La séquence de SPO11 est ensuite ajoutée en phase en C-terminal de la construction NLS-dCas9-Linker. Il est possible d'utiliser une séquence d'ADN complémentaire (cDNA), d'ADN génomique (gDNA) ou encore une séquence d'ADN complémentaire avec ajout de quelques introns. Il est possible d'utiliser la SPO11-1 et/ou la SPO11-2 de riz.

Le terminateur de la nopaline synthase (tNOS), adapté au riz, est ajouté en phase de la construction NLS-dCas9-Linker-SPO11.

Le promoteur de l'ubiquitine de maïs pZmUbi1 (Christensen AH et al, 1992, Plant Mol Biol, 18(4), pp 675-689 ou Christensen AH et Quai1 PH, 1995, Transgenic Res, 5(3), pp. 213-218), est un promoteur permettant une expression ubiquitaire et forte chez le riz.

Pour réaliser une transformation stable des cellules de riz, la transfection est réalisée avec un vecteur binaire, par exemple le vecteur binaire pCAMBIA5300 portant un gène de résistance à l'hygromycine interrompu par un intron du gène de catalase. Ce gène de résistance permet de sélectionner efficacement, sur un milieu sélectif, les individus ayant intégré dCas9-SPO11 dans leur génome. Ce vecteur contient également un gène de résistance bactérien à la kanamycine facilitant les clonages et les manipulations dans les hôtes bactériens.

### b) Réalisation du construit portant l'ARN guide

En ce qui concerne la région « handle » de l'ARN guide (ARNg), la séquence « native» de la bactérie *S. pyogenes* est utilisée. Pour la région « SDS » déterminant la spécificité de l'ARNg, celle-ci est choisie en fonction de la zone d'intérêt à cibler à l'aide de logiciels en libre accès sur internet (par exemple CRISPR PLANT).

L'ARN guide est placé sous le contrôle du promoteur U3 de la polymérase III du riz (cf. Miao et al. Cell Research, 2013, pp. 1-4). Alternativement, il est placé sous le contrôle du promoteur U6.

### - Vecteur binaire unique

La construction comprenant l'ARN guide placé sous le contrôle du promoteur U3 est intégrée au vecteur comprenant dCAS9-SPO11.

### - Vecteurs binaires séparés

Afin de cibler plusieurs régions, les ARN guides sont portés par un vecteur distinct, un vecteur binaire portant une résistance à la généticine (pCAMBIA2300), ce qui permet d'appliquer une double sélection pour la présence de l'ADN-T de dCas9-SPO11 et de l'ADN-T de l'ARNg.

Plusieurs stratégies de transformation sont possibles :
*Co-transformation* : à partir de deux vecteurs binaires, l'un portant la dCas9-SPO11 et l'autre le(s) ARNg(s). Ils sont introduits dans la même souche bactérienne ou dans deux souches bactériennes différentes qui sont ensuite mélangées avant co-culture avec les cellules végétales.
*Transformations séquentielles* : des transformants stables porteurs du construit dCas9-SPO11 sont produits et leurs grains utilisés pour produire des cals qui sont ensuite utilisés pour la transformation avec le construit d'ARNg par Agrobacterium ou par bombardement.
*Transformations indépendantes* : Des plantes portant la dCAS9-SPO11 sans guide sont générées d'une part, et des plantes portant des ARNg seuls sont générées d'autre part. Les transformants stables sont ensuite croisés entre eux de manière à réaliser de multiples combinaisons.

### c. Transformation du riz

La transformation du riz est réalisée à partir de cals d'embryons de grains matures suivant le protocole détaillé dans Sallaud C et al, 2003, Theor Appl Genet, 106(8), pp. 1396-1408.

### Utilisation de la technologie dCas9-SPO11 pour induire une recombinaison ciblée en fond sauvage SPO11

La protéine de fusion dCas9-SPO11 est produite avec la protéine SPO11 native (SPO11-1 ou SPO11-2, sous leur forme gDNA ou cDNA.
- par transformation directe de cals issus de semences F1 : des cals sont induits à partir des embryons de semences F1 matures obtenues par castration et fécondation manuelle entre deux lignées parentales d'intérêt agronomique. Les cals sont transformés simultanément avec l'ADN-T ou les ADN-T porteurs de dCas9-SPO11 et du (des) ARNg (co-transformation). Des transformants sans ARNg ou avec unARNg ciblant une autre région sont utilisés comme témoin pour tester l'efficacité du système. L'analyse des recombinaisons se fait au niveau des plantes F2.
- par transformation séparée de cals de chacun des parents : une lignée est transformée de façon stable et homozygote avec le construit dCas9-SPO11 et croisée avec l'autre lignée porteuse du (des) ARNg en insertion hétérozygote. Les plantes F1 porteuses des deux construits ou seulement du construit dCas9-SPO11 sont sélectionnées. Les recombinaisons au locus (loci) cible(s) sont quantifiées dans les populations F2 issues des deux types de plantes.

### Utilisation de la technologie dCas9-SPO11 pour induire une recombinaison ciblée en fond mutant spo11

Une des lignées parentales (grains portés par un hétérozygote SPO11/spo11) est transformée avec le construit dCas9-SPO11 et des plantes homozygotes pour le transgène et la mutation spo11 sont obtenues en génération T1. La seconde lignée parentale (grains portés par un hétérozygote SPO11/spo11) est transformée avec le construit porteur du ou des ARNg. Des plantes hétérozygotes pour le construit ARNg et la mutation spo11 sont obtenues en génération T1. Les deux types de plantes sont croisées : 4 génotypes de grains F1 sont obtenus, tous porteurs du construit dCas9-SPO11 mais porteurs ou non de l'ARNg et produisant ou non SPO11 endogène. L'analyse des recombinaisons se fait au niveau des populations F2.

## Revendications

1. Procédé pour induire des recombinaisons méiotiques ciblées dans une cellule eucaryote non humaine comprenant
- l'introduction dans ladite cellule :
a) d'une protéine de fusion comprenant un domaine Cas9 et un domaine Spo11 ou d'un acide nucléique codant ladite protéine de fusion ; et
b) d'un ou plusieurs ARN guides ou d'un ou plusieurs acides nucléiques codant lesdits ARN guides, lesdits ARN guides comprenant une structure ARN de fixation au domaine Cas9 de la protéine de fusion et une séquence complémentaire de la région chromosomique ciblée ; et
- l'induction de l'entrée en prophase I de méiose de ladite cellule.

2. Procédé selon la revendication 1, dans lequel la protéine de fusion comprend en outre une séquence signal de localisation nucléaire.

3. Procédé selon l'une quelconque des revendications précédentes, dans lequel le domaine Cas9 est une protéine Cas9 déficiente pour l'activité nucléase.

4. Procédé selon l'une quelconque des revendications précédentes, dans lequel l'expression de l'acide nucléique codant ladite protéine de fusion est placée sous le contrôle d'un promoteur constitutif, inductible ou spécifique de la méiose.

5. Procédé selon l'une quelconque des revendications précédentes, comprenant en outre l'introduction d'un ou plusieurs ARN guides supplémentaires ciblant une ou plusieurs autres régions chromosomiques, ou acides nucléiques codant lesdits ARN guides supplémentaires.

6. Procédé selon l'une quelconque des revendications précédentes, dans lequel la cellule est une levure, et l'induction de l'entrée en prophase I est de préférence obtenue par transfert de la levure d'un milieu riche à un milieu de sporulation.

7. Procédé selon l'une quelconque des revendications 1 à 5, dans lequel la cellule eucaryote est une cellule de plante.

8. Procédé selon l'une quelconque des revendications 1 à 7, dans lequel l'introduction de la protéine de fusion, ou de l'acide nucléique codant pour celle-ci, et du ou des ARNg, ou du ou des acides nucléiques codant pour ceux-ci, dans ladite cellule est simultanée ou séquentielle.

9. Procédé selon l'une quelconque des revendications 1 à 7, dans lequel l'introduction de l'acide nucléique codant pour la protéine de fusion et du ou des acides nucléiques codant pour le ou les ARNg dans ladite cellule est obtenue par croisement de deux cellules dans lesquelles ont respectivement été introduits l'acide nucléique codant pour la protéine de fusion et le ou les acides nucléiques codant pour le ou les ARNg.

10. Protéine de fusion telle que définie dans l'une quelconque des revendications 1 à 3.

11. Acide nucléique codant pour la protéine de fusion selon la revendication 10.

12. Cassette d'expression ou vecteur comprenant un acide nucléique selon la revendication 11.

13. Cellule hôte non humaine comprenant une protéine de fusion selon la revendication 10, un acide nucléique selon la revendication 11, une cassette ou un vecteur selon la revendication 12, ladite cellule hôte étant de préférence une cellule eucaryote, de manière tout particulièrement préférée une cellule de levure, de plante, de champignon ou une cellule animale.

14. Cellule hôte selon la revendication 13, dans laquelle ladite cellule hôte est
- une cellule de levure, de préférence sélectionnée dans le groupe constitué de *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus, Saccharomyces pastorianus* (aussi nommée *Saccharomyces carlsbergensis),* et des hybrides obtenus à partir d'au moins une souche appartenant à l'une de ces espèces, de manière encore préférée ladite cellule hôte est *Saccharomyces cerevisiae,* ou
- une cellule de plante, de préférence une cellule de plante sélectionnée dans le groupe constitué du riz, du blé, du soja, du maïs, de la tomate, *d'Arabidopsis thaliana,* de l'orge, du colza, du coton, de la canne à sucre, et de la betterave, de manière encore préférée ladite cellule hôte est une cellule de riz.

15. Procédé pour générer des variants d'un organisme eucaryote, à l'exception de l'homme, de préférence une levure ou une plante, comprenant :
- l'introduction dans une cellule dudit organisme:
a) d'une protéine de fusion comprenant un domaine Cas9 et un domaine Spo11, ou d'un acide nucléique codant ladite protéine de fusion ; et
b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- la genèse d'un variant de l'organisme à partir de ladite cellule recombinée.

16. Procédé pour identifier ou localiser l'information génétique codant pour une caractéristique d'intérêt dans un génome de cellule eucaryote non humaine, de préférence une levure ou une plante, comprenant :
- l'introduction dans la cellule eucaryote:
a) d'une protéine de fusion comprenant un domaine Cas9 et un domaine Spo11, ou d'un acide nucléique codant ladite protéine de fusion ; et
b) d'un ou plusieurs ARN guides, ou d'un ou plusieurs acides nucléiques codant lesdits ARN guides, lesdits ARN guides comprenant une structure ARN de fixation au domaine Cas9 et une séquence complémentaire d'une région chromosomique ciblée; et
- l'induction de l'entrée en prophase I de méiose de ladite cellule ;
- l'obtention de cellule(s) présentant la ou les recombinaisons recherchées au niveau de la ou des régions chromosomiques ciblées ; et
- l'analyse des génotypes et phénotypes des cellules recombinées afin d'identifier ou de localiser l'information génétique codant pour la caractéristique d'intérêt
la caractéristique d'intérêt étant de préférence un caractère quantitatif d'intérêt (QTL).

17. Utilisation d'une trousse comprenant une protéine de fusion selon la revendication 10, un acide nucléique selon la revendication 11, une cassette ou vecteur selon la revendication 12, ou une cellule hôte selon l'une quelconque des revendications 13 et 14, pour mettre en œuvre un procédé selon l'une quelconque des revendications 1 à 9, 15 et 16, en particulier pour (i) induire des recombinaisons méiotiques ciblées dans une cellule eucaryote non humaine, (ii) générer des variants d'un organisme eucaryote non humain, et/ou (iii) identifier ou localiser l'information génétique codant pour une caractéristique d'intérêt dans un génome de cellule eucaryote non humaine.

## Patentansprüche

1. Verfahren zur Induktion von zielgerichteten meiotischen Rekombinationen in einer nicht-humanen eukaryotischen Zelle, umfassend
- Einführung in besagte Zelle:
a) eines Fusionsproteins, umfassend eine Cas9-Domäne und eine Spo1-Domäne, oder einer Nukleinsäure, codierend besagtes Fusionsprotein; und
b) einer oder mehrerer guide-RNAs oder einer oder mehrerer Nukleinsäuren, codierend besagte guide-RNAs, wobei besagte guide-RNAs eine RNA-Struktur für eine Bindung an die Cas9-Domäne des Fusionsproteins und eine für die chromosomale Zielregion komplementäre Sequenz umfassen; und
- Induktion des Eintritts in Prophase I der Meiose besagter Zelle.

2. Verfahren gemäß Anspruch 1, wobei das Fusionsprotein außerdem eine Kernlokalisierungssignalsequenz umfasst.

3. Verfahren gemäß einem der vorhergehenden Ansprüche, wobei die Cas9-Domäne ein für die Nukleaseaktivität defizientes Cas9-Protein ist.

4. Verfahren gemäß einem der vorhergehenden Ansprüche, wobei die Expression der besagtes Fusionsprotein codierenden Nukleinsäure unter der Kontrolle eines konstitutiven, induzierbaren oder Meiose-spezifischen Promotors steht.

5. Verfahren gemäß einem der vorhergehenden Ansprüche, umfassend außerdem die Einführung einer oder mehrerer zusätzlicher guide-RNAs, die auf eine oder mehrere andere chromosomale Regionen gerichtet sind, oder Nukleinsäuren, codierend besagte zusätzliche guide-RNAs.

6. Verfahren gemäß einem der vorhergehenden Ansprüche, wobei die Zelle eine Hefe ist und die Induktion des Eintritts in Prophase I vorzugsweise durch einen Transfer der Hefe von einem Vollmedium in ein Sporulationsmedium erhalten wird.

7. Verfahren gemäß einem der Ansprüche 1 bis 5, wobei die eukaryotische Zelle eine Pflanzenzelle ist.

8. Verfahren gemäß einem der Ansprüche 1 bis 7, wobei die Einführung des Fusionsproteins oder der dafür codierenden Nukleinsäure und der oder den gRNAs oder der oder den dafür codierenden Nukleinsäuren in besagte Zelle gleichzeitig oder aufeinanderfolgend erfolgt.

9. Verfahren gemäß einem der Ansprüche 1 bis 7, wobei die Einführung der Nukleinsäure, codierend für das Fusionsprotein, und der oder den Nukleinsäuren, codierend für die gRNA oder gRNAs, in besagte Zelle durch Kreuzen von zwei Zellen erhalten wird, in welche die Nukleinsäure, codierend für das Fusionsprotein, und die Nukleinsäure oder Nukleinsäuren, codierend für die gRNA oder gRNAs, jeweils eingeführt worden sind.

10. Fusionsprotein, definiert wie in einem der Ansprüche 1 bis 3.

11. Nukleinsäure, codierend für das Fusionsprotein gemäß Anspruch 10.

12. Expressionskassette oder Vektor, umfassend eine Nukleinsäure gemäß Anspruch 11.

13. Nicht-humane Wirtszelle umfassend ein Fusionsprotein gemäß Anspruch 10, eine Nukleinsäure gemäß Anspruch 11, eine Kassette oder einen Vektor gemäß Anspruch 12, wobei besagte Wirtszelle vorzugsweise eine eukaryotische Zelle, besonders bevorzugt eine Hefe-, Pflanzen-, Pilz- oder Tierzelle ist.

14. Wirtszelle gemäß Anspruch 13, wobei besagte Wirtszelle ist
- eine Hefezelle, vorzugsweise ausgewählt aus der Gruppe, bestehend aus *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus, Saccharomyces pastorianus* (bezeichnet auch als *Saccharomyces carlsbergensis)* und Hybriden, die von wenigstens einem Stamm erhalten sind, der zu einer dieser Arten gehört, besonders bevorzugt besagte Wirtszelle *Saccharomyces cerevisiae* ist, oder
- eine Pflanzenzelle, vorzugsweise eine Pflanzenzelle, ausgewählt aus der Gruppe, bestehend aus Reis, Weizen, Soja, Mais, Tomate, *Arabidopsis thaliana,* Gerste, Raps, Baumwolle, Zuckerrohr und Zuckerrübe, besonders bevorzugt besagte Wirtszelle eine Reiszelle ist.

15. Verfahren zur Erzeugung von Varianten eines eukaryotischen Organismus, außer Mensch, vorzugsweise einer Hefe oder einer Pflanze, umfassend:
- Einführung in eine Zelle besagten Organismus:
a) eines Fusionsproteins, umfassend eine Cas9-Domäne und eine Spo11-Domäne, oder einer Nukleinsäure, codierend besagtes Fusionsprotein; und
b) einer oder mehrerer guide-RNAs oder einer oder mehrerer Nukleinsäuren, codierend besagte guide-RNAs, wobei besagte guide-RNAs eine RNA-Struktur für eine Bindung an die Cas9-Domäne und eine für die chromosomale Zielregion komplementäre Sequenz umfassen; und
- Induktion des Eintritts in Prophase I der Meiose besagter Zelle;
- Erhalt einer Zelle oder von Zellen, aufweisend die erwünschte Rekombination oder erwünschten Rekombinationen auf Niveau der chromosomalen Zielregion oder Zielregionen; und
- Erzeugung einer Variante des Organismus von besagter rekombinierter Zelle.

16. Verfahren zur Identifizierung oder Lokalisierung der genetischen Information, codierend für ein Charakteristikum von Interesse, in einem Genom einer nicht-humanen eukaryotischen Zelle, vorzugsweise einer Hefe oder einer Pflanze, umfassend:
- Einführung in die eukaryotische Zelle:
a) eines Fusionsproteins, umfassend eine Cas9-Domäne und eine Spo11-Domäne, oder einer Nukleinsäure, codierend besagtes Fusionsprotein; und
b) einer oder mehrerer guide-RNAs oder einer oder mehrerer Nukleinsäuren, codierend besagte guide-RNAs, wobei besagte guide-RNAs eine RNA-Struktur für eine Bindung an die Cas9-Domäne und eine für die chromosomale Zielregion komplementäre Sequenz umfassen; und
- Induktion des Eintritts in Prophase I der Meiose besagter Zelle;
- Erhalt einer Zelle oder von Zellen, aufweisend die erwünschte Rekombination oder erwünschten Rekombinationen auf Niveau der chromosomalen Zielregion oder Zielregionen; und
- Analyse der Genotypen und Phänotypen der rekombinierten Zellen, um die genetische Information, codierend für das Charakteristikum von Interesse, zu identifizieren oder zu lokalisieren;
wobei das Charakteristikum von Interesse vorzugsweise ein quantitatives Merkmal von Interesse (QTL) ist.

17. Verwendung eines Kits umfassend ein Fusionsprotein gemäß Anspruch 10, eine Nukleinsäure gemäß Anspruch 11, eine Kassette oder einen Vektor gemäß Anspruch 12 oder eine Wirtszelle gemäß einem der Ansprüche 13 und 14, zur Durchführung eines Verfahrens gemäß einem der Ansprüche 1 bis 9, 15 und 16, insbesondere um (i) zielgerichtete meiotische Rekombinationen in einer nicht-humanen eukaryotischen Zelle zu induzieren, (ii) Varianten eines nicht-humanen eukaryotischen Organismus zu erzeugen, und/oder (iii) die genetische Information, die für ein Charakteristikum von Interesse codiert, in einem Genom einer nicht-humanen eukaryotischen Zelle zu identifizieren und zu lokalisieren.

## Claims

1. A method for inducing targeted meiotic recombinations in a non-human eukaryotic cell comprising
- introducing into said cell:
a) a fusion protein comprising a Cas9 domain and a Spo11 domain, or a nucleic acid encoding said fusion protein; and
b) one or more guide RNAs or one or more nucleic acids encoding said guide RNAs, said guide RNAs comprising an RNA structure for binding to the Cas9 domain of the fusion protein and a sequence complementary to the targeted chromosomal region; and
- inducing said cell to enter meiotic prophase I.

2. The method according to claim 1, wherein the fusion protein further comprises a nuclear localization signal sequence.

3. The method according to any one of the preceding claims, wherein the Cas9 domain is a nuclease-deficient Cas9 protein.

4. The method according to any one of the preceding claims, wherein the expression of the nucleic acid encoding said fusion protein is placed under the control of a constitutive, inducible or meiosis-specific promoter.

5. The method according to any one of the preceding claims, further comprising introducing one or more additional guide RNAs targeting one or more other chromosomal regions, or nucleic acids encoding said additional guide RNAs.

6. The method according to any one of the preceding claims, wherein the cell is a yeast, and the induction to enter prophase I is preferably obtained by transfer of the yeast from rich medium to sporulation medium.

7. The method according to any one of claims 1 to 5, wherein the eukaryotic cell is a plant cell.

8. The method according to any one of claims 1 to 7, wherein the introduction of the fusion protein, or the nucleic acid encoding same, and the gRNA(s), or the nucleic acid(s) encoding same, into said cell is simultaneous or sequential.

9. The method according to any one of claims 1 to 7, wherein the introduction of the nucleic acid encoding the fusion protein and the nucleic acid(s) encoding the gRNA(s) into said cell is obtained by crossing two cells into which have been respectively introduced the nucleic acid encoding the fusion protein and the nucleic acid(s) encoding the gRNA(s).

10. A fusion protein as defined in any one of claims 1 to 3.

11. A nucleic acid encoding the fusion protein according to claim 10.

12. An expression cassette or a vector comprising a nucleic acid according to claim 11.

13. A non-human host cell comprising a fusion protein according to claim 10, a nucleic acid according to claim 11, a cassette or vector according to claim 12, wherein said host cell is preferably a eukaryotic cell, even more preferably a yeast, plant, fungal or animal cell.

14. The host cell according to claim 13, wherein said host cell is:
- a yeast cell, preferably selected from the group consisting of *Saccharomyces cerevisiae, Saccharomyces bayanus, Saccharomyces castelli, Saccharomyces eubayanus, Saccharomyces kluyveri, Saccharomyces kudriavzevii, Saccharomyces mikatae, Saccharomyces uvarum, Saccharomyces paradoxus, Saccharomyces pastorianus* (also called *Saccharomyces carlsbergensis),* and the hybrids obtained from at least one strain belonging to one of these species, more preferably said host cell is *Saccharomyces cerevisiae,* or
- a plant cell, preferably a plant cell selected from the group consisting of rice, wheat, soy, maize, tomato, *Arabidopsis thaliana,* barley, rapeseed, cotton, sugarcane and beet, more preferably said host cell is a rice cell.

15. A method for generating variants of a eukaryotic organism, with the exception of humans, preferably of a yeast or a plant, comprising:
- introducing into a cell of said organism:
a) a fusion protein comprising a Cas9 domain and a Spo11 domain, or a nucleic acid encoding said fusion protein; and
b) one or more guide RNAs, or one or more nucleic acids encoding said guide RNAs, said guide RNAs comprising an RNA structure for binding to the Cas9 domain and a sequence complementary to a targeted chromosomal region; and
- inducing said cell to enter meiotic prophase I;
- obtaining a cell or cells having the desired recombination(s) at the targeted chromosomal region(s); and
- generating a variant of the organism from said recombinant cell.

16. A method for identifying or locating the genetic information encoding a characteristic of interest in a non-human eukaryotic cell genome, preferably a yeast genome or a plant genome, comprising:
- introducing into the eukaryotic cell:
a) a fusion protein comprising a Cas9 domain and a Spo11 domain, or a nucleic acid encoding said fusion protein; and
b) one or more guide RNAs, or one or more nucleic acids encoding said guide RNAs, said guide RNAs comprising an RNA structure for binding to the Cas9 domain and a sequence complementary to a targeted chromosomal region; and
- inducing said cell to enter meiotic prophase I;
- obtaining a cell or cells having the desired recombination(s) at the targeted chromosomal region(s); and
- analyzing the genotypes and phenotypes of the recombinant cells in order to identify or to locate the genetic information encoding the characteristic of interest
the characteristic of interest being preferably a quantitative trait of interest (QTL).

17. Use of a kit comprising a fusion protein according to claim 10, a nucleic acid according to claim 11, a cassette or a vector according to claim 12, or a host cell according to any one of claims 13 and 14, to implement a method according to any one of claims 1 to 9, 15 and 16, in particular to (i) induce targeted meiotic recombinations in a non-human eukaryotic cell, (ii) generate variants of a non-human eukaryotic organism, and/or (iii) identify or locate the genetic information encoding a characteristic of interest in a non-human eukaryotic cell genome.


### SEQUENCE LISTING

<110> MEIOGENIX UNIVERSITE PIERRE ET MARIE CURIE (PARIS 6) INSTITUT CURIE CENTRE NATIONAL DE LA RECHERCHE SCIENTIFIQUE
<120> Procédé pour induire des recombinaisons méiotiques ciblées
<130> B1960PC
<160> 18
<170> PatentIn version 3.5
<210> 1
   <211> 12349
   <212> DNA
   <213> Artificial Sequence
<220>
   <223> Plasmide P1 comprenant un acide nucléique codant pour la protéine de fusion Cas9 sauvage-Spoll
<220>
   <221> promoter
   <222> (1)..(397)
   <223> promoteur ADH1
<220>
   <221> gene
   <222> (431)..(5974)
   <223> Gène codant la protéine de fusion Cas9-Spo11
<220>
   <221> terminator
   <222> (6010)..(6198)
   <223> terminateur ADH1
<220>
   <221> rep_origin
   <222> (6605)..(7287)
   <223> Origine de réplication bactérienne ColE1
<220>
   <221> gene
   <222> (7385)..(8045)
   <223> Gène de résistance à l'ampiciline
<220>
   <221> gene
   <222> (8809)..(9631)
   <223> Gène TRP1
<220>
   <221> rep_origin
   <222> (9688)..(10126)
   <223> Origine de réplication F1
<220>
   <221> gene
   <222> (10344)..(11363)
   <223> Gène de résistance au G418 (KanMx)
<400> 1
   caacttcttt tctttttttt tcttttctct ctcccccgtt gttgtctcac catatccgca 60
   atgacaaaaa aatgatggaa gacactaaag gaaaaaatta acgacaaaga cagcaccaac 120
   agatgtcgtt gttccagagc tgatgagggg tatctcgaag cacacgaaac tttttccttc 180
   cttcattcac gcacactact ctctaatgag caacggtata cggccttcct tccagttact 240
   tgaatttgaa ataaaaaaaa gtttgctgtc ttgctatcaa gtataaatag acctgcaatt 300
   attaatcttt tgtttcctcg tcattgttct cgttcccttt cttccttgtt tctttttctg 360
   cacaatattt caagctatac caagcataca atcaactcca agcttgaagc aagcctcctg 420
   aaagactagt atgggaaaac ctattcctaa tcctctgctg ggcctggatt ctaccggagg 480
   catggcccct aagaaaaagc ggaaggtgga cggcggaggt attcatggag ttcctgctgc 540
   gatggacaag aagtattcta tcggactgga tatcgggact aatagcgtcg ggtgggccgt 600
   gatcactgac gagtacaagg tgccctctaa gaagttcaag gtgctcggga acaccgaccg 660
   gcattccatc aagaaaaatc tgatcggagc tctcctcttt gattcagggg agaccgctga 720
   agcaacccgc ctcaagcgga ctgctagacg gcggtacacc aggaggaaga accggatttg 780
   ttaccttcaa gagatattct ccaacgaaat ggcaaaggtc gacgacagct tcttccatag 840
   gctggaagaa tcattcctcg tggaagagga taagaagcat gaacggcatc ccatcttcgg 900
   taatatcgtc gacgaggtgg cctatcacga gaaataccca accatctacc atcttcgcaa 960
   aaagctggtg gactcaaccg acaaggcaga cctccggctt atctacctgg ccctggccca 1020
   catgatcaag ttcagaggcc acttcctgat cgagggcgac ctcaatcctg acaatagcga 1080
   tgtggataaa ctgttcatcc agctggtgca gacttacaac cagctctttg aagagaaccc 1140
   catcaatgca agcggagtcg atgccaaggc cattctgtca gcccggctgt caaagagccg 1200
   cagacttgag aatcttatcg ctcagctgcc gggtgaaaag aaaaatggac tgttcgggaa 1260
   cctgattgct ctttcacttg ggctgactcc caatttcaag tctaatttcg acctggcaga 1320
   ggatgccaag ctgcaactgt ccaaggacac ctatgatgac gatctcgaca acctcctggc 1380
   ccagatcggt gaccaatacg ccgacctttt ccttgctgct aagaatcttt ctgacgccat 1440
   cctgctgtct gacattctcc gcgtgaacac tgaaatcacc aaggcccctc tttcagcttc 1500
   aatgattaag cggtatgatg agcaccacca ggacctgacc ctgcttaagg cactcgtccg 1560
   gcagcagctt ccggagaagt acaaggaaat cttctttgac cagtcaaaga atggatacgc 1620
   cggctacatc gacggaggtg cctcccaaga ggaattttat aagtttatca aacctatcct 1680
   tgagaagatg gacggcaccg aagagctcct cgtgaaactg aatcgggagg atctgctgcg 1740
   gaagcagcgc actttcgaca atgggagcat tccccaccag atccatcttg gggagcttca 1800
   cgccatcctt cggcgccaag aggacttcta cccctttctt aaggacaaca gggagaagat 1860
   tgagaaaatt ctcactttcc gcatccccta ctacgtggga cccctcgcca gaggaaatag 1920
   ccggtttgct tggatgacca gaaagtcaga agaaactatc actccctgga acttcgaaga 1980
   ggtggtggac aagggagcca gcgctcagtc attcatcgaa cggatgacta acttcgataa 2040
   gaacctcccc aatgagaagg tcctgccgaa acattccctg ctctacgagt actttaccgt 2100
   gtacaacgag ctgaccaagg tgaaatatgt caccgaaggg atgaggaagc ccgcattcct 2160
   gtcaggcgaa caaaagaagg caattgtgga ccttctgttc aagaccaata gaaaggtgac 2220
   cgtgaagcag ctgaaggagg actatttcaa gaaaattgaa tgcttcgact ctgtggagat 2280
   tagcggggtc gaagatcggt tcaacgcaag cctgggtacc taccatgatc tgcttaagat 2340
   catcaaggac aaggattttc tggacaatga ggagaacgag gacatccttg aggacattgt 2400
   cctgactctc actctgttcg aggaccggga aatgatcgag gagaggctta agacctacgc 2460
   ccatctgttc gacgataaag tgatgaagca acttaaacgg agaagatata ccggatgggg 2520
   acgccttagc cgcaaactca tcaacggaat ccgggacaaa cagagcggaa agaccattct 2580
   tgatttcctt aagagcgacg gattcgctaa tcgcaacttc atgcaactta tccatgatga 2640
   ttccctgacc tttaaggagg acatccagaa ggcccaagtg tctggacaag gtgactcact 2700
   gcacgagcat atcgcaaatc tggctggttc acccgctatt aagaagggta ttctccagac 2760
   cgtgaaagtc gtggacgagc tggtcaaggt gatgggtcgc cataaaccag agaacattgt 2820
   catcgagatg gccagggaaa accagactac ccagaaggga cagaagaaca gcagggagcg 2880
   gatgaaaaga attgaggaag ggattaagga gctcgggtca cagatcctta aagagcaccc 2940
   ggtggaaaac acccagcttc agaatgagaa gctctatctg tactaccttc aaaatggacg 3000
   cgatatgtat gtggaccaag agcttgatat caacaggctc tcagactacg acgtggacca 3060
   tatcgtccct cagagcttcc tcaaagacga ctcaattgac aataaggtgc tgactcgctc 3120
   agacaagaac cggggaaagt cagataacgt gccctcagag gaagtcgtga aaaagatgaa 3180
   gaactattgg cgccagcttc tgaacgcaaa gctgatcact cagcggaagt tcgacaatct 3240
   cactaaggct gagaggggcg gactgagcga actggacaaa gcaggattca ttaaacggca 3300
   acttgtggag actcggcaga ttactaaaca tgtcgcccaa atccttgact cacgcatgaa 3360
   taccaagtac gacgaaaacg acaaacttat ccgcgaggtg aaggtgatta ccctgaagtc 3420
   caagctggtc agcgatttca gaaaggactt tcaattctac aaagtgcggg agatcaataa 3480
   ctatcatcat gctcatgacg catatctgaa tgccgtggtg ggaaccgccc tgatcaagaa 3540
   gtacccaaag ctggaaagcg agttcgtgta cggagactac aaggtctacg acgtgcgcaa 3600
   gatgattgcc aaatctgagc aggagatcgg aaaggccacc gcaaagtact tcttctacag     3660
   caacatcatg aatttcttca agaccgaaat cacccttgca aacggtgaga tccggaagag     3720
   gccgctcatc gagactaatg gggagactgg cgaaatcgtg tgggacaagg gcagagattt 3780
   cgctaccgtg cgcaaagtgc tttctatgcc tcaagtgaac atcgtgaaga aaaccgaggt 3840
   gcaaaccgga ggcttttcta aggaatcaat cctccccaag cgcaactccg acaagctcat 3900
   tgcaaggaag aaggattggg accctaagaa gtacggcgga ttcgattcac caactgtggc 3960
   ttattctgtc ctggtcgtgg ctaaggtgga aaaaggaaag tctaagaagc tcaagagcgt 4020
   gaaggaactg ctgggtatca ccattatgga gcgcagctcc ttcgagaaga acccaattga 4080
   ctttctcgaa gccaaaggtt acaaggaagt caagaaggac cttatcatca agctcccaaa 4140
   gtatagcctg ttcgaactgg agaatgggcg gaagcggatg ctcgcctccg ctggcgaact 4200
   tcagaagggt aatgagctgg ctctcccctc caagtacgtg aatttcctct accttgcaag 4260
   ccattacgag aagctgaagg ggagccccga ggacaacgag caaaagcaac tgtttgtgga 4320
   gcagcataag cattatctgg acgagatcat tgagcagatt tccgagtttt ctaaacgcgt 4380
   cattctcgct gatgccaacc tcgataaagt ccttagcgca tacaataagc acagagacaa 4440
   accaattcgg gagcaggctg agaatatcat ccacctgttc accctcacca atcttggtgc 4500
   ccctgccgca ttcaagtact tcgacaccac catcgaccgg aaacgctata cctccaccaa 4560
   agaagtgctg gacgccaccc tcatccacca gagcatcacc ggactttacg aaactcggat 4620
   tgacctctca cagctcggag gggatccgga atttatggcc atggaggccc cggggatccg 4680
   tatggctttg gagggattgc ggaaaaaata taaaacaagg caggaattgg tcaaagcact 4740
   cactcctaaa agacggtcca ttcacttgaa ctccaatggt cactccaacg gaactccctg 4800
   ttcaaacgca gatgttttgg ctcatattaa gcatttcctg tcattggcgg ctaattcatt 4860
   agagcaacat caacagccta tttcaatcgt ctttcaaaac aaaaaaaaaa aaggcgatac 4920
   aagcagtcct gacattcaca caacattgga cttccctttg aatggcccgc atctatgcac 4980
   tcatcagttc aagttgaaaa gatgcgcaat ccttttaaac ttattgaaag tcgttatgga 5040
   aaaattaccg ctaggtaaaa acactacagt gagagatatc ttctactcca acgtggaatt 5100
   gtttcaaaga caagcaaacg tagtccagtg gctggacgtt atacgcttta atttcaagct 5160
   ctctccaaga aaatccttaa acattatacc agctcaaaag ggtttagttt attcgccttt 5220
   ccccattgat atttatgaca atattctgac atgtgaaaat gaaccaaaga tgcaaaagca 5280
   aacaattttc cctggtaagc cctgtctaat tccatttttc caagatgatg cggtcatcaa 5340
   gttagggaca acaagtatgt gtaatattgt aatagtggaa aaagaagctg tcttcaccaa 5400
   attagtaaat aattatcaca agttgagtac aaataccatg ctcattacag gtaagggatt 5460
   tccagatttc ttgacaaggt tattcctaaa aaaactagaa caatattgct ccaaattgat 5520
   atcggactgt tctatattta ccgatgcgga cccctatggg attagcatag ccctaaatta 5580
   tactcactcg aatgaacgca acgcttatat ttgcacgatg gcaaactata aaggaattcg 5640
   tattacgcaa gttttggcac aaaataatga agtgcataac aaatccattc aattattgag 5700
   tttgaatcag cgcgactact ccttagccaa gaatttgata gcatctctga ctgccaacag 5760
   ctgggatatt gcaacttcac cattaaagaa cgtcatcata gaatgtcagc gggaaatttt 5820
   tttccaaaag aaagctgaaa tgaacgagat tgatgccaga atttttgaat acaaatccca 5880
   ccaccatcat catcacggag actacaagga tgacgatgac aaggactaca aggatgacga 5940
   tgacaaggac tacaaggatg acgatgacaa gtaatgagtc gacaacccct gcagccaagc 6000
   taattccggg cgaatttctt atgatttatg atttttatta ttaaataagt tataaaaaaa 6060
   ataagtgtat acaaatttta aagtgactct taggttttaa aacgaaaatt cttgttcttg 6120
   agtaactctt tcctgtaggt caggttgctt tctcaggtat agcatgaggt cgctcttatt 6180
   gaccacacct ctaccggcat gcaagcttgg cgtaatcatg gtcatagctg tttcctgtgt 6240
   gaaattgtta tccgctcaca attccacaca acatacgagc cggaagcata aagtgtaaag 6300
   cctggggtgc ctaatgagtg aggtaactca cattaattgc gttgcgctca ctgcccgctt 6360
   tccagtcggg aaacctgtcg tgccagctgg attaatgaat cggccaacgc gcggggagag 6420
   gcggtttgcg tattgggcgc tcttccgctt cctcgctcac tgactcgctg cgctcggtcg 6480
   ttcggctgcg gcgagcggta tcagctcact caaaggcggt aatacggtta tccacagaat 6540
   caggggataa cgcaggaaag aacatgtgag caaaaggcca gcaaaaggcc aggaaccgta 6600
   aaaaggccgc gttgctggcg tttttccata ggctccgccc ccctgacgag catcacaaaa 6660
   atcgacgctc aagtcagagg tggcgaaacc cgacaggact ataaagatac caggcgtttc 6720
   cccctggaag ctccctcgtg cgctctcctg ttccgaccct gccgcttacc ggatacctgt 6780
   ccgcctttct cccttcggga agcgtggcgc tttctcatag ctcacgctgt aggtatctca 6840
   gttcggtgta ggtcgttcgc tccaagctgg gctgtgtgca cgaacccccc gttcagcccg 6900
   accgctgcgc cttatccggt aactatcgtc ttgagtccaa cccggtaaga cacgacttat 6960
   cgccactggc agcagccact ggtaacagga ttagcagagc gaggtatgta ggcggtgcta 7020
   cagagttctt gaagtggtgg cctaactacg gctacactag aaggacagta tttggtatct 7080
   gcgctctgct gaagccagtt accttcggaa aaagagttgg tagctcttga tccggcaaac 7140
   aaaccaccgc tggtagcggt ggtttttttg tttgcaagca gcagattacg cgcagaaaaa 7200
   aaggatctca agaagatcct ttgatctttt ctacggggtc tgacgctcag tggaacgaaa 7260
   actcacgtta agggattttg gtcatgagat tatcaaaaag gatcttcacc tagatccttt 7320
   taaattaaaa atgaagtttt aaatcaatct aaagtatata tgagtaaact tggtctgaca 7380
   gttaccaatg cttaatcagt gaggcaccta tctcagcgat ctgtctattt cgttcatcca 7440
   tagttgcctg actccccgtc gtgtagataa ctacgatacg ggagggctta ccatctggcc 7500
   ccagtgctgc aatgataccg cgagacccac gctcaccggc ctccagattt atcagcaata 7560
   aaccagccag ccggaagggc cgagcgcaga agtggtcctg caactttatc cgcctccatc 7620
   cagtctatta attgttgccg ggaagctaga gtaagtagtt cgccagttaa tagtttgcgc 7680
   aacgttgttg ccattgctac aggcatcgtg gtgtcacgct cgtcgtttgg tatggcttca 7740
   ttcagctccg gttcccaacg atcaaggcga gttacatgat cccccatgtt gtgcaaaaaa 7800
   gcggttagct ccttcggtcc tccgatcgtt gtcagaagta agttggccgc agtgttatca 7860
   ctcatggtta tggcagcact gcataattct cttactgtca tgccatccgt aagatgcttt 7920
   tctgtgactg gtgagtactc aaccaagtca ttctgagaat agtgtatgcg gcgaccgagt 7980
   tgctcttgcc cggcgtcaat acgggataat accgcgccac atagcagaac tttaaaagtg 8040
   ctcatcattg gaaaacgttc ttcggggcga aaactctcaa ggatcttacc gctgttgaga 8100
   tccagttcga tgtaacccac tcgtgcaccc aactgatctt cagcatcttt tactttcacc 8160
   agcgtttctg ggtgagcaaa aacaggaagg caaaatgccg caaaaaaggg aataagggcg 8220
   acacggaaat gttgaatact catactcttc ctttttcaat attattgaag catttatcag 8280
   ggttattgtc tcatgagcgg atacatattt gaatgtattt agaaaaataa acaaataggg 8340
   gttccgcgca catttccccg aaaagtgcca cctgacgtct aagaaaccat tattatcatg 8400
   acattaacct ataaaaatag gcgtatcacg aggccctttc gtctcgcgcg tttcggtgat 8460
   gacggtgaaa acctctgaca catgcagctc ccggagacgg tcacagcttg tctgtaagcg 8520
   gatgccggga gcagacaagc ccgtcagggc gcgtcagcgg gtgttggcgg gtgtcggggc 8580
   tggcttaact atgcggcatc agagcagatt gtactgagag tgcaccataa acgacattac 8640
   tatatatata atataggaag catttaatag acagcatcgt aatatatgtg tactttgcag 8700
   ttatgacgcc agatggcagt agtggaagat attctttatt gaaaaatagc ttgtcacctt 8760
   acgtacaatc ttgatccgga gcttttcttt ttttgccgat taagaattaa ttcggtcgaa 8820
   aaaagaaaag gagagggcca agagggaggg cattggtgac tattgagcac gtgagtatac 8880
   gtgattaagc acacaaaggc agcttggagt atgtctgtta ttaatttcac aggtagttct 8940
   ggtccattgg tgaaagtttg cggcttgcag agcacagagg ccgcagaatg tgctctagat 9000
   tccgatgctg acttgctggg tattatatgt gtgcccaata gaaagagaac aattgacccg 9060
   gttattgcaa ggaaaatttc aagtcttgta aaagcatata aaaatagttc aggcactccg 9120
   aaatacttgg ttggcgtgtt tcgtaatcaa cctaaggagg atgttttggc tctggtcaat 9180
   gattacggca ttgatatcgt ccaactgcat ggagatgagt cgtggcaaga ataccaagag 9240
   ttcctcggtt tgccagttat taaaagactc gtatttccaa aagactgcaa catactactc 9300
   agtgcagctt cacagaaacc tcattcgttt attcccttgt ttgattcaga agcaggtggg 9360
   acaggtgaac ttttggattg gaactcgatt tctgactggg ttggaaggca agagagcccc 9420
   gaaagcttac attttatgtt agctggtgga ctgacgccag aaaatgttgg tgatgcgctt 9480
   agattaaatg gcgttattgg tgttgatgta agcggaggtg tggagacaaa tggtgtaaaa 9540
   gactctaaca aaatagcaaa tttcgtcaaa aatgctaaga aataggttat tactgagtag 9600
   tatttattta agtattgttt gtgcacttgc cgatctatgc ggtgtgaaat accgcacaga 9660
   tgcgtaagga gaaaataccg catcaggaaa ttgtaaacgt taatattttg ttaaaattcg 9720
   cgttaaattt ttgttaaatc agctcatttt ttaaccaata ggccgaaatc ggcaaaatcc 9780
   cttataaatc aaaagaatag accgagatag ggttgagtgt tgttccagtt tggaacaaga 9840
   gtccactatt aaagaacgtg gactccaacg tcaaagggcg aaaaaccgtc tatcagggcg 9900
   atggcccact acgtgaacca tcaccctaat caagtttttt ggggtcgagg tgccgtaaag 9960
   cactaaatcg gaaccctaaa gggagccccc gatttagagc ttgacgggga aagccggcga 10020
   acgtggcgag aaaggaaggg aagaaagcga aaggagcggg cgctagggcg ctggcaagtg 10080
   tagcggtcac gctgcgcgta accaccacac ccgccgcgct taatgcgccg ctacagggcg 10140
   cgtcgcgcca ttcgccattc aggctgcgca actgttggga agggcgatcg gtgcgggcct 10200
   cttcgctatt acgccagctg gcgaaagggg gatgtgctgc aaggcgatta agttgggtaa 10260
   cgccagggtt ttcccagtca cgacgttgta aaacgacggc cagtcgtcca agctttcgat 10320
   catcgatgaa ttcgagctcg ttttcgacag cagtatagcg accagcattc acatacgatt 10380
   gacgcatgat attactttct gcgcacttaa cttcgcatct gggcagatga tgtcgaggcg 10440
   aaaaaaaata taaatcacgc taacatttga ttaaaataga acaactacaa tataaaaaaa 10500
   ctatacaaat gacaagttct tgaaaacaag aatcttttta ttgtcagtac tgattagaaa 10560
   aactcatcga gcatcaaatg aaactgcaat ttattcatat caggattatc aataccatat 10620
   ttttgaaaaa gccgtttctg taatgaagga gaaaactcac cgaggcagtt ccataggatg 10680
   gcaagatcct ggtatcggtc tgcgattccg actcgtccaa catcaataca acctattaat 10740
   ttcccctcgt caaaaataag gttatcaagt gagaaatcac catgagtgac gactgaatcc 10800
   ggtgagaatg gcaaaagctt atgcatttct ttccagactt gttcaacagg ccagccatta 10860
   cgctcgtcat caaaatcact cgcatcaacc aaaccgttat tcattcgtga ttgcgcctga 10920
   gcgagacgaa atacgcgatc gctgttaaaa ggacaattac aaacaggaat cgaatgcaac 10980
   cggcgcagga acactgccag cgcatcaaca atattttcac ctgaatcagg atattcttct 11040
   aatacctgga atgctgtttt gccggggatc gcagtggtga gtaaccatgc atcatcagga 11100
   gtacggataa aatgcttgat ggtcggaaga ggcataaatt ccgtcagcca gtttagtctg 11160
   accatctcat ctgtaacatc attggcaacg ctacctttgc catgtttcag aaacaactct 11220
   ggcgcatcgg gcttcccata caatcgatag attgtcgcac ctgattgccc gacattatcg 11280
   cgagcccatt tatacccata taaatcagca tccatgttgg aatttaatcg cggcctcgaa 11340
   acgtgagtct tttccttacc catggttgtt tatgttcgga tgtgatgtga gaactgtatc 11400
   ctagcaagat tttaaaagga agtatatgaa agaagaacct cagtggcaaa tcctaacctt 11460
   ttatatttct ctacaggggc gcggcgtggg gacaattcaa cgcgtctgtg aggggagcgt 11520
   ttccctgctc gcaggtctgc agcgaggagc cgtaattttt gcttcgcgcc gtgcggccat 11580
   caaaatgtat ggatgcaaat gattatacat ggggatgtat gggctaaatg tacgggcgac 11640
   agtcacatca tgcccctgag ctgcgcacgt caagactgtc aaggagggta ttctgggcct 11700
   ccatgtcgct ggccgggtga cccggcgggg acgaggcaag ctaaacagat ctggcgcgcc 11760
   ttaattaacc ccgagctcga gatcccgagc ttgcaaatta aagccttcga gcgtcccaaa 11820
   accttctcaa gcaaggtttt cagtataatg ttacatgcgt acacgcgtct gtacagaaaa 11880
   aaaagaaaaa tttgaaatat aaataacgtt cttaatacta acataactat aaaaaaataa 11940
   atagggacct agacttcagg ttgtctaact ccttcctttt cggttagagc ggatgtgggg 12000
   ggagggcgtg aatgtaagcg tgacataact aattacatga tatccttttg ttgtttccgg 12060
   gtgtacaata tggacttcct cttttctggc aaccaaaccc atacatcggg attcctataa 12120
   taccttcgtt ggtctcccta acatgtaggt ggcggagggg agatatacaa tagaacagat 12180
   accagacaag acataatggg ctaaacaaaa ctacaccaat tacactgcct cattgatggt 12240
   ggtacataac gaactaatac tgtagcccta gacttgatag ccatcatcat atcgaagttt 12300
   cactaccctt tttccatttg ccatctattg aagtaataat aggcgcatg 12349
<210> 2
   <211> 12349
   <212> DNA
   <213> Artificial Sequence
<220>
   <223> Plasmide P2 comprenant un acide nucléique codant pour la protéine de fusion Cas9*-Spo11
<220>
   <221> promoter
   <222> (1)..(397)
   <223> Promoteur ADH1
<220>
   <221> gene
   <222> (431)..(5974)
   <223> Gène codant la protéine de fusion Ca9*-Spo11
<220>
   <221> terminator
   <222> (6010)..(6198)
   <223> Terminateur ADH1
<220>
   <221> rep_origin
   <222> (6605)..(7287)
   <223> Origine de réplication bactérienne ColE1
<220>
   <221> gene
   <222> (7385)..(8045)
   <223> Gène de résistance à l'ampiciline
<220>
   <221> gene
   <222> (8809)..(9631)
   <223> Gène TRP1
<220>
   <221> rep_origin
   <222> (9688)..(10126)
   <223> Origine de réplication F1
<220>
   <221> gene
   <222> (10344)..(11363)
   <223> Gène de résistance au G418 (KanMX)
<400> 2
   caacttcttt tctttttttt tcttttctct ctcccccgtt gttgtctcac catatccgca        60
   atgacaaaaa aatgatggaa gacactaaag gaaaaaatta acgacaaaga cagcaccaac       120
   agatgtcgtt gttccagagc tgatgagggg tatctcgaag cacacgaaac tttttccttc       180
   cttcattcac gcacactact ctctaatgag caacggtata cggccttcct tccagttact       240
   tgaatttgaa ataaaaaaaa gtttgctgtc ttgctatcaa gtataaatag acctgcaatt       300
   attaatcttt tgtttcctcg tcattgttct cgttcccttt cttccttgtt tctttttctg       360
   cacaatattt caagctatac caagcataca atcaactcca agcttgaagc aagcctcctg       420
   aaagactagt atgggaaaac ctattcctaa tcctctgctg ggcctggatt ctaccggagg       480
   catggcccct aagaaaaagc ggaaggtgga cggcggaggt attcatggag ttcctgctgc       540
   gatggacaag aagtattcta tcggactggc catcgggact aatagcgtcg ggtgggccgt       600
   gatcactgac gagtacaagg tgccctctaa gaagttcaag gtgctcggga acaccgaccg       660
   gcattccatc aagaaaaatc tgatcggagc tctcctcttt gattcagggg agaccgctga       720
   agcaacccgc ctcaagcgga ctgctagacg gcggtacacc aggaggaaga accggatttg       780
   ttaccttcaa gagatattct ccaacgaaat ggcaaaggtc gacgacagct tcttccatag       840
   gctggaagaa tcattcctcg tggaagagga taagaagcat gaacggcatc ccatcttcgg       900
   taatatcgtc gacgaggtgg cctatcacga gaaataccca accatctacc atcttcgcaa       960
   aaagctggtg gactcaaccg acaaggcaga cctccggctt atctacctgg ccctggccca      1020
   catgatcaag ttcagaggcc acttcctgat cgagggcgac ctcaatcctg acaatagcga      1080
   tgtggataaa ctgttcatcc agctggtgca gacttacaac cagctctttg aagagaaccc      1140
   catcaatgca agcggagtcg atgccaaggc cattctgtca gcccggctgt caaagagccg 1200
   cagacttgag aatcttatcg ctcagctgcc gggtgaaaag aaaaatggac tgttcgggaa 1260
   cctgattgct ctttcacttg ggctgactcc caatttcaag tctaatttcg acctggcaga 1320
   ggatgccaag ctgcaactgt ccaaggacac ctatgatgac gatctcgaca acctcctggc 1380
   ccagatcggt gaccaatacg ccgacctttt ccttgctgct aagaatcttt ctgacgccat 1440
   cctgctgtct gacattctcc gcgtgaacac tgaaatcacc aaggcccctc tttcagcttc 1500
   aatgattaag cggtatgatg agcaccacca ggacctgacc ctgcttaagg cactcgtccg 1560
   gcagcagctt ccggagaagt acaaggaaat cttctttgac cagtcaaaga atggatacgc 1620
   cggctacatc gacggaggtg cctcccaaga ggaattttat aagtttatca aacctatcct 1680
   tgagaagatg gacggcaccg aagagctcct cgtgaaactg aatcgggagg atctgctgcg 1740
   gaagcagcgc actttcgaca atgggagcat tccccaccag atccatcttg gggagcttca 1800
   cgccatcctt cggcgccaag aggacttcta cccctttctt aaggacaaca gggagaagat 1860
   tgagaaaatt ctcactttcc gcatccccta ctacgtggga cccctcgcca gaggaaatag 1920
   ccggtttgct tggatgacca gaaagtcaga agaaactatc actccctgga acttcgaaga 1980
   ggtggtggac aagggagcca gcgctcagtc attcatcgaa cggatgacta acttcgataa 2040
   gaacctcccc aatgagaagg tcctgccgaa acattccctg ctctacgagt actttaccgt 2100
   gtacaacgag ctgaccaagg tgaaatatgt caccgaaggg atgaggaagc ccgcattcct 2160
   gtcaggcgaa caaaagaagg caattgtgga ccttctgttc aagaccaata gaaaggtgac 2220
   cgtgaagcag ctgaaggagg actatttcaa gaaaattgaa tgcttcgact ctgtggagat 2280
   tagcggggtc gaagatcggt tcaacgcaag cctgggtacc taccatgatc tgcttaagat 2340
   catcaaggac aaggattttc tggacaatga ggagaacgag gacatccttg aggacattgt 2400
   cctgactctc actctgttcg aggaccggga aatgatcgag gagaggctta agacctacgc 2460
   ccatctgttc gacgataaag tgatgaagca acttaaacgg agaagatata ccggatgggg 2520
   acgccttagc cgcaaactca tcaacggaat ccgggacaaa cagagcggaa agaccattct 2580
   tgatttcctt aagagcgacg gattcgctaa tcgcaacttc atgcaactta tccatgatga 2640
   ttccctgacc tttaaggagg acatccagaa ggcccaagtg tctggacaag gtgactcact 2700
   gcacgagcat atcgcaaatc tggctggttc acccgctatt aagaagggta ttctccagac 2760
   cgtgaaagtc gtggacgagc tggtcaaggt gatgggtcgc cataaaccag agaacattgt 2820
   catcgagatg gccagggaaa accagactac ccagaaggga cagaagaaca gcagggagcg 2880
   gatgaaaaga attgaggaag ggattaagga gctcgggtca cagatcctta aagagcaccc 2940
   ggtggaaaac acccagcttc agaatgagaa gctctatctg tactaccttc aaaatggacg 3000
   cgatatgtat gtggaccaag agcttgatat caacaggctc tcagactacg acgtggacgc 3060
   tatcgtccct cagagcttcc tcaaagacga ctcaattgac aataaggtgc tgactcgctc 3120
   agacaagaac cggggaaagt cagataacgt gccctcagag gaagtcgtga aaaagatgaa 3180
   gaactattgg cgccagcttc tgaacgcaaa gctgatcact cagcggaagt tcgacaatct 3240
   cactaaggct gagaggggcg gactgagcga actggacaaa gcaggattca ttaaacggca 3300
   acttgtggag actcggcaga ttactaaaca tgtcgcccaa atccttgact cacgcatgaa 3360
   taccaagtac gacgaaaacg acaaacttat ccgcgaggtg aaggtgatta ccctgaagtc 3420
   caagctggtc agcgatttca gaaaggactt tcaattctac aaagtgcggg agatcaataa 3480
   ctatcatcat gctcatgacg catatctgaa tgccgtggtg ggaaccgccc tgatcaagaa 3540
   gtacccaaag ctggaaagcg agttcgtgta cggagactac aaggtctacg acgtgcgcaa 3600
   gatgattgcc aaatctgagc aggagatcgg aaaggccacc gcaaagtact tcttctacag 3660
   caacatcatg aatttcttca agaccgaaat cacccttgca aacggtgaga tccggaagag 3720
   gccgctcatc gagactaatg gggagactgg cgaaatcgtg tgggacaagg gcagagattt 3780
   cgctaccgtg cgcaaagtgc tttctatgcc tcaagtgaac atcgtgaaga aaaccgaggt 3840
   gcaaaccgga ggcttttcta aggaatcaat cctccccaag cgcaactccg acaagctcat 3900
   tgcaaggaag aaggattggg accctaagaa gtacggcgga ttcgattcac caactgtggc 3960
   ttattctgtc ctggtcgtgg ctaaggtgga aaaaggaaag tctaagaagc tcaagagcgt 4020
   gaaggaactg ctgggtatca ccattatgga gcgcagctcc ttcgagaaga acccaattga 4080
   ctttctcgaa gccaaaggtt acaaggaagt caagaaggac cttatcatca agctcccaaa 4140
   gtatagcctg ttcgaactgg agaatgggcg gaagcggatg ctcgcctccg ctggcgaact 4200
   tcagaagggt aatgagctgg ctctcccctc caagtacgtg aatttcctct accttgcaag 4260
   ccattacgag aagctgaagg ggagccccga ggacaacgag caaaagcaac tgtttgtgga 4320
   gcagcataag cattatctgg acgagatcat tgagcagatt tccgagtttt ctaaacgcgt 4380
   cattctcgct gatgccaacc tcgataaagt ccttagcgca tacaataagc acagagacaa 4440
   accaattcgg gagcaggctg agaatatcat ccacctgttc accctcacca atcttggtgc 4500
   ccctgccgca ttcaagtact tcgacaccac catcgaccgg aaacgctata cctccaccaa 4560
   agaagtgctg gacgccaccc tcatccacca gagcatcacc ggactttacg aaactcggat 4620
   tgacctctca cagctcggag gggatccgga atttatggcc atggaggccc cggggatccg 4680
   tatggctttg gagggattgc ggaaaaaata taaaacaagg caggaattgg tcaaagcact 4740
   cactcctaaa agacggtcca ttcacttgaa ctccaatggt cactccaacg gaactccctg 4800
   ttcaaacgca gatgttttgg ctcatattaa gcatttcctg tcattggcgg ctaattcatt 4860
   agagcaacat caacagccta tttcaatcgt ctttcaaaac aaaaaaaaaa aaggcgatac 4920
   aagcagtcct gacattcaca caacattgga cttccctttg aatggcccgc atctatgcac 4980
   tcatcagttc aagttgaaaa gatgcgcaat ccttttaaac ttattgaaag tcgttatgga 5040
   aaaattaccg ctaggtaaaa acactacagt gagagatatc ttctactcca acgtggaatt 5100
   gtttcaaaga caagcaaacg tagtccagtg gctggacgtt atacgcttta atttcaagct 5160
   ctctccaaga aaatccttaa acattatacc agctcaaaag ggtttagttt attcgccttt 5220
   ccccattgat atttatgaca atattctgac atgtgaaaat gaaccaaaga tgcaaaagca 5280
   aacaattttc cctggtaagc cctgtctaat tccatttttc caagatgatg cggtcatcaa 5340
   gttagggaca acaagtatgt gtaatattgt aatagtggaa aaagaagctg tcttcaccaa 5400
   attagtaaat aattatcaca agttgagtac aaataccatg ctcattacag gtaagggatt 5460
   tccagatttc ttgacaaggt tattcctaaa aaaactagaa caatattgct ccaaattgat 5520
   atcggactgt tctatattta ccgatgcgga cccctatggg attagcatag ccctaaatta 5580
   tactcactcg aatgaacgca acgcttatat ttgcacgatg gcaaactata aaggaattcg 5640
   tattacgcaa gttttggcac aaaataatga agtgcataac aaatccattc aattattgag 5700
   tttgaatcag cgcgactact ccttagccaa gaatttgata gcatctctga ctgccaacag 5760
   ctgggatatt gcaacttcac cattaaagaa cgtcatcata gaatgtcagc gggaaatttt 5820
   tttccaaaag aaagctgaaa tgaacgagat tgatgccaga atttttgaat acaaatccca 5880
   ccaccatcat catcacggag actacaagga tgacgatgac aaggactaca aggatgacga 5940
   tgacaaggac tacaaggatg acgatgacaa gtaatgagtc gacaacccct gcagccaagc 6000
   taattccggg cgaatttctt atgatttatg atttttatta ttaaataagt tataaaaaaa 6060
   ataagtgtat acaaatttta aagtgactct taggttttaa aacgaaaatt cttgttcttg 6120
   agtaactctt tcctgtaggt caggttgctt tctcaggtat agcatgaggt cgctcttatt 6180
   gaccacacct ctaccggcat gcaagcttgg cgtaatcatg gtcatagctg tttcctgtgt 6240
   gaaattgtta tccgctcaca attccacaca acatacgagc cggaagcata aagtgtaaag 6300
   cctggggtgc ctaatgagtg aggtaactca cattaattgc gttgcgctca ctgcccgctt 6360
   tccagtcggg aaacctgtcg tgccagctgg attaatgaat cggccaacgc gcggggagag 6420
   gcggtttgcg tattgggcgc tcttccgctt cctcgctcac tgactcgctg cgctcggtcg 6480
   ttcggctgcg gcgagcggta tcagctcact caaaggcggt aatacggtta tccacagaat 6540
   caggggataa cgcaggaaag aacatgtgag caaaaggcca gcaaaaggcc aggaaccgta 6600
   aaaaggccgc gttgctggcg tttttccata ggctccgccc ccctgacgag catcacaaaa 6660
   atcgacgctc aagtcagagg tggcgaaacc cgacaggact ataaagatac caggcgtttc 6720
   cccctggaag ctccctcgtg cgctctcctg ttccgaccct gccgcttacc ggatacctgt 6780
   ccgcctttct cccttcggga agcgtggcgc tttctcatag ctcacgctgt aggtatctca 6840
   gttcggtgta ggtcgttcgc tccaagctgg gctgtgtgca cgaacccccc gttcagcccg 6900
   accgctgcgc cttatccggt aactatcgtc ttgagtccaa cccggtaaga cacgacttat 6960
   cgccactggc agcagccact ggtaacagga ttagcagagc gaggtatgta ggcggtgcta 7020
   cagagttctt gaagtggtgg cctaactacg gctacactag aaggacagta tttggtatct 7080
   gcgctctgct gaagccagtt accttcggaa aaagagttgg tagctcttga tccggcaaac 7140
   aaaccaccgc tggtagcggt ggtttttttg tttgcaagca gcagattacg cgcagaaaaa 7200
   aaggatctca agaagatcct ttgatctttt ctacggggtc tgacgctcag tggaacgaaa 7260
   actcacgtta agggattttg gtcatgagat tatcaaaaag gatcttcacc tagatccttt 7320
   taaattaaaa atgaagtttt aaatcaatct aaagtatata tgagtaaact tggtctgaca 7380
   gttaccaatg cttaatcagt gaggcaccta tctcagcgat ctgtctattt cgttcatcca 7440
   tagttgcctg actccccgtc gtgtagataa ctacgatacg ggagggctta ccatctggcc 7500
   ccagtgctgc aatgataccg cgagacccac gctcaccggc tccagattta atcagcaata 7560
   aaccagccag ccggaagggc cgagcgcaga agtggtcctg caactttatc cgcctccatc 7620
   cagtctatta attgttgccg ggaagctaga gtaagtagtt cgccagttaa tagtttgcgc 7680
   aacgttgttg ccattgctac aggcatcgtg gtgtcacgct cgtcgtttgg tatggcttca 7740
   ttcagctccg gttcccaacg atcaaggcga gttacatgat cccccatgtt gtgcaaaaaa 7800
   gcggttagct ccttcggtcc tccgatcgtt gtcagaagta agttggccgc agtgttatca 7860
   ctcatggtta tggcagcact gcataattct cttactgtca tgccatccgt aagatgcttt 7920
   tctgtgactg gtgagtactc aaccaagtca ttctgagaat agtgtatgcg gcgaccgagt 7980
   tgctcttgcc cggcgtcaat acgggataat accgcgccac atagcagaac tttaaaagtg 8040
   ctcatcattg gaaaacgttc ttcggggcga aaactctcaa ggatcttacc gctgttgaga 8100
   tccagttcga tgtaacccac tcgtgcaccc aactgatctt cagcatcttt tactttcacc 8160
   agcgtttctg ggtgagcaaa aacaggaagg caaaatgccg caaaaaaggg aataagggcg 8220
   acacggaaat gttgaatact catactcttc ctttttcaat attattgaag catttatcag 8280
   ggttattgtc tcatgagcgg atacatattt gaatgtattt agaaaaataa acaaataggg 8340
   gttccgcgca catttccccg aaaagtgcca cctgacgtct aagaaaccat tattatcatg 8400
   acattaacct ataaaaatag gcgtatcacg aggccctttc gtctcgcgcg tttcggtgat 8460
   gacggtgaaa acctctgaca catgcagctc ccggagacgg tcacagcttg tctgtaagcg 8520
   gatgccggga gcagacaagc ccgtcagggc gcgtcagcgg gtgttggcgg gtgtcggggc 8580
   tggcttaact atgcggcatc agagcagatt gtactgagag tgcaccataa acgacattac 8640
   tatatatata atataggaag catttaatag acagcatcgt aatatatgtg tactttgcag 8700
   ttatgacgcc agatggcagt agtggaagat attctttatt gaaaaatagc ttgtcacctt 8760
   acgtacaatc ttgatccgga gcttttcttt ttttgccgat taagaattaa ttcggtcgaa 8820
   aaaagaaaag gagagggcca agagggaggg cattggtgac tattgagcac gtgagtatac 8880
   gtgattaagc acacaaaggc agcttggagt atgtctgtta ttaatttcac aggtagttct 8940
   ggtccattgg tgaaagtttg cggcttgcag agcacagagg ccgcagaatg tgctctagat 9000
   tccgatgctg acttgctggg tattatatgt gtgcccaata gaaagagaac aattgacccg 9060
   gttattgcaa ggaaaatttc aagtcttgta aaagcatata aaaatagttc aggcactccg 9120
   aaatacttgg ttggcgtgtt tcgtaatcaa cctaaggagg atgttttggc tctggtcaat 9180
   gattacggca ttgatatcgt ccaactgcat ggagatgagt cgtggcaaga ataccaagag 9240
   ttcctcggtt tgccagttat taaaagactc gtatttccaa aagactgcaa catactactc 9300
   agtgcagctt cacagaaacc tcattcgttt attcccttgt ttgattcaga agcaggtggg 9360
   acaggtgaac ttttggattg gaactcgatt tctgactggg ttggaaggca agagagcccc 9420
   gaaagcttac attttatgtt agctggtgga ctgacgccag aaaatgttgg tgatgcgctt 9480
   agattaaatg gcgttattgg tgttgatgta agcggaggtg tggagacaaa tggtgtaaaa 9540
   gactctaaca aaatagcaaa tttcgtcaaa aatgctaaga aataggttat tactgagtag 9600
   tatttattta agtattgttt gtgcacttgc cgatctatgc ggtgtgaaat accgcacaga 9660
   tgcgtaagga gaaaataccg catcaggaaa ttgtaaacgt taatattttg ttaaaattcg 9720
   cgttaaattt ttgttaaatc agctcatttt ttaaccaata ggccgaaatc ggcaaaatcc 9780
   cttataaatc aaaagaatag accgagatag ggttgagtgt tgttccagtt tggaacaaga 9840
   gtccactatt aaagaacgtg gactccaacg tcaaagggcg aaaaaccgtc tatcagggcg 9900
   atggcccact acgtgaacca tcaccctaat caagtttttt ggggtcgagg tgccgtaaag 9960
   cactaaatcg gaaccctaaa gggagccccc gatttagagc ttgacgggga aagccggcga 10020
   acgtggcgag aaaggaaggg aagaaagcga aaggagcggg cgctagggcg ctggcaagtg 10080
   tagcggtcac gctgcgcgta accaccacac ccgccgcgct taatgcgccg ctacagggcg 10140
   cgtcgcgcca ttcgccattc aggctgcgca actgttggga agggcgatcg gtgcgggcct 10200
   cttcgctatt acgccagctg gcgaaagggg gatgtgctgc aaggcgatta agttgggtaa 10260
   cgccagggtt ttcccagtca cgacgttgta aaacgacggc cagtcgtcca agctttcgat 10320
   catcgatgaa ttcgagctcg ttttcgacag cagtatagcg accagcattc acatacgatt 10380
   gacgcatgat attactttct gcgcacttaa cttcgcatct gggcagatga tgtcgaggcg 10440
   aaaaaaaata taaatcacgc taacatttga ttaaaataga acaactacaa tataaaaaaa 10500
   ctatacaaat gacaagttct tgaaaacaag aatcttttta ttgtcagtac tgattagaaa 10560
   aactcatcga gcatcaaatg aaactgcaat ttattcatat caggattatc aataccatat 10620
   ttttgaaaaa gccgtttctg taatgaagga gaaaactcac cgaggcagtt ccataggatg 10680
   gcaagatcct ggtatcggtc tgcgattccg actcgtccaa catcaataca acctattaat 10740
   ttcccctcgt caaaaataag gttatcaagt gagaaatcac catgagtgac gactgaatcc 10800
   ggtgagaatg gcaaaagctt atgcatttct ttccagactt gttcaacagg ccagccatta 10860
   cgctcgtcat caaaatcact cgcatcaacc aaaccgttat tcattcgtga ttgcgcctga 10920
   gcgagacgaa atacgcgatc gctgttaaaa ggacaattac aaacaggaat cgaatgcaac 10980
   cggcgcagga acactgccag cgcatcaaca atattttcac ctgaatcagg atattcttct 11040
   aatacctgga atgctgtttt gccggggatc gcagtggtga gtaaccatgc atcatcagga 11100
   gtacggataa aatgcttgat ggtcggaaga ggcataaatt ccgtcagcca gtttagtctg 11160
   accatctcat ctgtaacatc attggcaacg ctacctttgc catgtttcag aaacaactct 11220
   ggcgcatcgg gcttcccata caatcgatag attgtcgcac ctgattgccc gacattatcg 11280
   cgagcccatt tatacccata taaatcagca tccatgttgg aatttaatcg cggcctcgaa 11340
   acgtgagtct tttccttacc catggttgtt tatgttcgga tgtgatgtga gaactgtatc 11400
   ctagcaagat tttaaaagga agtatatgaa agaagaacct cagtggcaaa tcctaacctt 11460
   ttatatttct ctacaggggc gcggcgtggg gacaattcaa cgcgtctgtg aggggagcgt 11520
   ttccctgctc gcaggtctgc agcgaggagc cgtaattttt gcttcgcgcc gtgcggccat 11580
   caaaatgtat ggatgcaaat gattatacat ggggatgtat gggctaaatg tacgggcgac 11640
   agtcacatca tgcccctgag ctgcgcacgt caagactgtc aaggagggta ttctgggcct 11700
   ccatgtcgct ggccgggtga cccggcgggg acgaggcaag ctaaacagat ctggcgcgcc 11760
   ttaattaacc ccgagctcga gatcccgagc ttgcaaatta aagccttcga gcgtcccaaa 11820
   accttctcaa gcaaggtttt cagtataatg ttacatgcgt acacgcgtct gtacagaaaa 11880
   aaaagaaaaa tttgaaatat aaataacgtt cttaatacta acataactat aaaaaaataa 11940
   atagggacct agacttcagg ttgtctaact ccttcctttt cggttagagc ggatgtgggg 12000
   ggagggcgtg aatgtaagcg tgacataact aattacatga tatccttttg ttgtttccgg 12060
   gtgtacaata tggacttcct cttttctggc aaccaaaccc atacatcggg attcctataa 12120
   taccttcgtt ggtctcccta acatgtaggt ggcggagggg agatatacaa tagaacagat 12180
   accagacaag acataatggg ctaaacaaaa ctacaccaat tacactgcct cattgatggt 12240
   ggtacataac gaactaatac tgtagcccta gacttgatag ccatcatcat atcgaagttt 12300
   cactaccctt tttccatttg ccatctattg aagtaataat aggcgcatg 12349
<210> 3
   <211> 7
   <212> PRT
   <213> Artificial Sequence
<220>
   <223> Séquence signal de localisation nucléaire
<400> 3
   Pro Lys Lys Lys Arg Lys Val
   1               5
<210> 4
   <211> 20
   <212> PRT
   <213> VIH-I
<400> 4
   Gly Arg Lys Lys Arg Arg Gln Arg Arg Arg Pro Pro Gln Pro Lys Lys
   1               5                   10                  15
   Lys Arg Lys Val
               20
<210> 5
   <211> 19
   <212> PRT
   <213> Virus de l'hépatite B humaine
<400> 5
   Pro Leu Ser Ser Ile Phe Ser Arg Ile Gly Asp Pro Pro Lys Lys Lys
   1               5                   10                  15
   Arg Lys Val
<210> 6
   <211> 1839
   <212> PRT
   <213> Artificial Sequence
<220>
   <223> Protéine de fusion comprenant la construction Cas9 sauvage -Spo11
<400> 6
   Met Gly Lys Pro Ile Pro Asn Pro Leu Leu Gly Leu Asp Ser Thr Gly
   1               5                   10                  15
   Gly Met Ala Pro Lys Lys Lys Arg Lys Val Asp Gly Gly Gly Ile His
               20                  25                  30
   Gly Val Pro Ala Ala Met Asp Lys Lys Tyr Ser Ile Gly Leu Asp Ile
           35                  40                  45
   Gly Thr Asn Ser Val Gly Trp Ala Val Ile Thr Asp Glu Tyr Lys Val
   50 55 60
   Pro Ser Lys Lys Phe Lys Val Leu Gly Asn Thr Asp Arg His Ser Ile
   65 70 75 80
   Lys Lys Asn Leu Ile Gly Ala Leu Leu Phe Asp Ser Gly Glu Thr Ala
   85 90 95
   Glu Ala Thr Arg Leu Lys Arg Thr Ala Arg Arg Arg Tyr Thr Arg Arg
   100 105 110
   Lys Asn Arg Ile Cys Tyr Leu Gln Glu Ile Phe Ser Asn Glu Met Ala
   115 120 125
   Lys Val Asp Asp Ser Phe Phe His Arg Leu Glu Glu Ser Phe Leu Val
   130 135 140
   Glu Glu Asp Lys Lys His Glu Arg His Pro Ile Phe Gly Asn Ile Val
   145 150 155 160
   Asp Glu Val Ala Tyr His Glu Lys Tyr Pro Thr Ile Tyr His Leu Arg
   165 170 175
   Lys Lys Leu Val Asp Ser Thr Asp Lys Ala Asp Leu Arg Leu Ile Tyr
   180 185 190
   Leu Ala Leu Ala His Met Ile Lys Phe Arg Gly His Phe Leu Ile Glu
   195 200 205
   Gly Asp Leu Asn Pro Asp Asn Ser Asp Val Asp Lys Leu Phe Ile Gln
   210 215 220
   Leu Val Gln Thr Tyr Asn Gln Leu Phe Glu Glu Asn Pro Ile Asn Ala
   225 230 235 240
   Ser Gly Val Asp Ala Lys Ala Ile Leu Ser Ala Arg Leu Ser Lys Ser
   245 250 255
   Arg Arg Leu Glu Asn Leu Ile Ala Gln Leu Pro Gly Glu Lys Lys Asn
   260 265 270
   Gly Leu Phe Gly Asn Leu Ile Ala Leu Ser Leu Gly Leu Thr Pro Asn
   275 280 285
   Phe Lys Ser Asn Phe Asp Leu Ala Glu Asp Ala Lys Leu Gln Leu Ser
   290 295 300
   Lys Asp Thr Tyr Asp Asp Asp Leu Asp Asn Leu Leu Ala Gln Ile Gly
   305                 310                 315                 320
   Asp Gln Tyr Ala Asp Leu Phe Leu Ala Ala Lys Asn Leu Ser Asp Ala
                   325                 330                 335
   Ile Leu Leu Ser Asp Ile Leu Arg Val Asn Thr Glu Ile Thr Lys Ala
               340                 345                 350
   Pro Leu Ser Ala Ser Met Ile Lys Arg Tyr Asp Glu His His Gln Asp
           355                 360                 365
   Leu Thr Leu Leu Lys Ala Leu Val Arg Gln Gln Leu Pro Glu Lys Tyr
       370                 375                 380
   Lys Glu Ile Phe Phe Asp Gln Ser Lys Asn Gly Tyr Ala Gly Tyr Ile
   385                 390                 395                 400
   Asp Gly Gly Ala Ser Gln Glu Glu Phe Tyr Lys Phe Ile Lys Pro Ile
                   405                 410                 415
   Leu Glu Lys Met Asp Gly Thr Glu Glu Leu Leu Val Lys Leu Asn Arg
               420                 425                 430
   Glu Asp Leu Leu Arg Lys Gln Arg Thr Phe Asp Asn Gly Ser Ile Pro
           435                 440                 445
   His Gln Ile His Leu Gly Glu Leu His Ala Ile Leu Arg Arg Gln Glu
       450                 455                 460
   Asp Phe Tyr Pro Phe Leu Lys Asp Asn Arg Glu Lys Ile Glu Lys Ile
   465                 470                 475                 480
   Leu Thr Phe Arg Ile Pro Tyr Tyr Val Gly Pro Leu Ala Arg Gly Asn
                   485                 490                 495
   Ser Arg Phe Ala Trp Met Thr Arg Lys Ser Glu Glu Thr Ile Thr Pro
               500                 505                 510
   Trp Asn Phe Glu Glu Val Val Asp Lys Gly Ala Ser Ala Gln Ser Phe
           515                 520                 525
   Ile Glu Arg Met Thr Asn Phe Asp Lys Asn Leu Pro Asn Glu Lys Val
       530                 535                 540
   Leu Pro Lys His Ser Leu Leu Tyr Glu Tyr Phe Thr Val Tyr Asn Glu
   545 550 555 560
   Leu Thr Lys Val Lys Tyr Val Thr Glu Gly Met Arg Lys Pro Ala Phe
   565 570 575
   Leu Ser Gly Glu Gln Lys Lys Ala Ile Val Asp Leu Leu Phe Lys Thr
   580 585 590
   Asn Arg Lys Val Thr Val Lys Gln Leu Lys Glu Asp Tyr Phe Lys Lys
   595 600 605
   Ile Glu Cys Phe Asp Ser Val Glu Ile Ser Gly Val Glu Asp Arg Phe
   610 615 620
   Asn Ala Ser Leu Gly Thr Tyr His Asp Leu Leu Lys Ile Ile Lys Asp
   625 630 635 640
   Lys Asp Phe Leu Asp Asn Glu Glu Asn Glu Asp Ile Leu Glu Asp Ile
   645 650 655
   Val Leu Thr Leu Thr Leu Phe Glu Asp Arg Glu Met Ile Glu Glu Arg
   660 665 670
   Leu Lys Thr Tyr Ala His Leu Phe Asp Asp Lys Val Met Lys Gln Leu
   675 680 685
   Lys Arg Arg Arg Tyr Thr Gly Trp Gly Arg Leu Ser Arg Lys Leu Ile
   690 695 700
   Asn Gly Ile Arg Asp Lys Gln Ser Gly Lys Thr Ile Leu Asp Phe Leu
   705 710 715 720
   Lys Ser Asp Gly Phe Ala Asn Arg Asn Phe Met Gln Leu Ile His Asp
   725 730 735
   Asp Ser Leu Thr Phe Lys Glu Asp Ile Gln Lys Ala Gln Val Ser Gly
   740 745 750
   Gln Gly Asp Ser Leu His Glu His Ile Ala Asn Leu Ala Gly Ser Pro
   755 760 765
   Ala Ile Lys Lys Gly Ile Leu Gln Thr Val Lys Val Val Asp Glu Leu
   770 775 780
   Val Lys Val Met Gly Arg His Lys Pro Glu Asn Ile Val Ile Glu Met
   785 790 795 800
   Ala Arg Glu Asn Gln Thr Thr Gln Lys Gly Gln Lys Asn Ser Arg Glu
   805 810 815
   Arg Met Lys Arg Ile Glu Glu Gly Ile Lys Glu Leu Gly Ser Gln Ile
   820 825 830
   Leu Lys Glu His Pro Val Glu Asn Thr Gln Leu Gln Asn Glu Lys Leu
   835 840 845
   Tyr Leu Tyr Tyr Leu Gln Asn Gly Arg Asp Met Tyr Val Asp Gln Glu
   850 855 860
   Leu Asp Ile Asn Arg Leu Ser Asp Tyr Asp Val Asp His Ile Val Pro
   865 870 875 880
   Gln Ser Phe Leu Lys Asp Asp Ser Ile Asp Asn Lys Val Leu Thr Arg
   885 890 895
   Ser Asp Lys Asn Arg Gly Lys Ser Asp Asn Val Pro Ser Glu Glu Val
   900 905 910
   Val Lys Lys Met Lys Asn Tyr Trp Arg Gln Leu Leu Asn Ala Lys Leu
   915 920 925
   Ile Thr Gln Arg Lys Phe Asp Asn Leu Thr Lys Ala Glu Arg Gly Gly
   930 935 940
   Leu Ser Glu Leu Asp Lys Ala Gly Phe Ile Lys Arg Gln Leu Val Glu
   945 950 955 960
   Thr Arg Gln Ile Thr Lys His Val Ala Gln Ile Leu Asp Ser Arg Met
   965 970 975
   Asn Thr Lys Tyr Asp Glu Asn Asp Lys Leu Ile Arg Glu Val Lys Val
   980 985 990
   Ile Thr Leu Lys Ser Lys Leu Val Ser Asp Phe Arg Lys Asp Phe Gln
   995 1000 1005
   Phe Tyr Lys Val Arg Glu Ile Asn Asn Tyr His His Ala His Asp
   1010 1015 1020
   Ala Tyr Leu Asn Ala Val Val Gly Thr Ala Leu Ile Lys Lys Tyr
   1025 1030 1035
   Pro Lys Leu Glu Ser Glu Phe Val Tyr Gly Asp Tyr Lys Val Tyr
   1040 1045 1050
   Asp Val Arg Lys Met Ile Ala Lys Ser Glu Gln Glu Ile Gly Lys
   1055 1060 1065
   Ala Thr Ala Lys Tyr Phe Phe Tyr Ser Asn Ile Met Asn Phe Phe
   1070 1075 1080
   Lys Thr Glu Ile Thr Leu Ala Asn Gly Glu Ile Arg Lys Arg Pro
   1085 1090 1095
   Leu Ile Glu Thr Asn Gly Glu Thr Gly Glu Ile Val Trp Asp Lys
   1100 1105 1110
   Gly Arg Asp Phe Ala Thr Val Arg Lys Val Leu Ser Met Pro Gln
   1115 1120 1125
   Val Asn Ile Val Lys Lys Thr Glu Val Gln Thr Gly Gly Phe Ser
   1130 1135 1140
   Lys Glu Ser Ile Leu Pro Lys Arg Asn Ser Asp Lys Leu Ile Ala
   1145 1150 1155
   Arg Lys Lys Asp Trp Asp Pro Lys Lys Tyr Gly Gly Phe Asp Ser
   1160 1165 1170
   Pro Thr Val Ala Tyr Ser Val Leu Val Val Ala Lys Val Glu Lys
   1175 1180 1185
   Gly Lys Ser Lys Lys Leu Lys Ser Val Lys Glu Leu Leu Gly Ile
   1190 1195 1200
   Thr Ile Met Glu Arg Ser Ser Phe Glu Lys Asn Pro Ile Asp Phe
   1205 1210 1215
   Leu Glu Ala Lys Gly Tyr Lys Glu Val Lys Lys Asp Leu Ile Ile
   1220 1225 1230
   Lys Leu Pro Lys Tyr Ser Leu Phe Glu Leu Glu Asn Gly Arg Lys
   1235 1240 1245
   Arg Met Leu Ala Ser Ala Gly Glu Leu Gln Lys Gly Asn Glu Leu
   1250 1255 1260
   Ala Leu Pro Ser Lys Tyr Val Asn Phe Leu Tyr Leu Ala Ser His
   1265 1270 1275
   Tyr Glu Lys Leu Lys Gly Ser Pro Glu Asp Asn Glu Gln Lys Gln
   1280 1285 1290
   Leu Phe Val Glu Gln His Lys His Tyr Leu Asp Glu Ile Ile Glu
   1295 1300 1305
   Gln Ile Ser Glu Phe Ser Lys Arg Val Ile Leu Ala Asp Ala Asn
   1310 1315 1320
   Leu Asp Lys Val Leu Ser Ala Tyr Asn Lys His Arg Asp Lys Pro
   1325 1330 1335
   Ile Arg Glu Gln Ala Glu Asn Ile Ile His Leu Phe Thr Leu Thr
   1340 1345 1350
   Asn Leu Gly Ala Pro Ala Ala Phe Lys Tyr Phe Asp Thr Thr Ile
   1355 1360 1365
   Asp Arg Lys Arg Tyr Thr Ser Thr Lys Glu Val Leu Asp Ala Thr
   1370 1375 1380
   Leu Ile His Gln Ser Ile Thr Gly Leu Tyr Glu Thr Arg Ile Asp
   1385 1390 1395
   Leu Ser Gln Leu Gly Gly Asp Pro Glu Phe Met Ala Met Glu Ala
   1400 1405 1410
   Pro Gly Ile Arg Met Ala Leu Glu Gly Leu Arg Lys Lys Tyr Lys
   1415 1420 1425
   Thr Arg Gln Glu Leu Val Lys Ala Leu Thr Pro Lys Arg Arg Ser
   1430 1435 1440
   Ile His Leu Asn Ser Asn Gly His Ser Asn Gly Thr Pro Cys Ser
   1445 1450 1455
   Asn Ala Asp Val Leu Ala His Ile Lys His Phe Leu Ser Leu Ala
   1460 1465 1470
   Ala Asn Ser Leu Glu Gln His Gln Gln Pro Ile Ser Ile Val Phe
   1475 1480 1485
   Gln Asn Lys Lys Lys Lys Gly Asp Thr Ser Ser Pro Asp Ile His
   1490 1495 1500
   Thr Thr Leu Asp Phe Pro Leu Asn Gly Pro His Leu Cys Thr His
   1505 1510 1515
   Gln Phe Lys Leu Lys Arg Cys Ala Ile Leu Leu Asn Leu Leu Lys
   1520 1525 1530
   Val Val Met Glu Lys Leu Pro Leu Gly Lys Asn Thr Thr Val Arg
   1535 1540 1545
   Asp Ile Phe Tyr Ser Asn Val Glu Leu Phe Gln Arg Gln Ala Asn
   1550 1555 1560
   Val Val Gln Trp Leu Asp Val Ile Arg Phe Asn Phe Lys Leu Ser
   1565 1570 1575
   Pro Arg Lys Ser Leu Asn Ile Ile Pro Ala Gln Lys Gly Leu Val
   1580 1585 1590
   Tyr Ser Pro Phe Pro Ile Asp Ile Tyr Asp Asn Ile Leu Thr Cys
   1595 1600 1605
   Glu Asn Glu Pro Lys Met Gln Lys Gln Thr Ile Phe Pro Gly Lys
   1610 1615 1620
   Pro Cys Leu Ile Pro Phe Phe Gln Asp Asp Ala Val Ile Lys Leu
   1625 1630 1635
   Gly Thr Thr Ser Met Cys Asn Ile Val Ile Val Glu Lys Glu Ala
   1640 1645 1650
   Val Phe Thr Lys Leu Val Asn Asn Tyr His Lys Leu Ser Thr Asn
   1655 1660 1665
   Thr Met Leu Ile Thr Gly Lys Gly Phe Pro Asp Phe Leu Thr Arg
   1670 1675 1680
   Leu Phe Leu Lys Lys Leu Glu Gln Tyr Cys Ser Lys Leu Ile Ser
   1685 1690 1695
   Asp Cys Ser Ile Phe Thr Asp Ala Asp Pro Tyr Gly Ile Ser Ile
   1700 1705 1710
   Ala Leu Asn Tyr Thr His Ser Asn Glu Arg Asn Ala Tyr Ile Cys
   1715 1720 1725
   Thr Met Ala Asn Tyr Lys Gly Ile Arg Ile Thr Gln Val Leu Ala
   1730 1735 1740
   Gln Asn Asn Glu Val His Asn Lys Ser Ile Gln Leu Leu Ser Leu
   1745 1750 1755
   Asn Gln Arg Asp Tyr Ser Leu Ala Lys Asn Leu Ile Ala Ser Leu
   1760 1765 1770
   Thr Ala Asn Ser Trp Asp Ile Ala Thr Ser Pro Leu Lys Asn Val
   1775 1780 1785
   Ile Ile Glu Cys Gln Arg Glu Ile Phe Phe Gln Lys Lys Ala Glu
   1790 1795 1800
   Met Asn Glu Ile Asp Ala Arg Ile Phe Glu Tyr Lys Asp Tyr Lys
   1805 1810 1815
   Asp Asp Asp Asp Lys Asp Tyr Lys Asp Asp Asp Asp Lys Asp Tyr
   1820 1825 1830
   Lys Asp Asp Asp Asp Lys
   1835
<210> 7
   <211> 1839
   <212> PRT
   <213> Artificial Sequence
<220>
   <223> Protéine de fusion comprenant la construction Cas9*-Spo11
<400> 7
   Met Gly Lys Pro Ile Pro Asn Pro Leu Leu Gly Leu Asp Ser Thr Gly
   1               5                   10                  15
   Gly Met Ala Pro Lys Lys Lys Arg Lys Val Asp Gly Gly Gly Ile His
               20                  25                  30
   Gly Val Pro Ala Ala Met Asp Lys Lys Tyr Ser Ile Gly Leu Ala Ile
           35                  40                  45
   Gly Thr Asn Ser Val Gly Trp Ala Val Ile Thr Asp Glu Tyr Lys Val
       50                  55                  60
   Pro Ser Lys Lys Phe Lys Val Leu Gly Asn Thr Asp Arg His Ser Ile
   65                  70                  75                  80
   Lys Lys Asn Leu Ile Gly Ala Leu Leu Phe Asp Ser Gly Glu Thr Ala
                   85                  90                  95
   Glu Ala Thr Arg Leu Lys Arg Thr Ala Arg Arg Arg Tyr Thr Arg Arg
               100                 105                 110
   Lys Asn Arg Ile Cys Tyr Leu Gln Glu Ile Phe Ser Asn Glu Met Ala
           115                 120                 125
   Lys Val Asp Asp Ser Phe Phe His Arg Leu Glu Glu Ser Phe Leu Val
       130                 135                 140
   Glu Glu Asp Lys Lys His Glu Arg His Pro Ile Phe Gly Asn Ile Val
   145                 150                 155                 160
   Asp Glu Val Ala Tyr His Glu Lys Tyr Pro Thr Ile Tyr His Leu Arg
                   165                 170                 175
   Lys Lys Leu Val Asp Ser Thr Asp Lys Ala Asp Leu Arg Leu Ile Tyr
               180                 185                 190
   Leu Ala Leu Ala His Met Ile Lys Phe Arg Gly His Phe Leu Ile Glu
           195                 200                 205
   Gly Asp Leu Asn Pro Asp Asn Ser Asp Val Asp Lys Leu Phe Ile Gln
       210                 215                 220
   Leu Val Gln Thr Tyr Asn Gln Leu Phe Glu Glu Asn Pro Ile Asn Ala
   225                 230                 235                 240
   Ser Gly Val Asp Ala Lys Ala Ile Leu Ser Ala Arg Leu Ser Lys Ser
                   245                 250                 255
   Arg Arg Leu Glu Asn Leu Ile Ala Gln Leu Pro Gly Glu Lys Lys Asn
               260                 265                 270
   Gly Leu Phe Gly Asn Leu Ile Ala Leu Ser Leu Gly Leu Thr Pro Asn
           275                 280                 285
   Phe Lys Ser Asn Phe Asp Leu Ala Glu Asp Ala Lys Leu Gln Leu Ser
       290                 295                 300
   Lys Asp Thr Tyr Asp Asp Asp Leu Asp Asn Leu Leu Ala Gln Ile Gly
   305                 310                 315                 320
   Asp Gln Tyr Ala Asp Leu Phe Leu Ala Ala Lys Asn Leu Ser Asp Ala
                   325                 330                 335
   Ile Leu Leu Ser Asp Ile Leu Arg Val Asn Thr Glu Ile Thr Lys Ala
               340                 345                 350
   Pro Leu Ser Ala Ser Met Ile Lys Arg Tyr Asp Glu His His Gln Asp
           355                 360                 365
   Leu Thr Leu Leu Lys Ala Leu Val Arg Gln Gln Leu Pro Glu Lys Tyr
       370                 375                 380
   Lys Glu Ile Phe Phe Asp Gln Ser Lys Asn Gly Tyr Ala Gly Tyr Ile
   385                 390                 395                 400
   Asp Gly Gly Ala Ser Gln Glu Glu Phe Tyr Lys Phe Ile Lys Pro Ile
                   405                 410                 415
   Leu Glu Lys Met Asp Gly Thr Glu Glu Leu Leu Val Lys Leu Asn Arg
               420                 425                 430
   Glu Asp Leu Leu Arg Lys Gln Arg Thr Phe Asp Asn Gly Ser Ile Pro
           435                 440                 445
   His Gln Ile His Leu Gly Glu Leu His Ala Ile Leu Arg Arg Gln Glu
       450                 455                 460
   Asp Phe Tyr Pro Phe Leu Lys Asp Asn Arg Glu Lys Ile Glu Lys Ile
   465                 470                 475                 480
   Leu Thr Phe Arg Ile Pro Tyr Tyr Val Gly Pro Leu Ala Arg Gly Asn
                   485                 490                 495
   Ser Arg Phe Ala Trp Met Thr Arg Lys Ser Glu Glu Thr Ile Thr Pro
               500                 505                 510
   Trp Asn Phe Glu Glu Val Val Asp Lys Gly Ala Ser Ala Gln Ser Phe
           515                 520                 525
   Ile Glu Arg Met Thr Asn Phe Asp Lys Asn Leu Pro Asn Glu Lys Val
       530                 535                 540
   Leu Pro Lys His Ser Leu Leu Tyr Glu Tyr Phe Thr Val Tyr Asn Glu
   545                 550                 555                 560
   Leu Thr Lys Val Lys Tyr Val Thr Glu Gly Met Arg Lys Pro Ala Phe
                   565                 570                 575
   Leu Ser Gly Glu Gln Lys Lys Ala Ile Val Asp Leu Leu Phe Lys Thr
               580                 585                 590
   Asn Arg Lys Val Thr Val Lys Gln Leu Lys Glu Asp Tyr Phe Lys Lys
           595                 600                 605
   Ile Glu Cys Phe Asp Ser Val Glu Ile Ser Gly Val Glu Asp Arg Phe
       610                 615                 620
   Asn Ala Ser Leu Gly Thr Tyr His Asp Leu Leu Lys Ile Ile Lys Asp
   625 630 635 640
   Lys Asp Phe Leu Asp Asn Glu Glu Asn Glu Asp Ile Leu Glu Asp Ile
   645 650 655
   Val Leu Thr Leu Thr Leu Phe Glu Asp Arg Glu Met Ile Glu Glu Arg
   660 665 670
   Leu Lys Thr Tyr Ala His Leu Phe Asp Asp Lys Val Met Lys Gln Leu
   675 680 685
   Lys Arg Arg Arg Tyr Thr Gly Trp Gly Arg Leu Ser Arg Lys Leu Ile
   690 695 700
   Asn Gly Ile Arg Asp Lys Gln Ser Gly Lys Thr Ile Leu Asp Phe Leu
   705 710 715 720
   Lys Ser Asp Gly Phe Ala Asn Arg Asn Phe Met Gln Leu Ile His Asp
   725 730 735
   Asp Ser Leu Thr Phe Lys Glu Asp Ile Gln Lys Ala Gln Val Ser Gly
   740 745 750
   Gln Gly Asp Ser Leu His Glu His Ile Ala Asn Leu Ala Gly Ser Pro
   755 760 765
   Ala Ile Lys Lys Gly Ile Leu Gln Thr Val Lys Val Val Asp Glu Leu
   770 775 780
   Val Lys Val Met Gly Arg His Lys Pro Glu Asn Ile Val Ile Glu Met
   785 790 795 800
   Ala Arg Glu Asn Gln Thr Thr Gln Lys Gly Gln Lys Asn Ser Arg Glu
   805 810 815
   Arg Met Lys Arg Ile Glu Glu Gly Ile Lys Glu Leu Gly Ser Gln Ile
   820 825 830
   Leu Lys Glu His Pro Val Glu Asn Thr Gln Leu Gln Asn Glu Lys Leu
   835 840 845
   Tyr Leu Tyr Tyr Leu Gln Asn Gly Arg Asp Met Tyr Val Asp Gln Glu
   850 855 860
   Leu Asp Ile Asn Arg Leu Ser Asp Tyr Asp Val Asp Ala Ile Val Pro
   865 870 875 880
   Gln Ser Phe Leu Lys Asp Asp Ser Ile Asp Asn Lys Val Leu Thr Arg
                   885                 890                 895
   Ser Asp Lys Asn Arg Gly Lys Ser Asp Asn Val Pro Ser Glu Glu Val
               900                 905                 910
   Val Lys Lys Met Lys Asn Tyr Trp Arg Gln Leu Leu Asn Ala Lys Leu
           915                 920                 925
   Ile Thr Gln Arg Lys Phe Asp Asn Leu Thr Lys Ala Glu Arg Gly Gly
       930                 935                 940
   Leu Ser Glu Leu Asp Lys Ala Gly Phe Ile Lys Arg Gln Leu Val Glu
   945                 950                 955                 960
   Thr Arg Gln Ile Thr Lys His Val Ala Gln Ile Leu Asp Ser Arg Met
                   965                 970                 975
   Asn Thr Lys Tyr Asp Glu Asn Asp Lys Leu Ile Arg Glu Val Lys Val
               980                 985                 990
   Ile Thr Leu Lys Ser Lys Leu Val  Ser Asp Phe Arg Lys  Asp Phe Gln
           995                 1000                1005
   Phe Tyr  Lys Val Arg Glu Ile  Asn Asn Tyr His His  Ala His Asp
       1010                 1015                 1020
   Ala Tyr  Leu Asn Ala Val Val  Gly Thr Ala Leu Ile  Lys Lys Tyr
       1025                 1030                 1035
   Pro Lys  Leu Glu Ser Glu Phe  Val Tyr Gly Asp Tyr  Lys Val Tyr
       1040                 1045                 1050
   Asp Val  Arg Lys Met Ile Ala  Lys Ser Glu Gln Glu  Ile Gly Lys
       1055                 1060                 1065
   Ala Thr  Ala Lys Tyr Phe Phe  Tyr Ser Asn Ile Met  Asn Phe Phe
       1070                 1075                 1080
   Lys Thr  Glu Ile Thr Leu Ala  Asn Gly Glu Ile Arg  Lys Arg Pro
       1085                 1090                 1095
   Leu Ile  Glu Thr Asn Gly Glu  Thr Gly Glu Ile Val  Trp Asp Lys
       1100                 1105                 1110
   Gly Arg  Asp Phe Ala Thr Val  Arg Lys Val Leu Ser  Met Pro Gln
   1115 1120 1125
   Val Asn Ile Val Lys Lys Thr Glu Val Gln Thr Gly Gly Phe Ser
   1130 1135 1140
   Lys Glu Ser Ile Leu Pro Lys Arg Asn Ser Asp Lys Leu Ile Ala
   1145 1150 1155
   Arg Lys Lys Asp Trp Asp Pro Lys Lys Tyr Gly Gly Phe Asp Ser
   1160 1165 1170
   Pro Thr Val Ala Tyr Ser Val Leu Val Val Ala Lys Val Glu Lys
   1175 1180 1185
   Gly Lys Ser Lys Lys Leu Lys Ser Val Lys Glu Leu Leu Gly Ile
   1190 1195 1200
   Thr Ile Met Glu Arg Ser Ser Phe Glu Lys Asn Pro Ile Asp Phe
   1205 1210 1215
   Leu Glu Ala Lys Gly Tyr Lys Glu Val Lys Lys Asp Leu Ile Ile
   1220 1225 1230
   Lys Leu Pro Lys Tyr Ser Leu Phe Glu Leu Glu Asn Gly Arg Lys
   1235 1240 1245
   Arg Met Leu Ala Ser Ala Gly Glu Leu Gln Lys Gly Asn Glu Leu
   1250 1255 1260
   Ala Leu Pro Ser Lys Tyr Val Asn Phe Leu Tyr Leu Ala Ser His
   1265 1270 1275
   Tyr Glu Lys Leu Lys Gly Ser Pro Glu Asp Asn Glu Gln Lys Gln
   1280 1285 1290
   Leu Phe Val Glu Gln His Lys His Tyr Leu Asp Glu Ile Ile Glu
   1295 1300 1305
   Gln Ile Ser Glu Phe Ser Lys Arg Val Ile Leu Ala Asp Ala Asn
   1310 1315 1320
   Leu Asp Lys Val Leu Ser Ala Tyr Asn Lys His Arg Asp Lys Pro
   1325 1330 1335
   Ile Arg Glu Gln Ala Glu Asn Ile Ile His Leu Phe Thr Leu Thr
   1340 1345 1350
   Asn Leu Gly Ala Pro Ala Ala Phe Lys Tyr Phe Asp Thr Thr Ile
   1355 1360 1365
   Asp Arg Lys Arg Tyr Thr Ser Thr Lys Glu Val Leu Asp Ala Thr
   1370 1375 1380
   Leu Ile His Gln Ser Ile Thr Gly Leu Tyr Glu Thr Arg Ile Asp
   1385 1390 1395
   Leu Ser Gln Leu Gly Gly Asp Pro Glu Phe Met Ala Met Glu Ala
   1400 1405 1410
   Pro Gly Ile Arg Met Ala Leu Glu Gly Leu Arg Lys Lys Tyr Lys
   1415 1420 1425
   Thr Arg Gln Glu Leu Val Lys Ala Leu Thr Pro Lys Arg Arg Ser
   1430 1435 1440
   Ile His Leu Asn Ser Asn Gly His Ser Asn Gly Thr Pro Cys Ser
   1445 1450 1455
   Asn Ala Asp Val Leu Ala His Ile Lys His Phe Leu Ser Leu Ala
   1460 1465 1470
   Ala Asn Ser Leu Glu Gln His Gln Gln Pro Ile Ser Ile Val Phe
   1475 1480 1485
   Gln Asn Lys Lys Lys Lys Gly Asp Thr Ser Ser Pro Asp Ile His
   1490 1495 1500
   Thr Thr Leu Asp Phe Pro Leu Asn Gly Pro His Leu Cys Thr His
   1505 1510 1515
   Gln Phe Lys Leu Lys Arg Cys Ala Ile Leu Leu Asn Leu Leu Lys
   1520 1525 1530
   Val Val Met Glu Lys Leu Pro Leu Gly Lys Asn Thr Thr Val Arg
   1535 1540 1545
   Asp Ile Phe Tyr Ser Asn Val Glu Leu Phe Gln Arg Gln Ala Asn
   1550 1555 1560
   Val Val Gln Trp Leu Asp Val Ile Arg Phe Asn Phe Lys Leu Ser
   1565 1570 1575
   Pro Arg Lys Ser Leu Asn Ile Ile Pro Ala Gln Lys Gly Leu Val
   1580 1585 1590
   Tyr Ser Pro Phe Pro Ile Asp Ile Tyr Asp Asn Ile Leu Thr Cys
       1595                1600                1605
   Glu Asn Glu Pro Lys Met Gln Lys Gln Thr Ile Phe Pro Gly Lys
       1610                1615                1620
   Pro Cys Leu Ile Pro Phe Phe Gln Asp Asp Ala Val Ile Lys Leu
       1625                1630                1635
   Gly Thr Thr Ser Met Cys Asn Ile Val Ile Val Glu Lys Glu Ala
       1640                1645                1650
   Val Phe Thr Lys Leu Val Asn Asn Tyr His Lys Leu Ser Thr Asn
       1655                1660                1665
   Thr Met Leu Ile Thr Gly Lys Gly Phe Pro Asp Phe Leu Thr Arg
       1670                1675                1680
   Leu Phe Leu Lys Lys Leu Glu Gln Tyr Cys Ser Lys Leu Ile Ser
       1685                1690                1695
   Asp Cys Ser Ile Phe Thr Asp Ala Asp Pro Tyr Gly Ile Ser Ile
       1700                1705                1710
   Ala Leu Asn Tyr Thr His Ser Asn Glu Arg Asn Ala Tyr Ile Cys
       1715                1720                1725
   Thr Met Ala Asn Tyr Lys Gly Ile Arg Ile Thr Gln Val Leu Ala
       1730                1735                1740
   Gln Asn Asn Glu Val His Asn Lys Ser Ile Gln Leu Leu Ser Leu
       1745                1750                1755
   Asn Gln Arg Asp Tyr Ser Leu Ala Lys Asn Leu Ile Ala Ser Leu
       1760                1765                1770
   Thr Ala Asn Ser Trp Asp Ile Ala Thr Ser Pro Leu Lys Asn Val
       1775                1780                1785
   Ile Ile Glu Cys Gln Arg Glu Ile Phe Phe Gln Lys Lys Ala Glu
       1790                1795                1800
   Met Asn Glu Ile Asp Ala Arg Ile Phe Glu Tyr Lys Asp Tyr Lys
       1805                1810                1815
   Asp Asp Asp Asp Lys Asp Tyr Lys Asp Asp Asp Asp Lys Asp Tyr
       1820                1825                1830
   Lys Asp Asp Asp Asp Lys
   1835
<210> 8
   <211> 1368
   <212> PRT
   <213> Streptococcus pyogenes
<400> 8
<210> 9
   <211> 398
   <212> PRT
   <213> Saccharomyces cerevisiae
<400> 9
   Met Ala Leu Glu Gly Leu Arg Lys Lys Tyr Lys Thr Arg Gln Glu Leu
   1 5 10 15
   Val Lys Ala Leu Thr Pro Lys Arg Arg Ser Ile His Leu Asn Ser Asn
   20 25 30
   Gly His Ser Asn Gly Thr Pro Cys Ser Asn Ala Asp Val Leu Ala His
   35 40 45
   Ile Lys His Phe Leu Ser Leu Ala Ala Asn Ser Leu Glu Gln His Gln
       50                  55                  60
   Gln Pro Ile Ser Ile Val Phe Gln Asn Lys Lys Lys Lys Gly Asp Thr
   65                  70                  75                  80
   Ser Ser Pro Asp Ile His Thr Thr Leu Asp Phe Pro Leu Asn Gly Pro
                   85                  90                  95
   His Leu Cys Thr His Gln Phe Lys Leu Lys Arg Cys Ala Ile Leu Leu
               100                 105                 110
   Asn Leu Leu Lys Val Val Met Glu Lys Leu Pro Leu Gly Lys Asn Thr
           115                 120                 125
   Thr Val Arg Asp Ile Phe Tyr Ser Asn Val Glu Leu Phe Gln Arg Gln
       130                 135                 140
   Ala Asn Val Val Gln Trp Leu Asp Val Ile Arg Phe Asn Phe Lys Leu
   145                 150                 155                 160
   Ser Pro Arg Lys Ser Leu Asn Ile Ile Pro Ala Gln Lys Gly Leu Val
                   165                 170                 175
   Tyr Ser Pro Phe Pro Ile Asp Ile Tyr Asp Asn Ile Leu Thr Cys Glu
               180                 185                 190
   Asn Glu Pro Lys Met Gln Lys Gln Thr Ile Phe Pro Gly Lys Pro Cys
           195                 200                 205
   Leu Ile Pro Phe Phe Gln Asp Asp Ala Val Ile Lys Leu Gly Thr Thr
       210                 215                 220
   Ser Met Cys Asn Ile Val Ile Val Glu Lys Glu Ala Val Phe Thr Lys
   225                 230                 235                 240
   Leu Val Asn Asn Tyr His Lys Leu Ser Thr Asn Thr Met Leu Ile Thr
                   245                 250                 255
   Gly Lys Gly Phe Pro Asp Phe Leu Thr Arg Leu Phe Leu Lys Lys Leu
               260                 265                 270
   Glu Gln Tyr Cys Ser Lys Leu Ile Ser Asp Cys Ser Ile Phe Thr Asp
           275                 280                 285
   Ala Asp Pro Tyr Gly Ile Ser Ile Ala Leu Asn Tyr Thr His Ser Asn
   290 295 300
   Glu Arg Asn Ala Tyr Ile Cys Thr Met Ala Asn Tyr Lys Gly Ile Arg
   305 310 315 320
   Ile Thr Gln Val Leu Ala Gln Asn Asn Glu Val His Asn Lys Ser Ile
   325 330 335
   Gln Leu Leu Ser Leu Asn Gln Arg Asp Tyr Ser Leu Ala Lys Asn Leu
   340 345 350
   Ile Ala Ser Leu Thr Ala Asn Ser Trp Asp Ile Ala Thr Ser Pro Leu
   355 360 365
   Lys Asn Val Ile Ile Glu Cys Gln Arg Glu Ile Phe Phe Gln Lys Lys
   370 375 380
   Ala Glu Met Asn Glu Ile Asp Ala Arg Ile Phe Glu Tyr Lys
   385 390 395
<210> 10
   <211> 102
   <212> DNA
   <213> Artificial Sequence
<220>
   <223> ARN guide UAS1 ciblant la région codante en 5' du gène YCR048W
<400> 10
   cacacctgaa gagcaagtct gttttagagc tagaaatagc aagttaaaat aaggctagtc 60
   cgttatcaac ttgaaaaagt ggcaccgagt cggtgctttt tt 102
<210> 11
   <211> 102
   <212> DNA
   <213> Artificial Sequence
<220>
   <223> ARN guide UAS2 ciblant la région codante en 3' du gène YCR048W
<400> 11
   ttccacaaca cgcccacctt gttttagagc tagaaatagc aagttaaaat aaggctagtc 60
   cgttatcaac ttgaaaaagt ggcaccgagt cggtgctttt tt 102
<210> 12
   <211> 102
   <212> DNA
   <213> Artificial Sequence
<220>
   <223> ARN guide UAS D/E ciblant le site du promoteur du gène GAL2
<400> 12
   gatcactccg aaccgagatt gttttagagc tagaaatagc aagttaaaat aaggctagtc       60
   cgttatcaac ttgaaaaagt ggcaccgagt cggtgctttt tt                         102
<210> 13
   <211> 102
   <212> DNA
   <213> Artificial Sequence
<220>
   <223> ARN guide SWC3 ciblant la région codante du gène SWC3
<400> 13
   tgcgattgtg taatgagtgg gttttagagc tagaaatagc aagttaaaat aaggctagtc       60
   cgttatcaac ttgaaaaagt ggcaccgagt cggtgctttt tt                         102
<210> 14
   <211> 102
   <212> DNA
   <213> Artificial Sequence
<220>
   <223> ARN guide UAS A ciblant le site du promoteur de GAL2
<400> 14
   caattcggaa agcttccttc gttttagagc tagaaatagc aagttaaaat aaggctagtc       60
   cgttatcaac ttgaaaaagt ggcaccgagt cggtgctttt tt                         102
<210> 15
   <211> 102
   <212> DNA
   <213> Artificial Sequence
<220>
   <223> ARN guide UAS B ciblant le site du promoteur du gène GAL2
<400> 15
   ttgcctcagg aaggcaccgg gttttagagc tagaaatagc aagttaaaat aaggctagtc       60
   cgttatcaac ttgaaaaagt ggcaccgagt cggtgctttt tt                         102
<210> 16
   <211> 102
   <212> DNA
   <213> Artificial Sequence
<220>
   <223> ARN guide PUT4 ciblant la région codante du gène PUT4
<400> 16
   gttttgaatt ccgatcacaa gttttagagc tagaaatagc aagttaaaat aaggctagtc 60
   cgttatcaac ttgaaaaagt ggcaccgagt cggtgctttt tt 102
<210> 17
   <211> 36
   <212> DNA
   <213> Artificial Sequence
<220>
   <223> linker de la construction dCas9-SPO11 pour la transformation de cellules de riz
<400> 17
   ccggaattta tggccatgga ggccccgggg atccgt 36
<210> 18
   <211> 24
   <212> DNA
   <213> Artificial Sequence
<220>
   <223> Linker de la construction dCas9-SPO11 pour la transformation de cellule de riz
<400> 18
   ggtattcatg gagttcctgc tgcg 24